(19) Europäisches Patentamt
European Patent Office
Office européen des brevets

(11) EP 1 413 315 A1

(12) **EUROPEAN PATENT APPLICATION**
published in accordance with Art. 158(3) EPC

(43) Date of publication:
**28.04.2004 Bulletin 2004/18**

(21) Application number: **02755755.2**

(22) Date of filing: **01.08.2002**

(51) Int Cl.[7]: **A61K 45/06**, A61K 31/41, A61K 31/4178, A61K 31/4184, A61K 31/4245, A61K 31/519, A61K 47/34, A61P 9/00, A61P 9/12, A61P 35/00, A61P 43/00

(86) International application number:
**PCT/JP2002/007862**

(87) International publication number:
**WO 2003/013609 (20.02.2003 Gazette 2003/08)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **03.08.2001 JP 2001236794**

(71) Applicant: **Takeda Chemical Industries, Ltd.**
**Osaka-shi, Osaka 541-0045 (JP)**

(72) Inventors:
- **KUSUMOTO, Keiji**
**Shimamoto-cho, Mishima-gun, Osaka 618-00 (JP)**
- **HOSHINO, Tetsuo**
**Toyono-cho, Toyono-gun, Osaka 563-0105 (JP)**
- **KAWAMURA, Ryu**
**Yodogawa-ku, Osaka-shi, Osaka 532-0033 (JP)**

(74) Representative: **Teipel, Stephan, Dr. et al**
**Lederer & Keller**
**Patentanwälte**
**Prinzregentenstrasse 16**
**80538 München (DE)**

(54) **SUSTAINED-RELEASE MEDICINES**

(57) Sustained-release medicines comprising (A) an angiotensin II antagonist combined with (B) one or more drugs selected from among remedies for hypertension, hypoglycemics, remedies for hyperlipemia, antithromboties, remedies for menopause and anticancer drugs. Using these medicines, remarkably excellent effects can be achieved compared with the case of using each active ingredient alone, which makes it possible to lessen the administration dose and relieve side effects.



Printed by Jouve, 75001 PARIS (FR)

## Description

Technical field

The present invention relates to sustained-release medicines and the like comprising (A) an angiotensin II antagonist (hereinafter, abbreviated as "AII antagonist" in some cases) combined with (B) one or more drugs selected from among remedies for hypertension, hypoglycemic drugs, remedies for hyperlipemia, antithrombotic drugs, remedies for menopause and anticancer drugs.

Background Art

Angiotensin II has a potent vasoconstriction activity, aldosterone producing activity and cell proliferating activity, and is considered to be one of mediators for various circulatory diseases. An angiotensin II antagonist which antagonizes angiotensin II for an angiotensin II receptor and suppresses the activity of angiotensin II is useful for preventing or treating circulatory diseases such as hypertension, cardiac diseases (e.g. heart failure, cardiac infarct etc.), cerebral stroke, nephritis and arteriosclerosis.
On the other hand, in the treatment of diabetes, therapy with a diabetic postplandial hyperglycemia improving drug, and therapy for improving reduction in insulin sensitivity to glucose uptake in peripheral tissues with an insulin sensitizer are used.
Further, in the treatment of hyperlipemia, a method of suppressing biosynthesis of cholesterol with HMG-Co A reductase (3-hydroxy-3-methylglutaryl coenzyme A reductase) inhibitor is used.
Inter alia, hypertension, impaired glucose tolerance and abnormality of lipid metabolism are known to easily complex with each other and, in particular, it is considered that hypertension and insulin resistance, or hypertension and arteriosclerosis exacerbate counterpart disease.

Disclosure of Invention

By combining an AII antagonist with drugs having other action mechanisms and formulating them into sustained-release medicines (e.g. sustained-release injectables etc.), the present invention aims at exerting considerably remarkable effects and compensating for various drawbacks observed when a drug is administered as a single agent, in various AII-mediated diseases, in particular, diseases alone such as hypertension, hyperlipemia, arteriosclerosis and diabetes or complexes thereof (e.g. thrombosis, menopausal disorder, cancer etc.).
The present inventors variously studied and, as a result, found that, by actually first using as a sustained-release preparation (e.g. sustained-release injectables etc.) with combining (A) an AII antagonist and (B) one or more drugs selected among remedies for hypertension, hypoglycemic drugs, remedies for hyperlipemia, antithrombotic drugs, remedies for menopause and anticancer drugs, considerably remarkable advantages which are not observed at administration of each single drug are offered in the natures needed as medicines such as pharmaceutical effects, safety, stability, dose, dosage form, usage and the like and, based on them, the present invention was completed.
That is, the present invention relates to:

[1] A sustained-release medicine comprising combining (A) an angiotensin II antagonist with (B) one or more drugs selected from a remedy for hypertension, a hypoglycemic drug, a remedy for hyperlipemia, an antithrombotic drug, a remedy for climacteric disturbance and an anticancer drug;
[2] The medicine according to the above-mentioned [1], wherein the angiotensin II antagonist is a compound represented by the formula (I):

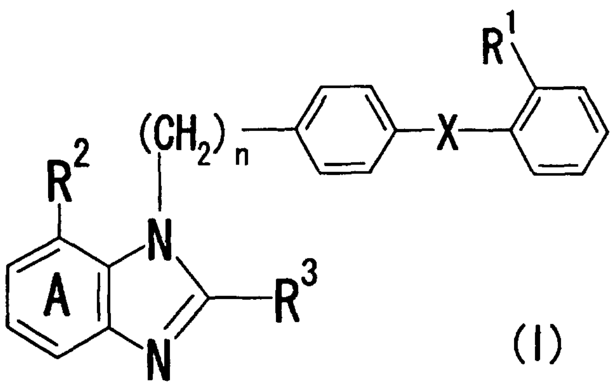

(I)

wherein $R^1$ represents a group capable of forming an anion or a group capable of converting to said gorup, X represents that a phenylene group and a phenyl group are linked directly or via a spacer having a chain of two or less of atoms, n represents 1 or 2, ring A represents a benzene ring which may further have a substituent, $R^2$ represents a group capable of forming an anion or a group capable of converting to said gorup, and $R^3$ represents a hydrocarbon group which may be bound through a hetero atom and may have a substituent, or a salt thereof;

[3] The medicine according to the above-mentioned [1], wherein the angiotensin II antagonist is Losartan, Eprosartan, Candesartan cilexetil, Candesartan, Valsartan, Telmisartan, Irebesartan, Olmesartan or Tasosartan;

[4] The medicine according to the above-mentioned [1], wherein the angiotensin II antagonist is 2-ethoxy-1-[[2'-(1H-tetrazol-5-yl)biphenyl-4-yl]methyl]benzimidazole-7-carboxylic acid or a salt thereof;

[5] The medicine according to the above-mentioned [1], wherein the angiotensin II antagonist is 1-(cyclohexyloxycarbonyloxy) ethyl 2-ethoxy-1-[[2'-(1H-tetrazol-5-yl)biphenyl-4-yl]methyl]benzimidazole-7-carboxylate or a salt thereof;

[6] The medicine according to the above-mentioned [1], wherein the angiotensin II antagonist is 2-ethoxy-1-[[2'-(2,5-dihydro-5-oxo-1,2,4-oxadiazol-3-yl)biphenyl-4-yl]methyl]benzimidazole-7-carboxylic acid or a salt thereof;

[7] The medicine according to the above-mentioned [1], wherein the remedy for hypertension is a drug selected from an angiotensin converting enzyme inhibitor, a diuretic, a calcium antagonist, a vasopressin antagonist, an angiotensin converting enzyme and neutral endopeptidase inhibitor, a β blocker and an aldosterone antagonist;

[8] The medicine according to the above-mentioned [7], wherein the angiotensin converting enzyme inhibitor is a drug selected from enalapril, cilazapril, tamocapril, trandolapril, lisinopril and ramipril;

[9] The medicine according to the above-mentioned [7], wherein the diuretic is a drug selected from indapamide, trichlormethiazide, bumetanide, hydrochlorothiazide and metolazone;

[10] The medicine according to the above-mentioned [7], wherein the calcium antagonist is a drug selected from amlodipine, nitrendipine and manidipine;

[11] The medicine according to the above-mentioned [7], wherein the vasopressin antagonist is a drug selected from tolvaptan, conivaptan hydrochloride and relcovaptan;

[12] The medicine according to the above-mentioned [7], wherein the angiotensin converting enzyme and neutral endopeptidase inhibitor are a drug selected from omapatrilat, fasidotril and sampatrilat ;

[13] The medicine according to the above-mentioned [7], wherein the β blocker is a drug selected from carvedilol, metoprolol and propranolol;

[14] The medicine according to the above-mentioned [7], wherein the aldrosterone antagonist is spironolactone;

[15] The medicine according to the above-mentioned [1], wherein the hypoglycemic drug is an insulin sensitizer, an insulin secretagogue or an insulin preparation;

[16] The medicine according to the above-mentioned [15], wherein the insulin sensitizer is a drug selected from pioglitazone hydrochloride, troglitazone and rosiglitazone maleate;

[17] The medicine according to the above-mentioned [15], wherein the insulin secretagogue is a drug selected from glibenclamide, nateglinide and repaglinide;

[18] The medicine according to the above-mentioned [1], wherein the remedy for hyperlipidemia is a statin medicament, a fibrate medicament or a nicotinic acid derivative;

[19] The medicine according to the above-mentioned [18], wherein the statin medicament is a drug selected from sodium cerivastatin, sodium pravastatin, simvastatin and calcium atrovastatin hydrate;

[20] The medicine according to the above-mentioned [18], wherein the fibrate medicament is fenofibrate, fenofibrinic acid, bezafibrate or gemfibrozil;

[21] The medicine according to the above-mentioned [18], wherein the nicotinic acid derivative is niceritrol or cholexamin;
[22] The medicine according to the above-mentioned [1], wherein the antithrombotic drug is a drug selected from a GPIIb/IIIa antagonist, low-molecular weight heparin, a thrombin inhibitor and an anti-platelet drug;
[23] The medicine according to the above-mentioned [22], wherein the GPIIb/IIIa antagonist is abciximab;
[24] The medicine according to the above-mentioned [22], the low molecular weight heparin is enoxaparin;
[25] The medicine according to the above-mentioned [22], wherein the thrombin inhibitor is argatroban;
[26] The medicine according to the above-mentioned [22], wherein the anti-platelet drug is clopidogrel sulfate or aspirin;
[27] The medicine according to the above-mentioned [1], wherein the remedy for climacteric disturbance is an estrogen selected from estradiol, estradiol valerate and conjugated estrogen;
[28] The medicine according to the above-mentioned [1], wherein the anticancer drug is a GnRH agonist or antagonist;
[29] The medicine according to the above-mentioned [28], wherein the GnRH agonist is leuprorelin or a salt thereof;
[30] The medicine according to the above-mentioned [1], which contains (A) an angiotensin II antagonist and (B) one or more drugs selected from a remedy for hypertension, a hypoglycemic drug, a remedy for hyperlipemia, an antithrombotic drug, a remedy for climacteric disturbance and an anticancer drug;
[31] The medicine according to the above-mentioned [1], which contains (A) a sustained-release preparation containing an angiotensin II antagonist and (B) a sustained-release preparation containing one or more drugs selected from a remedy for hypertension, a hypoglycemic drug, a remedy for hyperlipemia, an antithrombotic drug, a remedy for climacteric disturbance and an anticancer drug;
[32] The medicine according to the above-mentioned [1], which comprises a combination of (A) a sustained-release preparation containing an angiotensin II antagonist and (B) a sustained release preparation containing one or more drugs selected from a remedy for hypertension, a hypoglycemic drug, a remedy for hyperlipemia, an antithrombotic drug, a remedy for climacteric disturbance and an anticancer drug;
[33] The medicine according to the above-mentioned [1], which contains (C) a biodegradable polymer;
[34] The medicine according to the above-mentioned [33], wherein the biodegradable polymer is an $\alpha$-hydroxycarboxylic acid polymer;
[35] The medicine according to the above-mentioned [34], wherein the $\alpha$-hydroxycarboxylic acid polymer is a lactic acid-glycolic acid polymer;
[36] The medicine according to the above-mentioned [35], wherein a molar ratio of lactic acid to glycolic acid is 100/0 to 40/60;
[37] The medicine according to the above-mentioned [34], wherein a weight average molecular weight of the polymer is 3,000 to 50,000;
[38] The medicine according to the above-mentioned [1], which is for injection;
[39] The medicine according to the above-mentioned [1], which is an agent for prevention or treatment of circulatory diseases;
[40] The medicine according to the above-mentioned [1], which is an agent for prevention or treatment of hypertension;
[41] The medicine according to the above-mentioned [1], which is an agent for prevention or treatment of blood pressure circadian rhythm abnormality;
[42] The medicine according to the above-mentioned [1], which is an agent for prevention or treatment of organ disorder;
[43] The medicine according to the above-mentioned [1], which is an agent for prevention or treatment of cancer;
[44] The medicine according to the above-mentioned [1], which is an agent for protecting organs;
[45] A method for treating circulatory disease, hypertension, blood pressure circadian rhythm abnormality, organ disorder or cancer, which comprises administering the medicine according to the above-mentioned [1] to a mammal;
[46] A use of the medicine according to the above-mentioned [1] for preparing a medicament for treatment of circulatory disease, hypertension, blood pressure circadian rhythm abnormality, organ disorder or cancer; and
[47] A sustained-release medicine comprising a combination of two or three drugs selected from an angiotensin II antagonist, a remedy for hypertension, a hypoglycemic drug and a remedy for hyperlipemia.

The angiotensin II antagonism possessed by the AII antagonist in the present invention refers to the activity which competitively or non-competitively inhibits binding of angiotensin II to an angiotensin II receptor on a cell membrane, attenuates the potent vasoconstriction activity and vascular smooth muscle proliferating activity induced by angiotensin II, and alleviates the symptom of hypertension.
The AII antagonist used in the present invention may be peptidic or non-peptidic, and a compound having a non-peptidic antagonism which has an advantage of long duration of action is preferable. As the compound having the

angiotensin II antagonism, a compound having an oxygen atom in the molecule is preferable, inter alia, a compound having an ether linkage or a carbonyl group (the carbonyl group may form a hydroxy group by resonance) is preferable, a compound having an ether linkage or a ketone derivative is more preferable and, inter alia, an ether derivative is preferable.

The compound having a non-peptidic angiotensin II antagonism is not particularly limited, and imidazole derivatives disclosed in JP 56-71073 A, JP 56-71074 A, JP 57-98270 A, JP 58-157768 A, USP 4,355,040 and USP 4,340,598 and the like, imidazole derivatives disclosed in EP-253310, EP-291969, EP-324377, EP-403158, WO-9100277, JP 63-23868 A, JP 1-117876 A and the like, pyrrole, pyrazole and triazole derivatives disclosed in USP 5,183,899, EP-323841, EP-409332, JP 1-287071 A and the like, benzimidazole derivatives disclosed in USP 4,880,804, EP-0392317, EP-0399732, EP-0400835, EP-425921, EP-459136, JP 3-63264 A and the like, azaindene derivatives disclosed in EP-399731 and the like, pyrimidone derivatives disclosed in EP-407342 and the like, quinazoline derivatives disclosed in EP-411766 and the like, xanthine derivatives disclosed in EP-430300 and the like, fused imidazole derivatives disclosed in EP-434038 and the like, pyrimidinedione derivatives disclosed in EP-442473 and the like, thienopyridone derivatives disclosed in EP-443568 and the like, heterocyclic compounds disclosed in EP-445811, EP-483683, EP-518033, EP-520423, EP-588299, EP-603712 and the like, and compounds described in Journal of Medicinal Chemistry, Vol. 39, No. 3, pp.625-656, 1996 are used. As the compound having a non-peptidic angiotensin II antagonism, any compounds may be used as far as they are non-peptidic compounds having an angiotensin II antagonism besides the non-peptidic compounds described in the aforementioned known references. Inter alia, Losartan (DuP753), potassium Losartan, Eprosartan (SK & F108566), Candesartan cilexetil (TCV-116), Valsartan (CGP-48933), Telmisartan (BIBR277), Irbesartan (SR47436), Tasosartan (ANA-756), olmesartan medoxomil and their metabolic active substances (Candesartan, olmesartan etc) are preferably used.

In addition, as the non-peptidic compound having an angiotensin II antagonism, for example, a benzimidazole derivative represented by the formula (I):

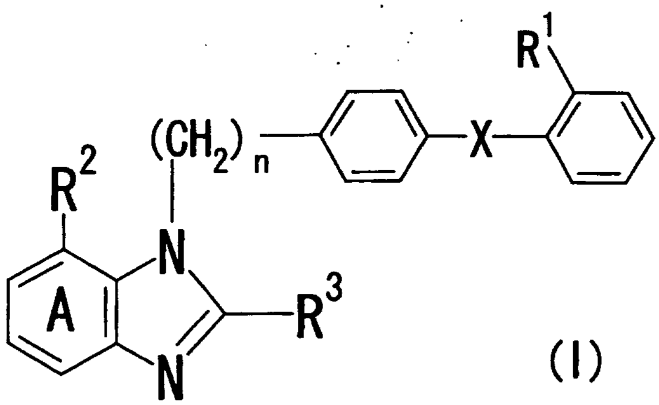

(I)

wherein $R^1$ represents a group capable of forming an anion or a group capable of converting to said gorup, X represents that a phenylene group and a phenyl group are linked directly or via a spacer having a chain of two or less of atoms, n represents 1 or 2, ring A represents a benzene ring which may further have a substituent, $R^2$ represents a group capable of forming an anion or a group capable of converting to said gorup and $R^3$ represents a hydrocarbon residue which may be bound through a hetero atom and may have a substituent (preferably, a hydrocarbon residue which may have a substituent and is linked via an oxygen atom), or a salt thereof is preferably used.

In the above formula (I), examples of a group capable of forming an anion (a group having a hydrogen atom which can be released as a proton) for $R^1$ include (1) a carboxyl group, (2) a tetrazolyl group, (3) a trifluoromethanesulfonic acid amide group ($-NHSO_2CF_3$), (4) a phosphoric acid group, (5) a sulfonic acid group, and (6) a 5 to 7-membered (preferably 5 to 6-membered) monocyclic optionally substituted heterocyclic residue containing one or more of N, S and O.

Examples of the "5 to 7-membered (preferably 5 to 6-membered) monocyclic optionally substituted heterocyclic residue containing one or more of N, S and O" include

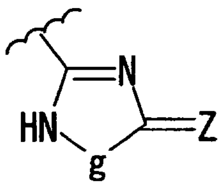 , 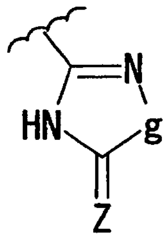 , 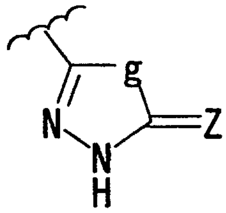 , 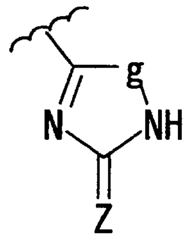 ,

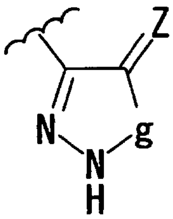 , 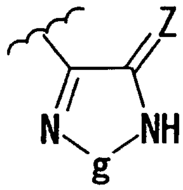 , 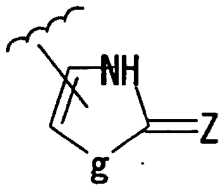 , 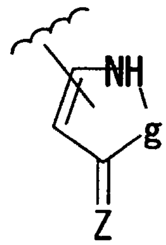 ,

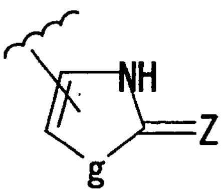 , 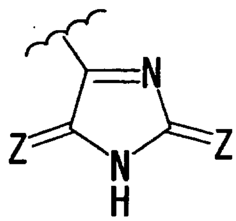 , 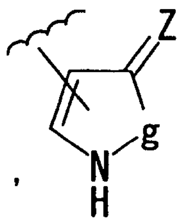 , 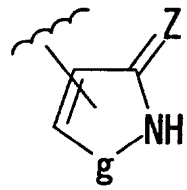 ,

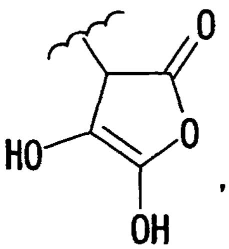 , 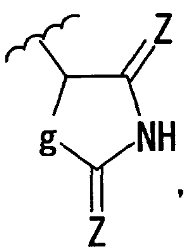 , 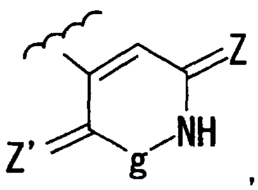 , 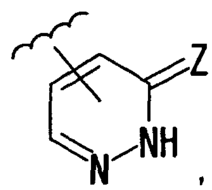 ,

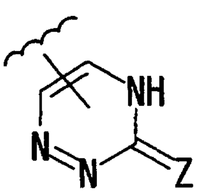 , 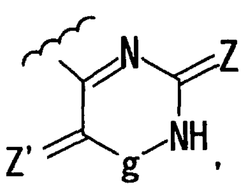 , 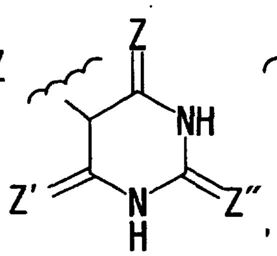 , 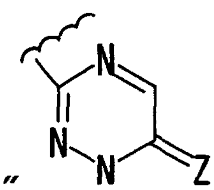 ,

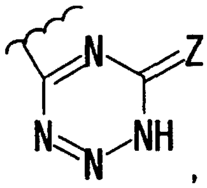 , 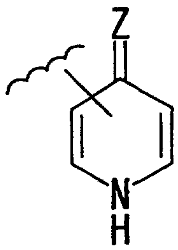 , 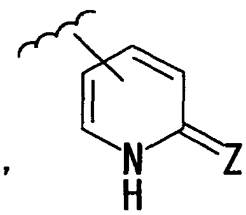 , 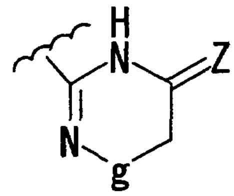 ,

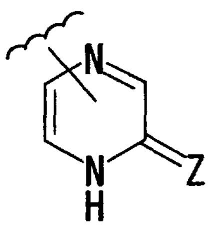 , 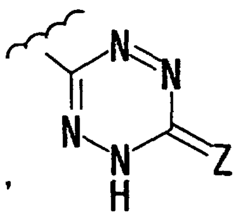 , 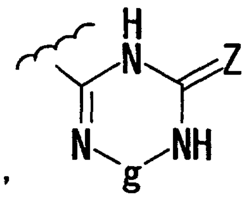 , 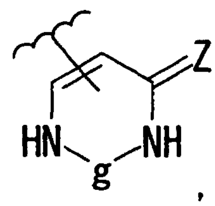 ,

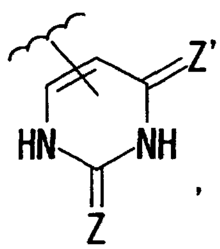 , 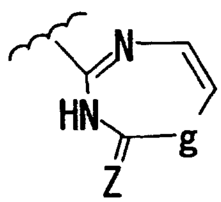 , 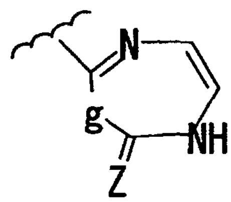 , 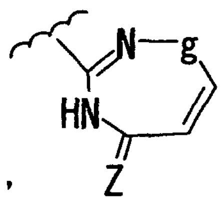

, and when g in the above formula represents -NH- and the like, the binding between a heterocyclic residue represented by $R^1$ and a phenyl group to which the heterocyclic residue is linked may be bound via one of plural existing nitrogen atoms, in addition to the aforementioned carbon-carbon binding. For example, when $R^1$ is represented by

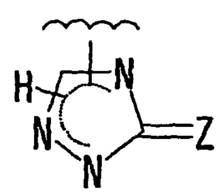 ,

specifically, the binding represents :

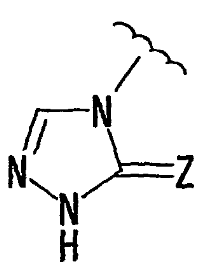 , 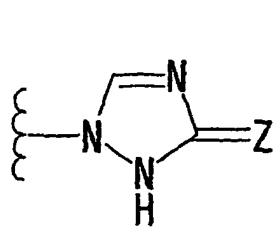 , 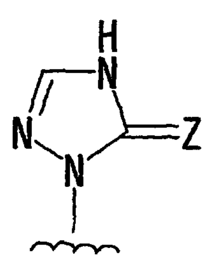 or 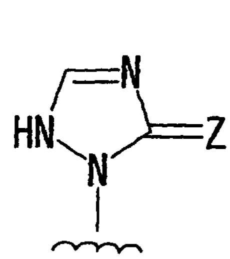

Other examples of binding via a nitrogen atom include:

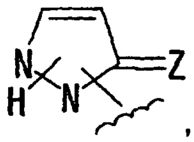 , 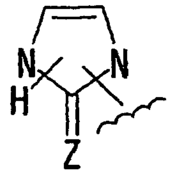 , 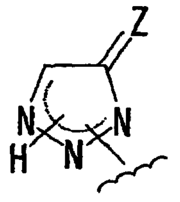 , 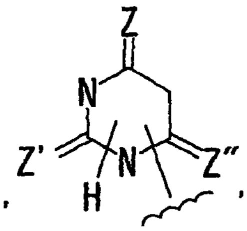 ,

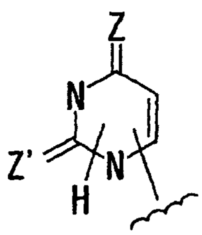 , 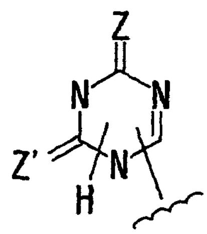 , 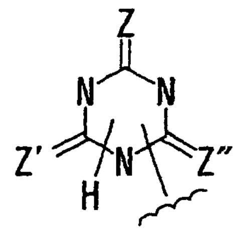

In the above formula, g represents $-CH_2-$, -NH-, -O- or $-S(O)_m-$, >= Z, >= Z' and >= Z" represent a carbonyl group, a thiocarbonyl group or an optionally oxidized sulfur atom (e.g. S, S(O), $S(O)_2$ etc.) (preferably carbonyl or thiocarbonyl group, more preferably carbonyl group), respectively, and m represents an integer of 0, 1 or 2.

As the heterocyclic residue represented by $R^1$, for example, a group having at the same time a -NH- group or a -OH group as a proton donor and a carbonyl group, a thiocarbonyl group or a sulfinyl group as a proton acceptor, such as an oxadiazolone ring, an oxadiazolothione ring and a thiadiazolone ring, is preferred. In addition, the hetercyclic residue represented by $R^1$ may form a condensed ring by binding with a cyclic substituent and, as the heterocyclic residue represented by $R^1$, a 5 to 6-membered ring residue is preferable, and a 5-membered ring residue is more preferable.

As a heterocyclic residue represented by $R^1$, a group represented by the formula:

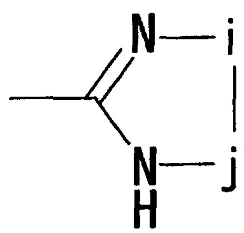

wherein i represents -O- or -S-, and j represents >=O, >=S or $>=S(O)_m$, m is as defined above, (inter alia, 2,5-dihydro-5-oxo-1,2,4-oxadiazol-3-yl, 2,5-dihydro-5-thioxo-1,2,4-oxadiazol-3-yl, 2,5-dihydro-5-oxo-1,2,4-thiadiazol-3-yl, particularly, 2,5-dihydro-5-oxo-1,2,4-oxadiazol-3-yl) is preferred.

In addition, there are tautomers in the above-mentioned heterocyclic residue ($R^1$) as shown below. For example, when Z is O and g is O in

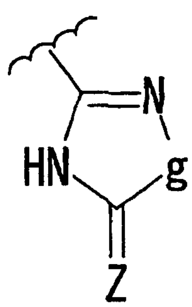

there are three tautomers of a', b' and c' of

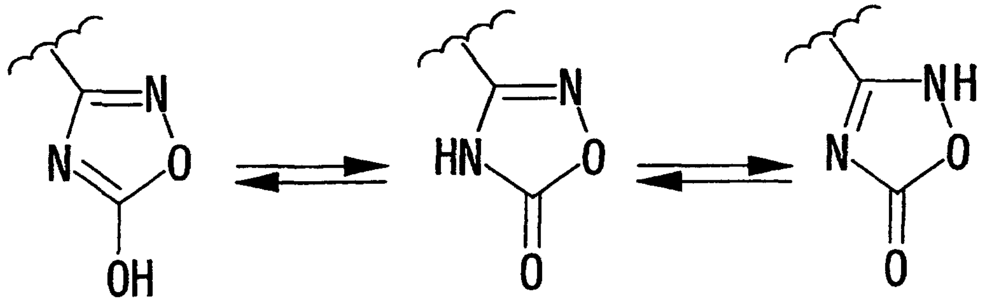

a' b' c'

, and the heterocyclic residue represented by the formula:

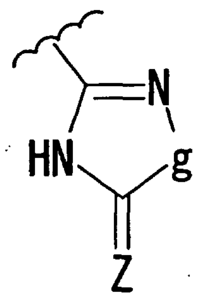

includes all of a', b' and c'.

The group capable of forming an anion for $R^1$ may be protected with an optionally substituted lower ($C_{1-4}$) alkyl group or acyl group (e.g. lower ($C_{2-5}$)alkanoyl, benzoyl etc.) at a substitutable position.

Examples of the optionally substituted lower ($C_{1-4}$) alkyl group include (1) a lower ($C_{1-4}$)alkyl group optionally substituted with 1 to 3 phenyl groups which may have a halogen atom, nitro, lower ($C_{1-4}$) alkyl, lower ($C_{1-4}$) alkoxy and the like (e.g. methyl, triphenylmethyl, p-$_4$) methoxybenzyl, p-nitrobenzyl etc.), (2) a lower ($C_{1-4}$) alkoxy-lower ($C_{1-4}$)alkyl group (e.g. methoxymethyl, ethoxymethyl etc.), and (3) a group represented by the formula -CH($R^4$)-OCO$R^5$ [wherein $R^4$ represents (a) hydrogen, (b) a straight or branched lower alkyl group having 1 to 6 carbons(e.g. methyl, ethyl, n-propyl, isopropyl, n-butyl, isobutyl, t-butyl, n-pentyl, isopentyl, neopentyl etc.), (c) a straight or branched lower alkenyl group having 2 to 6 carbons or (d) a cycloalkyl group having 3 to 8 carbons (e.g. cyclopentyl, cyclohexyl, cycloheptyl etc.), and $R^5$ represents (a) a straight or branched lower alkyl group having 1 to 6 carbons (e.g. methyl, ethyl, n-propyl, isopropyl, n-butyl, isobutyl, sec-butyl, t-butyl, n-pentyl, isopentyl, neopentyl etc.), (b) a straight or branched lower alkenyl group having 2 to 6 carbons, (c) a lower alkyl group having 1 to 3 carbons which is substituted with a cycloalkyl group having 3 to 8 carbons (e.g. cyclopentyl, cyclohexyl, cycloheptyl etc.) or an optionally substituted aryl group (e.g. phenyl or naphthyl group which may have halogen atom, nitro, lower ($C_{1-4}$)alkyl, lower ($C_{1-4}$)alkoxy etc.) (e.g. benzyl, p-chlorobenzyl, phenethyl, cyclopentylmethyl, cyclohexylmethyl etc.), (d) a lower alkenyl group having 2 to 3 carbons which is substituted with cycloalkyl having 3 to 8 carbons or an optionally substituted aryl group (e.g. phenyl or naphthyl group which may have halogen atom, nitro, lower ($C_{1-4}$)alkyl, lower ($C_{1-4}$) alkoxy etc.) (e.g. a group having an alkenyl moiety such as vinyl, propenyl, allyl, isopropenyl etc., such as cinnamyl etc.), (e) an optionally substituted aryl group (e.g. phenyl or naphthyl group which may have halogen atom, nitro, lower ($C_{1-4}$)alkyl, lower ($C_{1-4}$)alkoxy etc., such as phenyl, p-tolyl, naphthyl etc), (f) a straight or branched lower alkoxy group having 1 to 6 carbons (e.g. methoxy, ethoxy, n-propoxy, isopropoxy, n-butoxy, isobutoxy, sec-butoxy, t-butoxy, n-pentyloxy, isopentyloxy, neopentyloxy etc.), (g) a straight or branched lower alkenyloxy group having 2 to 8 carbons (e.g. allyloxy, isobutenyloxy etc.), (h) a cycloalkyloxy group having 3 to 8 carbons (e.g. cyclopentyloxy, cyclohexyloxy, cycloheptyloxy etc,), (i) a lower alkoxy group having 1 to 3 carbons which is substituted with cycloalkyl having 3 to 8 carbons (e.g. cyclopentyl, cyclohexyl, cycloheptyl etc.)

or an optionally substituted aryl group (e.g. phenyl or naphthyl group which may have halogen atom, nitro, lower ($C_{1-4}$) alkyl, lower ($C_{1-4}$) alkoxy etc,) (e.g. a group having an alkoxy moiety such as methoxy, ehoxy, n-propoxy, isopropoxy etc., such as benzyloxy, phenethyloxy, cyclopentylmethoxy, cyclohexylmethoxy etc.), (j) a lower alkenyloxy group having 2 to 3 carbons which is substituted with cycloalkyl having 3 to 8 carbons (e.g. cyclopentyl, cyclohexyl, cycloheptyl etc.) or an optionally substituted aryl group (e.g. phenyl or naphthyl group which may have halogen atom, nitro, lower ($C_{1-4}$)alkyl, lower ($C_{1-4}$)alkoxy etc.) (e.g. a group having an alkenyloxy moiety such as vinyloxy, propenyloxy, allyloxy, isopropenyloxy etc, such as cinnamyloxy etc.) or (k) an optionally substituted aryloxy group (e.g. phenoxy or naphthoxy group which may have halogen atom, nitro, lower ($C_{1-4}$)alkyl, lower ($C_{1-4}$)alkoxy etc., such as phenoxy, p-nitrophenoxy, naphthoxy etc.)].

In addition, the group capable of forming an anion for $R^1$ may have substituents such as an optionally substituted lower ($C_{1-4}$)alkyl group (examples thereof are exemplified by those for the "optionally substituted lower ($C_{1-4}$) alkyl group" as a protective group for a group capable of forming an anion for $R^1$), halogen atom, nitro, cyano, lower ($C_{1-4}$) alkoxy, and amino which may be substituted with 1 to 2 lower ($C_{1-4}$)alkyls at a replaceable position, in addition to protective groups such as the aforementioned optionally substituted lower ($C_{1-4}$)alkyl group and acyl group (e.g. lower ($C_{2-5}$)alkanoyl, benzoyl etc.).

In the above formula, the group capable of converting to a group capable of forming an anion for $R^1$ (a group having a hydrogen atom which can be released as a proton) may be a group which can be converted to a group capable of forming an anion (so-called prodrug) under a biological, that is, physiological condition (e.g. in vivo reaction such as oxidation, reduction and hydrolysis by an enzyme in a living body), or a group which can be converted to a group capable of forming an anion represented by $R^1$ (so-called synthetic intermediate) by a chemical reaction, such as cyano, N-hydroxycarbamimidoyl group ($-C(=N-OH)-NH_2$), or (1) a carboxyl group, (2) a tetrazolyl group, (3) a trifluoromethanesulfonic acid amide group ($-NHSO_2CF_3$), (4) a phosphoric acid group, (5) a sulfonic acid group, and (6) a 5 to 7-membered (preferably 5 to 6-membered) monocyclic optionally substituted heterocyclic residue containing one or more of N, S and O, each of which is protected with an optionally substituted lower ($C_{1-4}$)alkyl group or acyl group.

As $R^1$, carboxyl, tetrazolyl or 2,5-dihydro-5-oxo-1,2,4-oxadiazol-3-yl (preferably tetrazolyl), cyano, or N-hydroxycarbamimidoyl (preferably cyano), each of which may be protected with optionally substituted lower ($C_{1-4}$)alkyl (e.g. methyl, triphenylmethyl, methoxymethyl, ethoxymethyl, p-methoxybenzyl, p-nitrobenzyl etc.) or acyl group (e.g. lower ($C_{2-5}$)alkanoyl, benzoyl etc.), is preferable and, inter alia, tetrazolyl is preferably used.

In the above formula, X represents that adjacent phenylene group and phenyl group are linked directly or via a spacer having a chain of two or less of atoms (preferably direct bond). Any spacers having a chain of two or less of atoms may be used as far as they are a divalent.chain in which the number of atoms constituting a straight chain moiety is 1 or 2, and spacers may have a side chain. Specific examples include lower ($C_{1-4}$)alkylene, -CO-, -O-,-S-, -NH-, -CO-NH-, $-O-CH_2-$, $-S-CH_2-$, and -CH=CH-, in which the number of atoms constituting a straight chain moiety is 1 or 2.

In the above formula, n represents an integer of 1 or 2 (preferably 1).

In the above formula, ring A represents a benzene ring which may further have a substituent in addition to substituent $R^2$, and examples of the substituent include (1) halogen (e.g. F, Cl, Br etc.), (2) cyano, (3) nitro, (4) optionally substituted lower ($C_{1-4}$)alkyl, (5) lower ($C_{1-4}$) alkoxy, (6) optionally substituted amino group (e.g. amino, N-lower ($C_{1-4}$) alkylamino (e.g. methylamino etc.), N,N-di-lower ($C_{1-4}$)alkylamino (e.g. dimethylamino etc.), N-arylamino (e.g. phenylamino etc.), alicyclic amino (e.g morpholino, piperidino, piperazino, N-phenylpiperazino etc.) etc.), (7) a group represented by the formula -CO-D' [wherein D' represents a hydroxy group, or lower ($C_{1-4}$) alkoxy in which the alkyl moiety may be substituted with a hydroxy group, lower ($C_{1-4}$)alkoxy, lower ($C_{2-6}$)alkanoyloxy (e.g. acetoxy, pivaloyloxy etc.), lower ($C_{1-6}$) alkoxycarbonyloxy (e.g. methoxycarbonyloxy, ethoxycarbonyloxy etc.) or lower ($C_{3-6}$) cycloalkoxycarbonyloxy (e.g: cyclohexyloxycarbonyloxy etc.)], or (8) tetrazolyl, trifluoromethanesulfonic acid amide group, a phosphoric acid group or a sulfonic acid group, each of which may be protected with optionally substituted lower ($C_{1-4}$)alkyl (examples thereof are exemplified by those for the "optionally substituted lower ($C_{1-4}$)alkyl group" as a protective group for a group capable of forming an anion for $R^1$) or acyl (e.g. lower ($C_{2-5}$) alkanoyl, benzoyl etc.).

These substituents may be substituted at 1 to 2 substitutable positions on a benzene ring. As the substituent possessed further by ring A in addition to the substituent $R^2$, an optionally substituted lower ($C_{1-4}$)alkyl (e.g. lower ($C_{1-4}$)alkyl which may be substituted with hydroxy group, carboxyl group, halogen, etc.) and halogen are preferable. It is more preferable that ring A has no substituent other than substituent $R^2$.

In the above formula, examples of the group capable of forming an anion (a group having a hydrogen atom which can be released as a proton) for $R^2$ include (1) an optionally esterified or amidated carboxyl group, (2) a tetrazolyl group, (3) a trifluoromethanesulfonic acid amide group ($-NHSO_2CF_3$), (4) a phosphoric acid group, and (5) a sulfonic acid group. These groups may be protected with an optionally substituted lower alkyl group (examples thereof are exemplified by those for the "optionally substituted lower ($C_{1-4}$)alkyl group" as a protective group for a group capable of forming an anion for $R^1$) or an acyl group (e.g. lower ($C_{2-5}$)alkanoyl, benzoyl etc.), and may be any groups as far as they are a group capable of forming an anion or a group capable of converting to said group under a biological, that is, physiological condition (e.g. in vivo reaction such as oxidation, reduction and hydrolysis by an enzyme in a living

body), or chemically.

Examples of the optionally esterified or amidated carboxyl as $R^2$ include a group represented by the formula-CO-D [wherein D represents (1) a hydroxy group, (2) optionally substituted amino (e.g. amino, N-lower ($C_{1-4}$) alkylamino, N,N-di-lower ($C_{1-4}$)alkylamino etc.) or (3) optionally substituted alkoxy {e.g. (i) a lower ($C_{1-6}$)alkoxy group in which an alkyl moiety may be substituted with a hydroxy group, optionally substituted amino (e.g. amino, N-lower ($C_{1-4}$) alkylamino, N,N-di-lower ($C_{1-4}$)alkylamino, piperidino, morpholino etc.), halogen, lower ($C_{1-6}$)alkoxy, lower ($C_{1-6}$) alkylthio, lower ($C_{3-8}$)cycloalkoxy or optionally substituted dioxolenyl (e.g. 5-methyl-2-oxo-1,3-dioxolen-4-yl etc.), or (ii) a group represented by the formula -O-CH($R^6$)-OCOR$^7$ [wherein $R^6$ represents (a) hydrogen, (b) a straight or branched lower alkyl group having 1 to 6 carbons (e.g. methyl, ethyl, n-propyl, isopropyl, n-butyl, isobutyl, t-butyl, n-pentyl, isopentyl, neopentyl etc.), (c) a straight or branched lower alkenyl group having 2 to 6 carbons, or (d) a cycloalkyl group having 3 to 8 carbons (e.g. cyclopentyl, cyclohexyl, cycloheptyl etc.), and $R^7$ represents (a) a straight or branched lower alkyl group having 1 to 6 carbons (e.g. methyl, ethyl, n-propyl, isopropyl, n-butyl, isobutyl, sec-butyl, t-butyl, n-pentyl, isopentyl, neopentyl etc.), (b) a straight or branched lower alkenyl group having 2 to 6 carbons, (c) a lower alkyl group having 1 to 3 carbons which is substituted with a cycloalkyl group having 3 to 8 carbons (e.g. cyclopentyl, cyclohexyl, cycloheptyl etc.) or an optionally substituted aryl group (e.g. phenyl or naphthyl group which may have halogen atom, nitro, lower ($C_{1-4}$)alkyl, lower ($C_{1-4}$)alkoxy etc.) (e.g. benzyl, p-chlorobenzyl, phenethyl, cyclopentylmethyl, cyclohexylmethyl etc.), (d) a lower alkenyl group having 2 to 3 carbons which is substituted with cycloalkyl having 3 to 8 carbons or an optionally substituted aryl group (e.g. phenyl or naphthyl group which may have halogen atom, nitro, lower ($C_{1-4}$)alkyl, lower ($C_{1-4}$) alkoxy etc.) (e.g. a group having an alkenyl moiety such as vinyl, propenyl, allyl, isopropenyl etc., such as cinnamyl etc.), (e) an optionally substituted aryl group (e.g. phenyl or naphthyl group which may have halogen atom, nitro, lower ($C_{1-4}$)alkyl, lower ($C_{1-4}$)alkoxy etc., such as phenyl, p-tolyl, naphthyl etc.), (f) a straight or branched lower alkoxy group having 1 to 6 carbons (e.g. methoxy, ethoxy, n-propoxy, isopropoxy, n-butoxy, isobutoxy, sec-butoxy, t-butoxy, n-pentyloxy, isopentyloxy, neopentyloxy etc.), (g) a straight or branched lower alkenyloxy group having 2 to 8 carbons (e.g. allyloxy, isobutenyloxy etc.), (h) a cycloalkyloxy group having 3 to 8 carbons (e.g. cyclopentyloxy, cyclohexyloxy, cycloheptyloxy etc.), (i) a lower alkoxy group having 1 to 3 carbons which is substituted with cycloalkyl having 3 to 8 carbons (e.g. cyclopentyl, cyclohexyl, cycloheptyl etc.) or an optionally substituted aryl group (e.g. phenyl or naphthyl group which may have halogen group, nitro, lower ($C_{1-4}$)alkyl, lower ($C_{1-4}$) alkoxy etc.) (e.g. a group having an alkoxy moiety such as methoxy, ethoxy, n-propoxy, isopropoxy etc., such as benzyloxy, phenethyloxy, cyclopentylmethoxy, cyclohexylmethoxy etc.), (j) a lower alkenyloxy having 2 to 3 carbons which is substituted with cycloalkyl having 3 to 8 carbons (e.g. cyclopentyl, cyclohexyl, cycloheptyl etc.) or an optionally substituted aryl group (e.g. phenyl or naphthyl group which may have halogen atom, nitro, lower ($C_{1-4}$)alkyl, lower ($C_{1-4}$)alkoxy etc.) (e.g. a group having an alkenyloxy moiety such as vinyloxy, propenyloxy, allyloxy, isopropenyloxy etc., such as cinnamyloxy etc.) or (k) an optionally substituted aryloxy group (e.g. phenoxy or naphthoxy group which may have halogen atom, nitro, lower ($C_{1-4}$)alkyl, lower ($C_{1-4}$)alkoxy etc., such as phenoxy, p-nitrophenoxy, naphthoxy etc.)}].

As $R^2$, an optionally esterified carboxyl is preferable. Specific examples thereof include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyryloxymethoxycarbonyl, isobutyryloxymethoxycarbonyl, 1-(ethoxycarbonyloxy)ethoxycarbonyl, 1-(acetoxy)ethoxycarbonyl, 1-(isobutyryloxy)ethoxycarbonyl, cyclohexylcarbonyloxymethoxycarbonyl, benzoyloxymethoxycarbonyl, cinnamyloxycarbonyl, cyclopentylcarbonyloxymethoxycarbonyl and the like, and may be any groups as far as they are a group capable of forming an anion (e.g. $COO^-$, a derivative thereof etc.) or a group capable of converting to said gorup under a biological, that is, physiological condition (e.g. in vivo reaction such as oxidation, reduction and hydrolysis by an enzyme in a living body) or chemically, or may be a carboxyl group, or a prodrug thereof.

As the aforementioned $R^2$, a group represented by the formula-CO-D [wherein D represents (1) a hydroxy group or (2) a lower ($C_{1-4}$)alkoxy in which the alkyl moiety may be substituted with hydroxy group, amino, halogen, lower ($C_{2-6}$) alkanoyloxy (e.g. acetoxy, pivaloyloxy etc.), lower ($C_{3-8}$) cycloalkanoyloxy, lower ($C_{1-6}$)alkoxycarbonyloxy (e.g. methoxycarbonyloxy, ethoxycarbonyloxy etc.), lower ($C_{3-8}$) cycloalkoxycarbonyloxy (e.g. cyclohexyloxycarbonyloxy etc.), lower ($C_{1-4}$)alkoxy or lower ($C_{3-8}$)cycloalkoxy] is preferable and, inter alia, carboxyl is preferable.

In the above formula, examples of the "hydrocarbon residue" in the "hydrocarbon residue which may be bound through a hetero atom and may have a substituent" represented by $R^3$ include (1) an alkyl group, (2) an alkenyl group, (3) an alkynyl group, (4) a cycloalkyl group, (5) an aryl group, and (6) aralkyl group and, inter alia, an alkyl group, an alkenyl group and a cycloalkyl group are preferable.

The alkyl group of aforementioned (1) may be a straight or branched lower alkyl group having around 1 to 8 carbons, and for example, methyl, ethyl, propyl, isopropyl, butyl, isobutyl, sec-butyl, t-butyl, pentyl, i-pentyl, hexyl, heptyl and octyl are exemplified.

The alkenyl group of aforementioned (2) may be a straight or branched lower alkenyl group having around 2 to 8 carbons, and for example, vinyl, propenyl, 2-butenyl, 3-butenyl, isobutenyl and 2-octenyl are exemplified.

The alkynyl group of aforementioned (3) may be a straight or branched lower alkynyl group having around 2

to 8 carbons, and for example, ethynyl, 2-propynyl, 2-butynyl, 2-pentynyl, and 2-octynyl are exemplified.

The cycloalkyl group of aforementioned (4) includes, for example, a lower cycloalkyl having around 3 to 6 carbons, and cyclopropyl, cyclobutyl, cyclopentyl, cyclohexyl, etc. are exemplified.

The aformentioned alkyl group, alkenyl group, alkynyl group or cycloalkyl group may be substituted with a hydroxy group, an optionally substituted amino group (e.g. amino, N-lower ($C_{1-4}$)alkylamino, N,N-di-lower ($C_{1-4}$) alkylamino etc.), halogen, lower ($C_{1-4}$)alkoxy group, or lower ($C_{1-4}$)alkylthio group.

Examples of the aralkyl group of aforementioned (5) include phenyl-lower ($C_{1-4}$)alkyl such as benzyl and phenethyl, and examples of the aryl group of aforementioned (6) include phenyl.

The aforementioned aralkyl group or aryl group may have, for example, halogen (e.g. F, Cl, Br etc.), nitro, optionally substituted amino group (e.g. amino, N-lower ($C_{1-4}$)alkylamino, N,N-di-lower ($C_{1-4}$)alkylamino etc.), lower ($C_{1-4}$)alkoxy (e.g. methoxy, ethoxy etc.), lower ($C_{1-4}$) alkylthio (e.g. methylthio, ethylthio etc.), or lower ($C_{1-4}$) alkyl (e.g. methyl, ethyl etc.) at an arbitrary position on the benzene ring thereof.

Among the foregoing, as the "hydrocarbon residue" in the "hydrocarbon residue which may be bound through a hetero atom and may have a substituent" represented by $R^3$, an optionally substituted alkyl or alkenyl group (e.g. lower ($C_{1-5}$)alkyl or lower ($C_{2-5}$)alkenyl group which may be substituted with hydroxy group, amino group, halogen or lower ($C_{1-4}$)alkoxy group etc.) is preferable and, inter alia, lower ($C_{1-5}$)alkyl (more preferably ethyl) is preferable.

Examples of the "hetero atom" in the "hydrocarbon residue which may be bound through a hetero atom and may have a substituent" represented by $R^3$ include -O-, -S(O)$_m$- [m represents an integer of 0 to 2], and -NR'- [R' represents a hydrogen atom or lower ($C_{1-4}$)alkyl] and, inter alia, -O- is preferably used.

Among the foregoing, as $R^3$, a lower ($C_{1-5}$) alkyl or lower ($C_{2-5}$)alkenyl group which may be bound through -O-,-S(O)$_m$- [m represents an integer of 0 to 2] or -NR'- [R' represents hydrogen atom or lower ($C_{1-4}$)alkyl] and may be substituted with a substituent selected from hydroxy group, amino group, halogen and lower ($C_{1-4}$)alkoxy group is preferable and, inter alia, lower ($C_{1-5}$)alkyl or lower ($C_{1-5}$) alkoxy (more preferably ethoxy) is preferable.

Among the non-peptidic compound having angiotensin II antagonism represented by the formula (I), a benzimidazole-7-carboxylic acid derivative represented by the formula (I') :

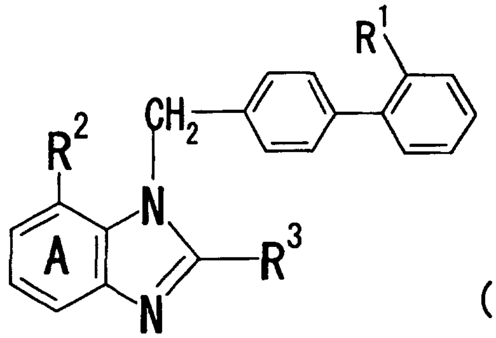

(I')

wherein $R^1$ represents (1) a carboxyl group, (2) a tetrazolyl group or (3) a group represented by the formula:

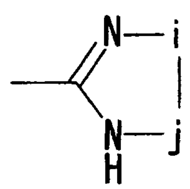

wherein i represents -O- or -S-, j represents >=S, >=S or >=S(O)$_m$, and m is as defined above,
ring A represents a benzene ring which may be substituted with lower ($C_{1-4}$)alkyl (e.g. lower ($C_{1-4}$)alkyl which may be substituted with hydroxy group, carboxyl group, halogen etc.) or halogen in addition to substituent $R^2$ (preferably a benzene ring having no substituent other than substituent $R^2$), $R^2$ represents a group represented by the formula -CO-D [wherein D represents (1) hydroxy group or (2) lower ($C_{1-4}$) alkoxy in which the alkyl moiety may be substituted with hydroxy group, amino, halogen, lower ($C_{2-6}$) alkanoyloxy (e.g. acetoxy, pivaloyloxy etc.), lower ($C_{3-8}$)cycloalkanoyloxy, lower ($C_{1-6}$)alkoxycarbonyloxy (e.g. methoxycarbonyloxy, ethoxycarbonyloxy etc.), lower ($C_{3-8}$)cycloalkoxycarbonyloxy (e.g. cyclohexyloxycarbonyloxy etc.), lower ($C_{1-4}$)alkoxy or lower ($C_{3-8}$)cycloalkoxy, and $R^3$ represents a lower ($C_{1-5}$) alkyl or lower ($C_{2-5}$)alkenyl group (preferably lower ($C_{1-5}$) alkyl or lower ($C_{1-5}$)alkoxy; more preferably ethoxy)

which may be bound through -O-, -S(O)$_m$- [m represents an integer of 0 to 2] or -NR'- [R' represents hydrogen atom or lower ($C_{1-4}$)alkyl] and may be substituted with a substituent selected from a hydroxy group, an amino group, a halogen and a lower ($C_{1-4}$) alkoxy group] or a pharmacologically acceptable salt thereof is preferable and, inter alia, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]benzimidazole-7-carboxylate [Candesartan cilexetil], pivaloyloxymethyl 2-ethoxy-1-[[2'-(1H-tetrazol-5-yl)biphenyl-4-yl]methyl]benzimidazole-7-carboxylate, and 2-ethoxy-1-[[2'-(2,5-dihydro-5-oxo-1,2,4-oxadiazol-3-yl)biphenyl-4-yl]methyl]benzimidazole-7-carboxylic acid or salts thereof are preferable.

The aforementioned benzimidazole derivative can be synthesized by the known method described, for example, in EP-425921, EP-459136, EP-553879, EP-578125, EP-520423, and EP-668272 or a similar method thereto. When Candesartan cilaxetil is used, it is better to use a stable C-type crystal described in EP-459136.

The compound having an angiotensin II antagonism used in the present invention or a prodrug thereof may be itself, or a pharmacologically acceptable salt. When the compound having an angiotensin II antagonism has an acidic group such as a carboxyl group and the like, examples of such the salt include salts with inorganic bases (e.g. alkali metal such as sodium, potassium etc., alkaline earth metal such as calcium, magnesium etc., and transition metal such as zinc, iron, copper etc.) and organic bases (e.g. organic amines such as trimethylamine, triethylamine, pyridine, picoline, ethanolamine, diethanolamine, triethanolamine, dicyclohexylamine, and N,N'-dibenzylethylenediamine, and basic amino acids such as arginine, lysine, and ornithine). When the compound having an angiotensin II antagonism has a basic group such as an amino group and the like, examples include salts with inorganic acids and organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), and acidic amino acids such as aspartic acid and glutamic acid.

A prodrug of the compound having an angiotensin II antagonism used in the present invention [hereinafter, referred to as AII antagonistic compound in some cases] refers to a compound which is converted to an AII antagonistic compound by a reaction with an enzyme, gastric acid or the like under in vivo physiological condition, that is, a compound which is changed into an AII antagonistic compound by enzymatic oxidation, reduction, hydrolysis or the like, or a compound which is changed into an AII antagonistic compound by hydrolysis with gastric acid or the like. Examples of the prodrug of an AII antagonistic compound include compounds in which an amino group of the AII antagonistic compound is acylated, alkylated or phosphorylated (e.g. compounds in which an amino group of the AII antagonistic compound is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated); compounds in which a hydroxy group of the AII antagonistic compound is acylated, alkylated, phosphorylated or borated (e.g. compounds in which a hydroxy group of the AII antagonistic compound is acetylated, palmitoylated, propanoylated, pivaloylated, succinylated, fumarylated, alanylated or dimethylaminomethylcarbonylated); compounds in which a carboxyl group of the AII antagonistic compound is esterified or amidated (e.g. compounds in which a carboxyl group of the AII antagonistic compound is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, or methylamidated); and the like. These compounds can be prepared from an AII antagonistic compound according to a method known per se.

Alternatively, the prodrug of an AII antagonistic compound may be compounds which can be converted to an AII antagonistic compound under a physiological condition, such as those described in "Development of Medicaments", vol.7, Molecular Design, pp.163-198, published by Hirokawashoten in 1990.

In addition, the AII antagonistic compound may be a hydrate or a non-hydrate.

Further, active ingredients other than the AII antagonist to be used in the present invention which will be described in detail below may be any of a salt, a free compound, a prodrug, a hydrate, and a non-hydrate.

Examples of such the salt or prodrug include those exemplified for the AII antagonistic compound abovementioned.

Examples of the remedy for hypertension to be used in the present invention include an angiotensin converting enzyme (ACE) inhibitor, a diuretic agent, a calcium antagonist, a vasopressin antagonist, an angiotensin converting enzyme and neutral endopeptidase (ACE/NEP) inhibitor, a β blocker and an aldosterone antagonist.

Examples of the ACE inhibitor include enalapril, cilazapril, tamocapril, trandolapril, lisinopril and ramipril.

Examples of the diuretic agent include indapamide, trichlormethiazide, bumetanide, hydrochlorothiazide and metolazone.

Examples of the calcium antagonist include amlodipine, nitrendipine and manidipine.

Examples of the vasopressin antagonist include Tolvaptan, Conivaptan hydrochloride and Relcovaptan.

The ACE/NEP inhibitor is a protease inhibitor having the activity of inhibiting both angiotensin converting enzyme (ACE) and neutral endopeptidase (NEP) and is a medicament which inhibits production of angiotensin II and,

at the same time, inhibits degradation of atrial natriuretic peptide to treat hypertension, and examples thereof include omapatrilat, fasidotril and sampatrilat.

Examples of the β blocker include carvedilol, metoprolol and propranolol.

Examples of the aldosterone antagonist include spironolactone and eplerenone.

Herein, as another aspect of the present invention, for example, an aspect of use of aldosterone antagonist such as eplerenone (epoxymexrenone), a medicine comprising a combination of AII antagonist and aldosterone antagonist may be used, for example, according to WO 96/40257. Specifically, the present invention includes:

[1] a medicine comprising a combination of 2-ethoxy-1-[[2'-(1H-tetrazol-5-yl)biphenyl-4-yl]methyl]benzimidazole-7-carboxylic acid or a salt thereof and eplerenone,
[2] a medicine comprising a combination of 1-(cyclohexyloxycarbonyloxy)ethyl 2-ethoxy-1-[[2'-(1H-tetrazol-5-yl) biphenyl-4-yl]methyl]benzimidazole-7-carboxylate or a salt thereof and eplerenone,
[3] a medicine comprising a combination of 2-ethoxy-1-[[2'-(2,5-dihydro-5-oxo-1,2,4-oxadiazol-3-yl)biphenyl-4-yl] methyl]benzimidazole-7-carboxylic acid or a salt thereof and eplerenone, and
[4] a medicine comprising a combination of one or more AII antagonists selected from Losartan, Eprosartan, Valsartan, Telmisartan, Irbesartan, Olmesartan medoxomil, Olmesartan and Tasosartan, and eplerenone.

When specific AII antagonist and aldosterone antagonist are used in combination as described above, these drugs may be formulated into a preparation by separately or simultaneously mixing with a pharmacologically acceptable carrier, excipient, binder, diluent and the like, and the drugs can be orally or parenterally administered as a pharmaceutical composition. When drugs are separately formulated into a preparation, separately formulated preparations may be mixed to administrate using a diluent and the like upon use, and separately formulated individual preparations may be administered to the same subject simultaneously, or separately at different times. A kit product for mixing to administrate separately formulated preparations using a diluent and the like upon use (e.g. an injectable kit, which contains amples containing individual powdery drugs and a diluent for dissolving two or more drugs by mixing upon use), and a kit product for administering separately formulated individual preparations to the same subject simultaneously, or separately at different times (e.g. a tablet kit for administering two or more tablets simultaneously, or separately at different times, in which tablets containing individual drugs are placed into the same bag or separate bags and, if needed, a column for describing times to administer the drug is provided) are included in the medicine of the present invention.

The medicine comprising a combination of AII antagonist and aldosterone antagonist can be orally or parenterally administered, for example, as granules, powders, a dust, a tablet, a capsule, a syrup, an emulsion, suppositories (e.g. rectal suppositories, vaginal suppositories etc.), injectables (e.g. subcutaneous injectables, intravenous injectables, intramuscular injectables, intraperitoneal injectables etc.), drops, external preparations (e.g. transnasal preparations, percutaneous preparations, ointments etc.) , emulsions, elixirs, suspensions or solutions, and these preparations can be formulated according to the methods known per se which are generally used in formulation steps. In the present specification, parenteral includes subcutaneous injection, intraveneous injection, intramuscular injection, intraperitoneal injection and drip.

A formulation for injection, for example, a sterile injectable aqueous suspension or oily suspension can be prepared using a suitable dispersing agent or wetting agent and a suspending agent according to a method known in the art. The sterile injectable formulation may be a sterile injectable solution or suspension in a diluent or solvent, such as an aqueous solution, which is nontoxic and can be parenterally administered. A usable vehicle or an acceptable solvent include water, Ringer' solution, an isotonic sodium chloride solution and the like. Further, sterile fixed oils can be usually used as a solvent or a suspending solvent.

For this purpose, any fixed oils and fatty acids can be used, including natural or synthetic or semi-synthetic fatty oils or fatty acids, and natural or synthetic or semi-synthetic mono- or di- or triglycerides.

Further, additives such as a preservative, an isotonic, a solubilizer, a stabilizer and a soothing agent may be appropriately used.

Rectal suppositories can be prepared by mixing the drug and a suitable non-stimulatory additive, for example, additives which are solid at room temperature, but are liquid at the temperature of intestinal tract, and are molten in a rectum to release the drug, such as cocoa butter and polyethylene glycols.

Examples of a solid dosage form for oral administration include the aforementioned powders, granules, tablets, pills and capsules. In such the dosage forms, an active ingredient can be mixed with at least one additive, for example, sucrose, lactose, cellulose sugar, mannitol, maltitol, dextran, starches, agar, alginates, chitins, chitosans, pectins, tragacanth gums, gums arabic, gelatins, collagens, casein, albumin, synthetic or semi-synthetic polymers or glycerides. Such the dosage forms may usually contain further additives, and examples thereof include inert diluents, lubricants such as magnesium stearate, preservatives such as parabens and sorbic acid, antioxidants such as ascorbic acid, α-tocopherol and cysteine, excipients, disintegrating agents, binders, thickeners, buffers, sweetners, flavoring

agents, perfumes, and coating agents. Tablets and pills may be also prepared by further subjecting to enteric coating. A liquid preparation for oral administration includes pharmaceutically acceptable emulsions, syrups, elixirs, suspensions and solutions, and they may contain an inert diluent which is usually used in the art, for example, water.

It is preferable that a medicine comprising a combination of AII antagonist and aldosterone antagonist contains 0.6 to 39 % by weight (inter alia, 0.7 to 27 % by weight) of the AII antagonist and 0.06 to 35 % by weight (inter alia, 0.6 to 23 % by weight) of the aldosterone antagonist, respectively, in a pharmaceutical composition containing the active ingredients. This composition may be any of compositions in which two or more drugs are formulated into a preparation separately or simultaneously.

A dose of the medicine comprising a combination of AII antagonist and aldosterone antagonist is based on minimum recommended clinical doses of individual drugs, and can be appropriately selected depending on a combination of an subject to be administrated, an age and a weight of the subject, symptom, an administration time, an administration method, a dosage form, and a drug.

A dosage of a particular patient depends on an age, a weight, general health condition, a sex, a diet, an administration time, an administration method, an excretion rate and a combination of drugs, and a symptom degree of disease of a patient subjected to therapy thereupon, and is determined in view of them or other factors.

Typically, an individual daily dose is in a range of about 1/50 of a minimum recommended clinical dose or more and a maximum recommended level or less (preferably a minimum recommended clinical dose or less, more preferably 1/2 of a minimum recommended clinical dose or less) in view of the circumstance where they are administered alone. For example, in treatment of hypertension or heart failure of an adult (body weight about 60 kg), Candesartan cilexetil in a range of about 1 to 50 mg/human/day (preferably about 1 to 35 mg/human/day) and eplerenone in a range of about 0.1 to 30 mg/human/day (preferably about 2 to 30 mg/human/day) can be effectively combined. Naturally, these dose ranges can be regulated on a unit base necessary for dividing a daily dose and, as described above, a dose is determined in view of a nature and degree of disease, an age, a weight, general health condition and a sex of a patient, a diet, an administration time, an administration method, an excretion rate and a combination of drugs, or other factors. A unit dosage is administered once to three times per day (preferably once per day).

The aforementioned medicine comprising a combination of AII antagonist and aldosterone antagonist may be used by further combining with a diuretic agent (e.g. hydrochlorothiazide) which is usually used together with an AII antagonist.

Examples of the hypoglycemic drug (remedy for diabetes) include insulin preparations (e.g. animal insulin preparations extracted from pancreas of cow or pig; human insulin preparations synthesized by genetic engineering using Escherichia coli or yeast; zinc insulin; zinc protamineinsulin; fragment or derivative of insulin (e.g. INS-1 etc.) etc.), insulin sensitivity improving agents (e.g. pioglitazone hydrochloride, troglitazone, rosiglitazone or maleate thereof, GI-262570, JTT-501, MCC-555, YM-440, KRP-297, CS-011, FK-614 etc.), α-glucosidase inhibitors (e.g. voglibose, acarbose, miglitole, emiglitate etc.), biguanide agents (e.g. phenformin, metformin, buformin etc.), insulin secretagogues [sulfonylurea agent (e.g. tolbutamide, glibenclamide, gliclazid, chlorpropamide, tolazamide, acetohexamide, glyclopyramide, glimepiride, glipizide, glybuzole etc.), repaglinide, nateglinide, mitiglinide or calucium salt hydrate thereof, GLP-1 etc. ; particularly, glibenclamide and nateglinide are preferable],

Other dipeptidylpeptidase IV inhibitors (e.g. NVP-DPP-278, PT-100 etc.), β3 agonists (e.g. CL-316243, SR-58611-A, UL-TG-307, SB-226552, AJ-9677, BMS-196085, AZ-40140 etc.), amilin agonists (e.g. pramlintide etc.), phosphotyrosinephosphatase inhibitors (e.g. vanadic acid etc.), gluconeogenesis inhibitors (e.g. glycogenphosphorylase inhibitor, glucose-6-phosphatase inhibitor, glucagon antagonist etc.), SGLUT (sodium-glucose cotransporter) inhibitor (e.g. T-1095 etc.) and the like.

Inter alia, insulin sensitizer, insulin secretagogues and insulin preparations are preferable.

As the insulin sensitivity improving drug, pioglitazone hydrochloride, troglitazone or rosiglitazone are preferable.

As the insulin secretagogue, glibenclamide, nateglinide and repaglinide are preferable.

Examples of the remedy for hyperlipemia include statin medicaments, fibrate medicaments, and nicotinic acid derivatives.

Examples of the statin medicament include cerivastatin or a sodium salt thereof, pravastatin or a sodium salt thereof, simvastatin, atrovastatin or a calcium hydrate thereof.

Examples of the fibrate medicament include fenofibrate, fenofibrinic acid, bezafibrate and gemfibrozil.

Examples of the nicotinic acid derivative include niceritrol and Cholexamin.

Examples of the antithrombotic drug include GPIIb/IIIa antagonists, low-molecular weight heparin, thrombin inhibitors and anti-platelet drugs.

Examples of the GPIIb/IIIa antagonist include abciximab.

Examples of the low-molecular weight heparin include enoxaparin.

Examples of the thrombin inhibitor include argatroban.

Examples of the anti-platelet drug include clopidogrel sulfate and aspirin.

Examples of the remedy for climacteric disturbance include estrogen (e.g. estradiol, estradiol valerate, conjugated estrogen etc.).

Examples of the anticancer drug include the following hormone therapeutic agents, chemotherapeutic agents, immunotherapeutic agents and medicaments which inhibit the activity of cell growth factor and its receptor.

Examples of the "hormone therapeutic agent" include fosfestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogen (e.g. tamoxifen citrate, toremifene citrate etc.), pill preparation, mepitiostane, testololactone, aminoglutetimide, LH-RH agonist (e.g. goserelin acetate, buserelin, leuprorelin etc.), droloxifene, epitiostanol, ethynylestradiol sulfonate, aromatase inhibitor (e.g. fadrozole hydrochloride, anastrozole, letrozole, exemestane, borosole, formestane etc.), anti-androgen (e.g. flutamide, bicalutamide, nilutamide etc.), 5$\alpha$-reductase inhibitor (e.g. finasteride, epristeride etc.), adrenocortical hormone medicament (e.g. dexamethasone, prednisolone, betamethasone, triamcinolone etc.), androgen synthesis inhibitor (e.g. abiraterone etc.), retinoid and medicament which delays metabolism of retinoid (e.g. liarozole etc.) and the like.

Examples of the "chemotherapeutic agent" include alkylating agents, metabolism antagonists, anticancer antibiotics and plant-derived anticancer agents.

Examples of the "alkylating agent" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucide, carboplatin, cisplatin, miboplatin, nedaplatin, oxaliplatin, altretamin, ambamustin, dibrospidium hydrochloride, fotemustin, predonimustin, pumitepa, livomustin, temozolomide, treosulfane, trofosfamide, zinostatin stimalamer, carboquone, adozelesin, cystemustin and bizelesin.

Examples of the "metabolism antagonist" include mercaptopurine, 6-mercaptopurine riboside, thioinosine, methotrexate, enocitabine, cytarabine, cytarabine ocfosfate, ancitabine hydrochloride, 5-FU medicaments (e.g. fluorouracil, tegafur, UFT, doxifluridine, carmofur, galocitabine, emitefur, etc.), aminopterin, calcium leucovorin, tabloid, butocin, calcium folinate, levofolinate calcium, cladribine, emitefur, fludarabine, gemcitabine, hydroxycarbamide, pentostatin, piritrexim, idoxuridine, mitoguazone, thiazofurine and ambamustin.

Examples of the "anticancer antibiotic" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride and idarubicin hydrochloride.

Examples of the "plant-derived anticancer agent" include etoposide, etoposide phosphate, vinblastine sulfate, vincristine sulfate, vindesine sulfate, teniposide, paclitaxel, docetaxel and vinorelbine.

Examples of the "immunotherapeutic agent (BRM)" include picibanil, krestin, sizofiran, lentinan, ubenimex, interferon, interleukin, macrophage colony stimulating factor, granulocyte colony stimulating factor, erythropoietin, lymphotoxin, BCG vaccine, Corynebacterium parvum, levamisole, polysaccharide K and procodazole.

The "cell growth factor" in the "medicament which inhibits the activity of cell growth factor and its receptor" may be any substances as far as they promote the growth of cells. Usually, examples thereof include factors which are a peptide having a molecular weight of 20,000 or less and exert the activity at low concentration by binding with a receptor, and specifically, (1) EGF (epidermal growth factor) or a substance having substantially the same activity as that of EGF [e.g. EGF, heregulin (HER2 ligand) etc.], (2) insulin or a substance having substantially the same activity as that of insulin [e.g. insulin, IGF (insulin-like growth factor)-1, IGF-2 etc.], (3) FGF (fibroblast growth factor) or a substance having substantially the same activity as that of FGF [e.g. acidic FGF, basic FGF, KGF (keratinocyte growth factor), FGF-10 etc.], (4) other cell growth factors [e.g. CSF (colony stimulating factor), EPO (erythropoietin), IL-2 (interleukin-2), NGF (nerve growth factor), PDGF (platelet-derived growth factor), TGF$\beta$(transforming growth factor $\beta$), HGF (hepatocyte growth factor), VEGF (vascular endothelial growth factor) etc.] and the like are exemplified.

The "receptor of cell growth factor" may be any receptors as far as they have the ability of binding with the cell growth factor, and examples thereof include EGF receptor, heregulin receptor (HER2), insulin receptor-1, insulin receptor-2, IGF receptor, FGF repector-1 and FGF receptor-2.

Examples of the "medicament which inhibits the activity of cell growth factor" include Herceptin (HER2 receptor antibody).

Besides the aforementioned medicaments, N-asparaginase, aceglatone, procarbazine hydrochloride, protoporphyrin cobalt complex, mercury hematoporphyrin sodium, topoisomerase 1I inhibitor (e.g. irinotecan, topotecan etc.), topoisomerase II inhibitor (e.g. sobuzoxane etc.), differentiation inducer (e.g. retinoid, vitamin Ds etc.), vascularization inhibitor and $\alpha$-blocker (e.g. tamuslosin hydrochloride etc.) can be also used.

Particularly preferable examples of the anticancer drug include GnRH agonist and antagonist.

Examples of the GnRH agonist·antagonist include GnRH agonists and antagonists which are effective for hormone-dependent diseases, in particular, sex hormone-dependent diseases such as sex hormone-dependent cancers (e.g. prostate cancer, uterus cancer, breast cancer, pituitary gland tumor etc.), benign prostatic hyperplasia, endometriosis, hysteromyoma, precocious puberty, dysmenorrhea, amenorrhea, premenstrual syndrome, multilocular ovary syndrome and the like, and contraception (or when the rebound effect after interruption of dosing is utilized, infertility). Further examples include GnRH agonist and antagonist effective for benign or malignant tumor which is sex hormone-independent but GnRH-sensitive.

Specific examples of GnRH agonist·antagonist include peptides descried, for example, in Treatment with GnRH analogs: Controversies and perspectives [published by The Prathenon Publishing Group Ltd. in 1996], JP-A 3-503165, JP-A 3-101695, JP-A 7-97334 and JP-A 8-259460.

As the GnRH antagonist, for example, a peptide represented by the general formula [1]:

$$X\text{-}D2Nal\text{-}D4ClPhe\text{-}D3Pal\text{-}Ser\text{-}A\text{-}B\text{-}Leu\text{-}C\text{-}Pro\text{-}DAlaNH_2$$

wherein X represents N(4$H_2$-furoyl)Gly or NAc, A represents a residue selected from NMeTyr, Tyr, Aph(Atz) and NMeAph(Atz), B represents a residue selected from DLys(Nic), DCit, DLys(AzaglyNic), DLys(AzaglyFur), DhArg($Et_2$), DAph(Atz) and DhCi, and C represents Lys(Nisp), Arg or hArg($Et_2$), or a salt thereof is used.

In addition, examples of the non-peptidic GnRH antagonist include those described in WO 95/28405 (JP-A 8-295693), WO 97/14697 (JP-A 9-169767), WO 97/14682 (JP-A 9-169735), WO 96/24597 (JP-A 9-169768), WO 00/00493 describing thienopyridine compounds "e.g. 3-(N-Benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine etc.", and WO 00/56739 describing thienopyrimidine compounds "e.g. 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione etc.".

As the GnRH agonists, for example, a physiologically active peptide represented by the general formula [II]:

5-oxo-Pro-His-Trp-Ser-Tyr-Y-Leu-Arg-Pro-Z

wherein Y represents a residue selected from DLeu, DAla, DTrp, DSer(tBu), D2Nal and DHis(ImBzl), and Z represents NH-$C_2H_5$ or Gly-$NH_2$, or a salt thereof is used. In particular, a peptide in which Y is DLeu, and Z is NH-$C_2H_5$, or a salt thereof (i.e. a peptide represented by 5-oxo-Pro-His-Trp-Ser-Tyr-DLeu-Leu-Arg-Pro-NH-$C_2H_5$) or a salt thereof, in particular acetate thereof (leuprorelin acetate: manufactured by Takeda Chemical Industries, Ltd.)) is particularly suitable.

Peptides exemplified as the GnRH agonist ·antagonist may be pharmacologically acceptable salts. When the peptides have a basic group such as amino group and the like, examples of such the salts include salts with inorganic acids (e.g. hydrochloric acid, sulfuric acid, nitric acid, boric acid etc.), and organic acids (e.g. carbonic acid, bicarbonic acid, succinic acid, acetic acid, propionic acid, trifluoroacetic acid etc.).

When the peptides have an acidic group such as carboxyl group and the like, examples include salts with inorganic bases (e.g. alkali metal such as sodium, potassium etc., alkaline earth metal such as calcium, magnesium etc.) and organic bases (e.g. organic amines such as triethlyamine etc., basic amino acids such as arginine etc.). In addition, the peptides may form a metal complex compound (e.g. copper complex, zinc complex etc.).

These peptides or salts thereof can be prepared by the methods described in the aforementioned references or gazettes or similar methods thereto.

Other preferable examples of GnRH agonist other than the abovementioned leuprorelin (leuprorelin acetate) include:

(1) Goserelin

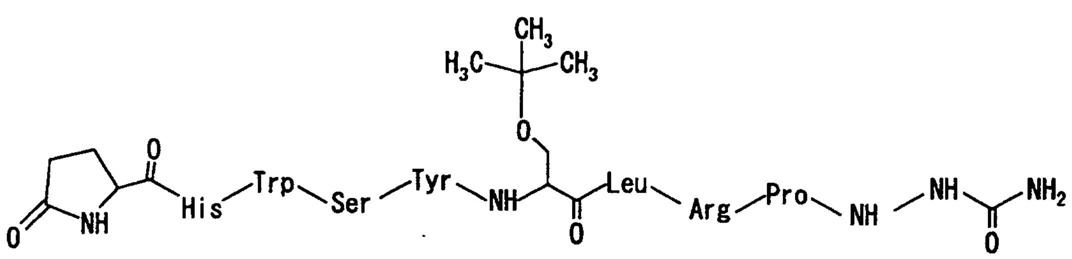

(US-A No.4100274, JP-A 52-136172),
(2) Buserelin

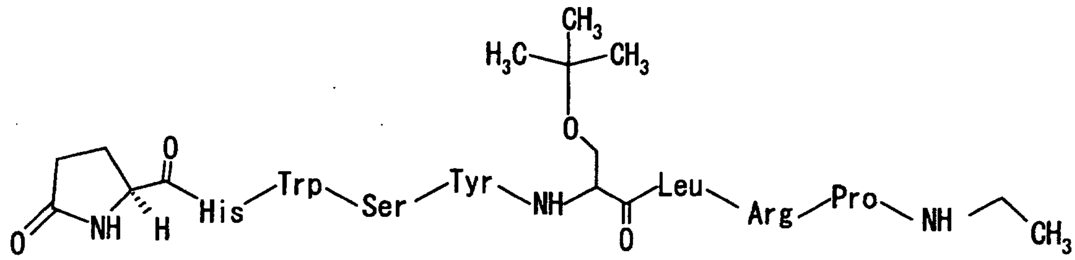

(USP 4,024,248, DE 2438352, JP-A 51-41359),
(3) Triptorelin

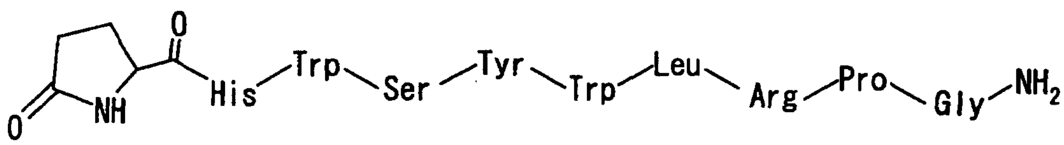

(US-A No.4010125, JP-A 52-31073),
(4) Nafarelin

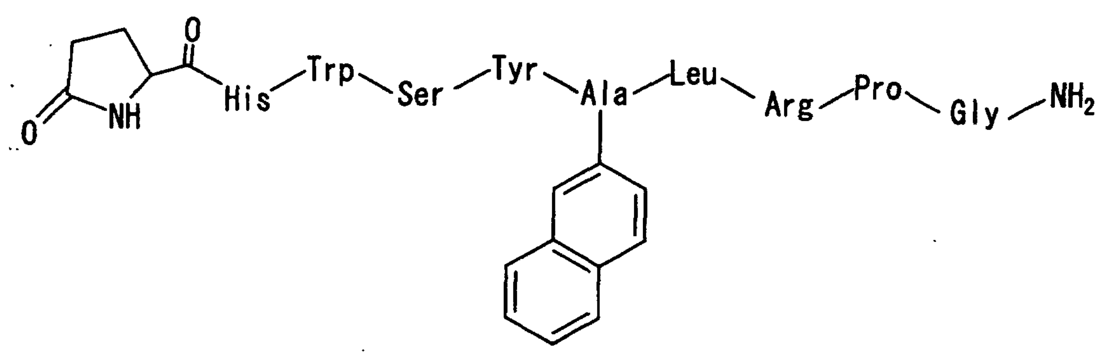

(US-A No.4234571, JP-A 55-164663, JP-A 63-264498, JP-A 64-25794),
(5) Histrelin

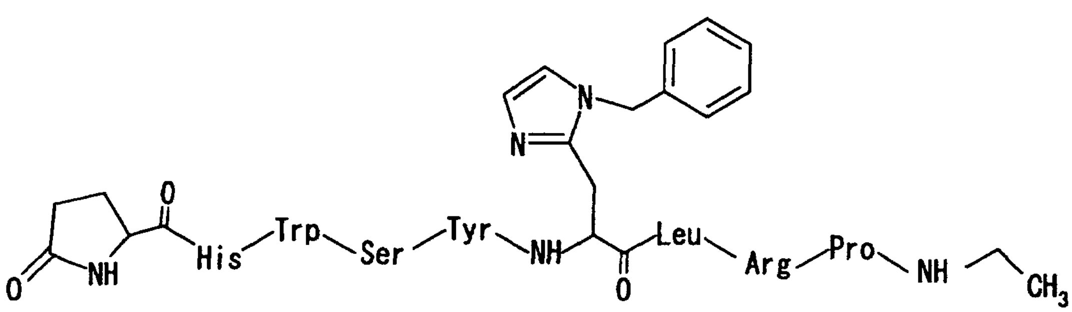

(6) Deslorelin

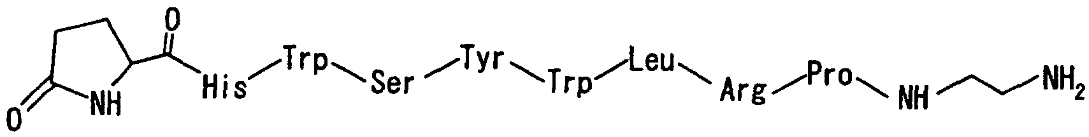

(US-A No.4569967, US-A No.4218439),
(7) Meterelin

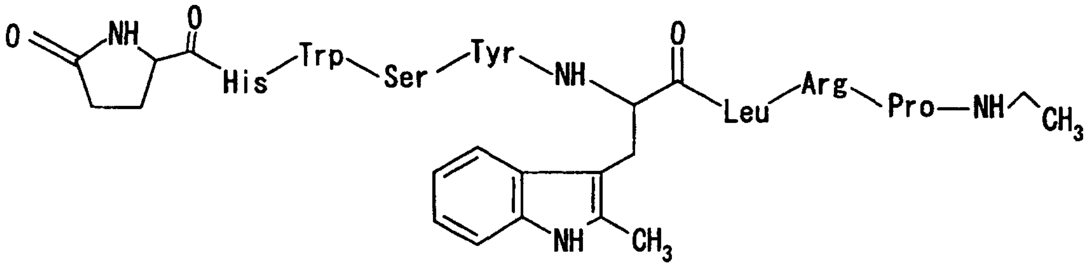

(PCT WO 91/18016),
(8) Gonadrelin

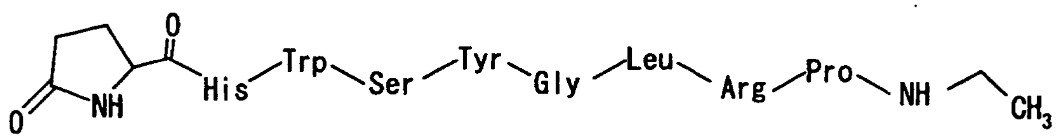

(DE 2213737), and the like, and salts thereof.

Among the forgoing, leuprorelin (leuprorelin acetate), 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof, and 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof are preferable. The salts are exemplified by those for the aforementioned AII antagonist.

In the present invention, (A) an AII antagonist and (B) one or more drugs selected from a remedy for hypertension, a hypoglycemic drug, a remedy for hyperlipemia, an antithrombotic drug, a remedy for climacteric disturbance and an anticancer drug are used in combination, and theses drugs can be orally or parenterally administered as a pharmaceutical composition by separately or simultaneously formulating into a sustained-release preparation. When drugs are separately formulated into a preparation, separately formulated preparations may be mixed to administrate using a diluent and the like upon use, and separately formulated individual sustained-release preparations may be administered to the same subject simultaneously, or separately at different times. A kit product for mixing to administrate separately formulated preparations using a diluent and the like upon use (e.g. an injectable kit, which contains amples containing individual powdery drugs and a diluent for dissolving two or more drugs by mixing upon use), and a kit product for administering separately formulated individual sustained-release preparations to the same subject simultaneously, or separately at different times (e.g. a tablet kit for administering two or more tablets simultaneously, or separately at different times, in which tablets containing individual drugs are placed into the same bag or separate bags and, if needed, a column for describing times to administer the drug is provided) are included in the medicine of the present invention.

A preferable form of the sustained-release medicine of the present invention includes those of following (1) to (3).

(1) A sustained-release medicine containing (A) an AII antagonist and (B) one or more (preferable two to three)

drugs selected from a remedy for hypertension, a hypoglycemic drug, a remedy for hyperlipemia, an antithrombotic drug, a remedy for climacteric disturbance and an anticancer drug.

This refers to a sustained-release preparation containing "(A) an AII antagonist" and "(B) one or more (preferably two to three) drugs selected from a remedy for hypertension, a hypoglycemic drug, a remedy for hyperlipemia, an antithrombotic drug, a remedy for climacteric disturbance and an anticancer drug" in a minimum constituent unit of the preparation, wherein both (A) and (B) were simultaneously formulated into the preparation.

(2) A sustained-release medicine containing (A) a sustained-release preparation containing an AII antagonist and (B) a sustained-release preparation containing one or more (preferably two to three) drugs selected from a remedy for hypertension, a hypoglycemic drug, a remedy for hyperlipemia, an antithrombotic drug, a remedy for climacteric disturbance and an anticancer drug.

The minimum constituent unit of this medicine is "(A) a sustained-release preparation containing an AII antagonist" and "(B) a sustained-release preparation containing one or more (preferably two to three) drugs selected from a remedy for hypertension, a hypoglycemic drug, a remedy for hyperlipemia, an antithrombotic drug, a remedy for climacteric disturbance and an anticancer drug", and separately formulated two or more sustained-release preparations are used as a single preparation by mixing into the same preparation, optionally together with a dilluent and the like before use or upon use.

Here, when the sustained-release preparation of (B) contains two or more drugs, two or more (preferably two to three) drugs selected from "a remedy for hypertension", "a hypoglycemic drug", "a remedy for hyperlipemia", "an antithrombotic drug", "a remedy for climacteric disturbance" and "an anticancer drug" may be simultaneously formulated into a preparation and may be contained in the same sustained-release preparation, or by formulating separately into a preparation, plural drugs may be formulated into separate two or more (preferably two to three) sustained-release preparations.

Upon use, "(A) the sustained-release preparation containing an AII antagonist" and "(B) the sustained-release preparation containing one or more drugs selected from a remedy for hypertension, a hypoglycemic drug, a remedy for hyperlipemia, an antithrombotic drug, a remedy for climacteric disturbance and an anticancer drug" are administered by the same route at the same time.

(3) A sustained-release medicine comprising a combination of (A) a sustained-release preparation containing an AII antagonist and (B) a sustained-release preparation containing one or more (preferably two to three) drugs selected from a remedy for hypertension, a hypoglycemic, a remedy for hyperlipemia, an antithrombotic, a remedy for climacteric disturbance and an anticancer drug.

The minimum constituent unit of this medicine is "(A) a sustained-release preparation containing an AII antagonist" and "(B) a sustained-release preparation containing one or more (preferably two to three) drugs selected from a remedy for hypertension, a hypoglycemic drug, a remedy for hyperlipemia, an antithrombotic drug, a remedy for climacteric disturbance and an anticancer drug", and separately formulated two or more sustained-release preparations are separately used together.

Here, when the sustained-release preparation of (B) contains two or more drugs, two or more (preferably two to three) drugs selected from "a remedy for hypertension", "a hypoglycemic drug", "a remedy for hyperlipemia", "an antithrombotic drug", "a remedy for climacteric disturbance" and "an anticancer drug" may be simultaneously formulated into a preparation, and may be contained in the same sustained-release preparation, or plural drugs may be separately formulated into a preparation and may be formulated into separate two or more (preferably two to three) sustained-release preparations.

Upon use, "(A) the sustained-release preparation containing an AII antagonist" and "(B) the sustained-release preparation containing one or more (preferably two to three) drugs selected from a remedy for hypertension, a hypoglycemic drug, a remedy for hyperlipemia, an antithrombotic drug, a remedy for climacteric disturbance and an anticancer drug" may be administered simultaneously or by separate routes at different times.

Particularly preferable forms as the sustained-release medicine of the present invention are as follows:

(i) A sustained-release medicine comprising a combination of Candesartan and actos. Specifically, a sustained-release medicine containing Candesartan and actos, a sustained-release medicine containing a sustained-release preparation containing Candesartan and a sustained-release preparation containing actos, or a sustained-release medicine comprising a combination of a sustained-release preparation containing Cardesartan and a sustained-release preparation containing actos. Particularly preferred is a sustained-release medicine containing Candesartan and actos.

(ii) A sustained-release medicine comprising a combination of Candesartan cilexetil and actos. Specifically, a sustained-release medicine containing Candesartan cilexetil and actos, a sustained-release medicine containing a sustained-release preparation containing Candesartan cilexetil and a sustained-release preparation containing actos, or a sustained-release medicine comprising a combination of a sustained-release preparation containing

Candesartan cilexetil and a sustained-release preparation containing actos. Particularly preferred is a sustained-release medicine containing Candesartan cilexetil and actos.

(iii) A sustaiend-release medicine comprising a combination of Candesartan and atrovastatin. Specifically, a sustained-release medicine containing Candesartan and atrovastatin, a sustained-release medicine containing a sustained-release preparation containing Candesartan and a sustained-release preparation containing atrovastatin, or a sustained-release medicine comprising a combination of a sustained-release preparation containing Candesartan and a sustained-release preparation containing atrovastatin. Particularly preferred is a sustained-release medicine containing Candesartan and atrovastatin.

(iv) A sustained-release medicine comprising a combination of Candesartan cilexetil and atrovastatin. Specifically, a sustained-release medicine containing Candesartan cilexetil and atrovastatin, a sustained-release medicine containing a sustained-release preparation containing Candesartan cilexetil and a sustained-release preparation containing atrovastatin, or a sustained-release medicine comprising a combination of a sustained-release preparation containing Candesartan cilexetil and a sustained-release preparation containing atrovastatin. Particularly preferred is a sustained-release medicine containing Candesartan cilexetil and atrovastatin.

(v) A sustained-release medicine comprising a combination of Candesartan and enoxaparin. Specifically, a sustained-release medicine containing Candesartan and enoxaparin, a sustained-release medicine containing a sustained-release preparation containing Candesartan and a sustained-release preparation containing enoxaparin, or a sustained-release medicine comprising a combination of a sustained-release preparation containing Candesartan and a sustained-release preparation containing enoxaparin. Particularly preferred is a sustained-release medicine containing Candesartan and enoxaparin.

(vi) A sustained-release medicine comprising a combination of Candesartan cilexetil and enoxaparin. Specifically, a sustained-release medicine containing Candesartan cilexetil and enoxaparin, a sustained-release medicine containing a sustained-release preparation containing Candesartan cilexetil and a sustained-release preparation containing enoxaparin, or a sustained-release medicine comprising a combination of a sustained-release preparation containing Candesartan cilexetil and a sustained-release preparation containing enoxaparin. Particularly preferred is a sustained-release medicine containing Candesartan cilexetil and enoxaparin.

(vii) A sustained-release medicine comprising a combination of Candesartan and estradiol. Specifically, a sustained-release medicine containing Candesartan and estradiol, a sustained-release medicine containing a sustained-release preparation containing Candesartan and a sustained-release preparation containing estradiol, or a sustained-release medicine comprising a combination of a sustained-release preparation containing Candesartan and a sustained-release preparation containing estradiol. Particularly preferred is a sustained-release medicine containing Candesartan and estradiol.

(viii) A sustained-release medicine comprising a combination of Candesartan cilexetil and estradiol. Specifically, a sustained-release medicine containing Candesartan cilexetil and estradiol, a sustained-release medicine containing a sustained-release preparation containing Candesartan cilexetil and a sustained-release preparation containing estradiol, or a sustained-release medicine comprising a combination of a sustained-release preparation containing Candesartan cilexetil and a sustained-release preparation containing estradiol. Particularly preferred is a sustained-release medicine containing Candesartan cilexetil and estradiol.

(ix) A sustained-release medicine comprising a combination of Candesartan and Leuplin. Specifically, a sustained-release medicine containing Candesartan and Leuplin, a sustained-release medicine containing a sustained-release preparation containing Candesartan and a sustained-release preparation containing Leuplin, or a sustained-release medicine comprising a combination of a sustained-release preparation containing Candesaran and a sustained-release preparation containing Leuplin. Particularly preferred is a sustained-release medicine containing Candesartan and Leuplin.

(x) A sustained-release medicine comprising a combination of Candesartan cilexetil and Leuplin. Specifically, a sustained-release medicine containing Candesartan cilexetil and Leuplin, a sustained-release medicine containing a sustained-release preparation containing Candesartan cilexetil and a sustained-release preparation containing Leuplin, or a sustained-release medicine comprising a combination of a sustained-release preparation containing Candesartan cilexetil and a sustained-release preparation containing Leuplin. Particularly preferred is a sustained-release medicine containing Candesartan cilexetil and Leuplin.

In the sustained-release medicine of the present invention, an AII antagonist is usually incorporated at 5 to 50% by weight (preferably 5 to 40% by weight) and as one or more drugs selected from a remedy for hypertension, a hypoglycemic drug, a remedy for hyperlipemia, an antithrombotic drug, a remedy for climacteric disturbance and an anticancer drug to be used together, a remedy for hypertension is usually incorporated at 1 to 50% by weight (preferably 5 to 40% by weight), a hypoglycemic drug is incorporated at 1 to 50% by weight (preferably 5 to 40% by weight), a remedy for hyperlipemia is incorporated at 1 to 50% by weight (preferably 1 to 40% by weight), an antithrombotic drug is incorporated at 1 to 50% by weight (preferably 2 to 40% by weight), a remedy for climacteric disturbance is incorporated at 0.1 to 50% by weight (preferably 0.1 to 40% by weight), and an anticancer drug is incorporated at 0.1 to 50% by weight (preferably 0.1 to 40% by weight) in one pharmaceutical composition containing respective active ingredients.

For example, the aforementioned particularly preferable forms will be described in detail as follows.

(1) In the sustained-release medicine (composition) comprising a combination of Candesartan or Candesartan cilexetil and actos, the contents of Candesartan or Candesartan cilexetil and actos relative to whole pharmaceutical composition are 5 to 50% by weight and 1 to 50% by weight, preferably 5 to 40% by weight and 1 to 40% by weight, respectively.

(2) In the sustained-release medicine (composition) comprising a combination of Candesartan or Candesartan cilexetil and atrovastatin, the contents of Candesartan or Candesartan cilexetil and atrovastatin relative to whole pharmaceutical composition are 5 to 50% by weight and 1 to 50% by weight, preferably 5 to 40% by weight and 2 to 40% by weight, respectively.

(3) In the sustained-release medicine (composition) comprising a combination of Candesartan or Candesartan cilexetil and enoxaparin, the contents of Candesartan or Candesartan cilexetil and enoxaparin relative to whole pharmaceutical composition are 5 to 50% by weight and 1 to 50% by weight, preferably 5 to 40% by weight and 2 to 50% by weight, respectively.

(4) In the sustained-release medicine (composition) comprising a combination of Candesartan or Candesartan cilexetil and estradiol, the contents of Candesartan or Candesartan cilexetil and estradiol relative to whole pharmaceutical composition are 5 to 50% by weight and 0.1 to 50% by weight, preferably 5 to 40% by weight and 0.2 to 40% by weight, respectively.

(5) In the sustained-release medicine (composition) comprising a combination of Candesartan or Candesartan cilexetil and Leuplin, the contents of Candesartan or Candesartan cilexetil and Leuplin relative to whole pharmaceutical composition are 5 to 50% by weight and 0.1 to 50% by weight, preferably 5 to 40% by weight and 0.1 to 40% by weight, respectively.

As described above, the administration form of the sustained-release medicine of the present invention is not particularly limited and, upon administration, an AII antagonist and one or more drugs selected from a remedy for hypertension, a hypoglycemic drug, a remedy for hyperlipemia, an antithrombotic drug, a remedy for climacteric disturbance and an anticancer drug (hereinafter, one or more drugs selected from a remedy for hypertension, a hypoglycemic drug, a remedy for hyperlipemia, an antithrombotic drug, a remedy for climacteric disturbance and an anticancer drug are abbreviated as concomitant drug in some cases) are combined, thereupon, it is enough that respective drugs are release-controlled in a sustained-release manner. Examples of such the administration form include (1) administration of a single preparation obtained by formulating an AII antagonist and a concomitant drug into a sustained-release preparation simultaneously, (2) simultaneous administration of two or more sustained-release preparations obtained by separately formulating an AII antagonist and a concomitant drug into sustained-release preparations by the same administration route, (3) administration of two or more sustained-release preparations obtained by separately formulating an AII antagonist and a concomitant drug into sustained-release preparations, by the same administration route at different times, (4) simultaneous administration of two or more sustained-release preparations obtained by separately formulating an AII antagonist and a concomitant drug into sustained-release preparations, by different administration routes, and (5) administration of two or more sustained-release preparations obtained by separately formulating an AII antagonist and a concomitant drug into sustained-release preparations, by different administration routes at different times (e.g. administration in an order of a sustained-release preparation containing an AII antagonist and a sustained-release preparation containing a concomitant drug, or administration in a reverse order).

As (C) biodegradable polymer used in the present invention, for example, a polymer and a copolymer which is synthesized from one or more of α-hydroxycarboxylic acids (e.g. glycolic acid, lactic acid etc.), hydroxydicarboxylic acids (e.g. malic acid etc.), hydroxytricarboxylic acids (e.g. citric acid etc.) and the like and has a free carboxyl group, or an ester thereof, or a mixture thereof; poly-α-cyanoacrylic acid ester; polyamino acid (e.g. poly-g-benzyl-L-glutamic acid etc.); anhydrous maleic acid copolymer (e.g. styrene-maleic acid copolymer etc.) are used.

A polymerization form may be any of random, block and graft. When the aforementioned α-hydroxyacids, hydroxydicarboxylic acids and hydroxytricarboxylic acids have an optically active center in the molecule, any of D-, L- and DL-isomers may be used. Among them, an α-hydroxycarboxylic acid polymer (preferably lactic acid-glycolic acid polymer), an ester thereof, and poly-α-cyanoacrylic acid ester are preferable. More preferred are a lactic acid-glycolic acid polymer and an ester thereof.

When a lactic acid-glycolic acid polymer is used as a biodegradable polymer, a composition ratio (mol%) is preferably 100/0 to 40/60, particularly preferably 100/0 to 50/50.

The weight average molecular weight of the aforementioned lactic acid-glycolic acid polymer is usually about 3,000 to about 50,000, preferably about 4,000 to about 40,000, more preferably about 5,000 to about 30,000. In addition,

a dispersity (weight average molecular weight/number average molecular weight) is usually preferably about 1.2 to about 4.0, more preferably about 1.5 to 3.5.

The weight average molecular weight, number average molecular weight and dispersity in the present specification refer to the molecular weight in terms of polystyrene measured by gel permeation chromatography (GPC) using, as standard substance, 14 kinds of polystyrenes having weight average molecular weights of 1,110,000, 707,000, 354,000, 189,000, 156,000, 98,900, 66,437, 37,200, 17,100, 9,830, 5,870, 2,500, 1,303, and 500, and the dispersity calculated therefrom. For the measurement, GPC column KF804L × 2 (manufactured by SHOWA DENKO K.K.) was used, and chloroform was used as a mobile phase. In addition, a biodegradable polymer was dissolved in an acetone-methanol mixed solvent, and a carboxyl group in this solution was titrated with an alcoholic potassium hydroxide solution using phenolphthalein as an indicator, whereby, a number average molecular weight by end-group determination is calculated. Hereinafter, this is referred to as number average molecular weight by end-group determination. The number average molecular weight by end-group determination is an absolute value, while the number average molecular weight determined by GPC is a relative value which varies depending on assay or analysis condition (e.g. kind of mobile phase, kind of column, standard substance, selection of slice width, selection of baseline etc.), so it is difficult to primarily digitalize it. However, for example, in a polymer which is synthesized by a non-catalytic dehydration polycondensation method from lactic acid and glycolic acid and has a free carboxyl group at the terminal, the number average molecular weight by GPC determination and the number average molecular weight by end-group determination are approximately consistent. Approximately consistent in the case of this lactic acid-glycolic acid polymer refers to that the number average molecular weight by end-group determination is in a range of about 0.2 to about 1.5 fold of the number average molecular weight by GPC determination, preferably in a range of about 0.3 to about 1.2-fold.

The lactic acid-glycolic acid polymer can be prepared, for example, by non-catalytic dehydration polycondensation from lactic acid and glycolic acid (JP-A 61-28521) or ring-opening polymerization with a catalyst from a cyclic compound such as lactide and glycolide (Encyclopedic Handbook of Biomaterials and Bioengineering Part A: Materials, Volume 2, Marcel Dekker, Inc., 1995).

A polymer synthesized by ring-opening polymerization is a polymer having no carboxyl group, but a polymer introduced a terminal free carboxyl group by chemically treating said polymer (J. Controlled Release, vol.41, pp. 249-257, 1996) can be used.

The aforementioned lactic acid-glycolic acid polymer having a terminal free carboxyl group can be prepared by a known process (e.g. non-catalytic dehydration polycondensation method, see JP-A 61-28521) without any problem and, further, a polymer having an unidentified free carboxyl group at the terminal can be prepared by a known process (for example, see WO94/15587).

In addition, as the lactic acid-glycolic acid polymer in which the terminal is converted into a free carboxyl group by chemical treatment after ring-opening polymerization, a polymer available, for example, from Boehringer Ingelheim KG may be used.

The ester of lactic acid-glycolic acid polymer can be prepared, for example, from a lactic acid-glycolic acid polymer having a free carboxyl group by a known-process (for example, see JP-A 7-278018).

These biodegradable polymers may be used alone, or by mixing two or more of them.

The sustained-release preparation of the present invention may contain (D) a multivalent metal, such the multivalent metal is not particularly limited as far as it is a compound having no adverse effect on a living body and, as a metal species, multivalent metals such as divalent (e.g. iron, zinc, copper, calcium, magnesium, aluminium, tin, manganese etc.), trivalent (e.g. iron, aluminium, manganese etc.), and tetravalent (e.g. tin etc.) are used.

In the sustained-release preparation of the present invention, these metals may be present as a compound with an inorganic substance or an organic substance or as a metal oxide (hereinafter, referred to as multivalent metal compound), or may be present as a metal ion, or may form a complex with any one of or both of a physiologically active substance and a biodegradable polymer.

Preferable examples of the multivalent metal include iron, aluminium, zinc, calcium, magnesium and the like. Particularly preferable examples of the multivalent metal include zinc and, inter alia, zinc derived from zinc oxide is preferably used.

As an inorganic substance, for example, hydrogen halide (e.g. hydrochloric acid, hydrobromic acid, hydriodic acid, hydrofluoric acid etc.), and inorganic acid such as sulfuric acid, nitric acid, thiocyanic acid and the like are used.

As an organic substance, for example, organic acid such as aliphatic carboxylic acid and aromatic acid, and acetylacetone and the like are used. As aliphatic carboxylic acid, aliphatic carboxylic acid having 1 to 9 carbons (e.g. aliphatic monocarboxylic acid, aliphatic dicarboxylic acid, aliphatic tricarboxylic acid etc.) is preferably used. Aliphatic carboxylic acid may be any of saturated or unsaturated.

As aliphatic monocarboxylic acid, for example, saturated aliphatic monocarboxylic acid having 1 to 9 carbons (e.g. carbonic acid, acetic acid, propionic acid, butyric acid, valeric acid, caproic acid, enanthic acid, caprylic acid, pelargonic acid, capric acid etc.) and unsaturated aliphatic monocarboxylic acid having 2 to 9 carbons (e.g. acrylic acid, propiolic acid, methacrylic acid, crotonic acid, isocrotonic acid etc.) are used.

As aliphatic dicarboxylic acid, for example, saturated aliphatic dicarboxylic acid having 2 to 9 carbons (e.g. malonic acid, succinic acid, glutaric acid, adipic acid, pimelic acid etc.) and unsaturated aliphatic dicarboxylic acid having 2 to 9 carbons (e.g. maleic acid, fumaric acid, citraconic acid, mesaconic acid etc.) are used.
As aliphatic tricarboxylic acid, for example, saturated aliphatic tricarboxylic acid having 2 to 9 carbons (e.g. tricarballylic acid, 1,2,3-butanetricarboxylic acid etc.) is used.
The aforementioned aliphatic carboxylic acid may have 1 to 2 hydroxy groups, and examples thereof include glycholic acid, lactic acid, glyceric acid, tartronic acid, malic acid, tartaric aid, citric acid, and the like.
Aliphatic carboxylic acid is preferably aliphatic monocarboxylic acid, more preferably aliphatic monocarboxylic acid having 2 to 9 carbons. Particularly preferable examples of aliphatic carboxylic acid include acetic acid.
As aromatic acid, for example, benzoic acid, salicylic acid and phenolsulfonic acid are used.
Examples of the metal compound include:

salts of iron and inorganic acids [e.g. iron halide (e.g. iron chloride, iron bromide, iron iodide, iron fluoride etc.), iron sulfate, iron nitrate, iron thiocyanate etc.], salts of iron and organic acids [e.g. iron aliphatic carboxylate salt (e.g. iron carbonate, iron acetate, iron glycholate, iron lactate, iron tartarate, etc.), aromatic iron salt (e.g. iron benzoate, iron salicylate, iron phenolsulfonate etc.)], iron acetylacetonate and the like,
salts of zinc and inorganic acids [e.g. zinc halide (e.g. zinc chloride, zinc bromide, zinc iodide, zinc fluoride etc.), zinc sulfate, zinc nitrate, zinc thiocyanate, etc.], salts of zinc and organic acids [e.g. zinc aliphatic carboxylate salt (e.g. zinc carbonate, zinc acetate, zinc glycholate, zinc lactate, zinc tartarate etc.), aromatic zinc salt (e.g. zinc benzoate, zinc salicylate, zinc phenolsulfonate etc.)], zinc acetylacetonate and the like,
salts of calcium and inorganic acids [e.g. calcium halide (e.g. calcium chloride, calcium bromide, calcium iodide, calcium fluoride etc.), calcium sulfate, calcium nitrate, calcium thiocyanate, etc.], salts of calcium and organic acids [e.g. calcium aliphatic carboxylate salt (e.g. calcium carbonate, calcium acetate, calcium propionate, calcium oxalate, calcium tartarate, calcium lactate, calcium citrate, calcium gluconate etc.), aromatic calcium salt (e.g. calcium benzoate, calcium salicylate etc.)], calcium acetylacetonate and the like,
salts of magnesium and inorganic acids [e.g. magnesium halide (e.g. magnesium chloride, magnesium bromide, magnesium iodide, magnesium fluoride etc.), magnesium sulfate, magnesium nitrate, magnesium thiocyanate etc.], slats of magnesium and organic acids [e.g. magnesium aliphatic carboxylate salt (e.g. magnesium carbonate, magnesium acetate, magnesium propionate, magnesium oxalate, magnesium tartarate, magnesium lactate, magnesium citrate, magnesium gluconate etc.) aromatic magnesium salt (e.g. magnesium benzoate, magnesium salicylate etc.)], magnesium acetylacetonate and the like, and
metal oxides (e.g. iron oxide, zinc oxide, calcium oxide, magnesium oxide, aluminum oxide, copper oxide, manganese oxide etc.).

As the multivalent metal compound, preferably, iron chloride, iron acetylacetonate, zinc acetate, zinc acetylacetonate, calcium acetate, calcium acetylacetonate, magnesium acetate, magnesium acetylacetonate, zinc oxide and the like are used and, more preferably, zinc acetate and zinc oxide are used.
In the present invention, all or a part of a multivalent metal which may be contained may be used as the same or different kinds of metal salts of a biodegradable polymer. This metal salt of a biodegradable polymer can be prepared by the method described, for example, in JP-A 09-221420 or a similar method thereto.
The "sustained-release preparation containing an AII antagonist", the "sustained-release preparation containing one or more drugs selected from a remedy for hypertension, a hypoglycemic drug, a remedy for hyperlipemia, an antithrombotic drug, a remedy for climacteric disturbance and an anticancer drug" and the "sustained -release medicine containing an AII antagonist and one or more drugs selected from a remedy for hypertension, a hypoglycemic drug, a remedy for hyperlipemia, an antithrombotic drug, a remedy for climacteric disturbance and an anticancer drug" in the aforementioned three preferable forms:

(1) a sustained-release medicine containing (A) an AII antagonist and (B)one more drugs selected from a remedy for hypertension, a hypoglycemic drug, a remedy for hyperlipemia, an antithrombotic drug, a remedy for climacteric disturbance and an anticancer drug,
(2) a sustained-release medicine containing (A) a sustained-release preparation containing an AII antagonist and (B) a sustained-release preparation containing one or more drugs selected from a remedy for hypertension, a hypoglycemic drug, a remedy for hyperlipemia, an antithrombotic drug, a remedy for climacteric disturbance and an anticancer drug,
(3) a sustained-release medicine comprising a combination of (A) a sustained- release preparation containing an AII antagonist and (B) a sustained-release preparation containing one or more drug selected from a remedy for hypertension, a hypoglycemic drug, a remedy for hyperlipemia, an antithrombotic drug, a remedy for climacteric disturbance and an anticancer drug; will be described in detail below.

[Sustained-release preparation containing AII antagonist]

The preferable embodiment of sustained-release preparation containing an AII antagonist includes a sustained-release preparation containing an AII antagonist [e.g. the aforementioned compound having an angiotensin II antagonism, a prodrug thereof or a salt thereof (hereinafter, these are generically referred to as compound having an AII antagonism in some cases)], a biodegradable polymer and, further optionally, a multivalent metal. Production methods thereof include a process of removing a solvent from a solution containing a compound having an AII antagonism, a biodegradable polymer, and optionally, a multivalent metal. By using a complex of either of or both of compound having an AII antagonism and biodegradable polymer and multivalent metal as raw material, a multivalent metal may be contained in the solution, or a multivalent metal may be contained in the solution by adding a multivalent metal compound. Alternatively, a part of or all of the added multivalent metal compound may form a complex with either of or both of the compound having an AII antagonism and biodegradable polymer in the solution.

The content of compound having an AII antagonism in the sustained-release medicine containing an AII antagonist varies depending on a kind of compound having an AII antagonism, a pharmacological effect desired and a duration of effect etc. and, when the sustained-release preparation containing an AII antagonist is composed of two of a compound having an AII antagonism and a biodegradable polymer, the content is usually about 1 to 50% by weight, more preferably about 5 to about 45% by weight relative to the sum of two of the compound having an AII antagonism and the biodegradable polymer. In addition, when the preparation is composed of three of a compound having an AII antagonism, a multivalent metal compound and a biodegradable polymer, the content of compound having an AII antagonism is usually about 1 to about 50% by weight, more preferably about 15 to 45% by weight and, on the other hand, the content of multivalent metal compound is usually about 0 to about 20% by weight, more preferably about 2 to about 15% by weight, relative to the sum of three of the compound having an AII antagonism, the multivalent metal compound and the biodegradable polymer.

A sustained-release medicine containing an AII antagonist may further contain (D') a component obtained by treating a hardly water-soluble multivalent metal compound with water.

The "component obtained by treating a hardly water-soluble multivalent metal compound with water" is a component which is produced by water treatment under a hardly water-soluble multivalent metal compound alone in the coexistence of physiologically active compound (preferably hardly water-soluble physiologically active compound) or/and biodegradable polymer, and release control or stabilization of a physiologically active compound (preferably hardly water-soluble physiologically active compound) can be easily achieved by changing the treating condition, environment and the like. Water treatment is treatment in a short time, for example, within one hour, preferably within 30 minutes, more preferably within 10 minutes.

The amount of water to be added varies depending on an amount of a multivalent metal compound, a kind and an amount of a physiologically active compound, a kind and an amount of a biodegradable polymer, and a kind and a amount of a solution of a biodegradable polymer in an organic solvent, and is selected generally, for example, from a range of 3 to 500%, more preferably 5 to 300% relative to the weight of multivalent metal compound.

Water to be added may be partially insoluble in an organic solvent for a biodegradable polymer, and finer dispersion or emulsification by a known method such as homogenizer and ultrasound is preferable.

If necessary, a multivalent metal compound (e.g. zinc acetate etc.), a basic amino acid (e.g. arginine, histidine, lysine etc.), albumin, gelatin, agar, dextrin, polyvinyl alcohol, carbonic acid, oxalic acid, citric acid, phosphoric acid, hydrochloric acid, sodium hydroxide, citric acid, sodium ethylenediaminetetraacetate, sodium hydrogen sulfite, polyol componud (e.g. polyethylene glycol), paraoxybenzoic acid esters (methylparaben, propylparaben etc.), benzyl alcohol, chlorobutanol, thimerosal and the like may be added to water to be added.

Examples of the component obtained by treating a hardly water-soluble multivalent metal compound with water include a water-treated multivalent metal compound which is hardly water-soluble, obtained by mixing hardly water-soluble multivalent metal compound and water. Herein, a part of or all of the water-treated multivalent metal compound which is hardly water-soluble may be a hardly water-soluble multivalent metal compound (unchanged). In addition, it is preferable that the water-treated multivalent metal compound which is hardly water-soluble forms an emulsion with a solution of biodegradable polymer in an organic solvent under the coexistence of water in a step of preparing the sustained-release preparation of the present invention. It is preferable that the sustained-release preparation of the present invention is prepared via an emulsion containing a physiologically active compound (preferably hardly water-soluble physiologically active compound), a hardly water-soluble multivalent metal compound and water in an inner phase, and a solution of a biodegradable polymer in an organic solvent in an outer phase. And, by forming an emulsion under the coexistence of water, a stable and uniform emulsion system can be prepared in short time.

In the "component obtained by treating a hardly water-soluble multivalent metal compound with water" of the present invention, the same or different multivalent metal (water-untreated multivalent metal compound) as the multivalent metal used as a raw material may be present in a sustained-release preparation containing an AII antagonist, and such the multivalent metal may be present as a multivalent metal compound (a compound with an inorganic substance or an organic substance, or a metal oxide or the like), or as a metal ion, and may form a complex with either of or both of physiologically active compound, a prodrug thereof or a salt thereof and a biodegradable polymer, respectively.

Examples of a preferable process for preparing a sustained-release preparation containing an AII antagonist include a method of removing water and a solvent from a solution containing a physiologically active compound (preferably hardly water-soluble physiologically active compound), a component obtained by treating a hardly water-soluble multivalent metal compound with water, and a biodegradable polymer. By using a complex of either of or both of a physiologically active compound and a biodegradable polymer with a multivalent metal as raw material, a multivalent metal other than a multivalent metal derived from the "component obtained by treating a hardly water-soluble multivalent metal compound with water" may be contained in the solution, or a multivalent metal other than a multivalent metal derived from the "component obtained by treating a hardly water-soluble multivalent metal compound with water" may be contained in the solution by adding to water upon water treatment of a multivalent metal compound in preparation of the "component obtained by treating a hardly water-soluble multivalent metal compound with water". A part or all of the multivalent metal other than the multivalent metal derived from the "component obtained by treating a hardly water-soluble multivalent metal compound" may form a complex with either of or both of a physiologically active compound and a biodegradable polymer in the solution.

Contents of the physiologically active compound and the "compound obtained by treating a hardly water-soluble multivalent metal compound with water" in a sustained-release preparation containing an AII antagonist vary depending on a kind of physiologically active compound, a pharmacological effect desired and a duration of effect and the like, and when a physiologically active compound, a "component obtained by treating a hardly water-soluble multivalent metal compound with water" and a biodegradable polymer are used as a starting raw material, the content of physiologically active compound is usually about 1 to about 50% by weight, more preferably about 15 to about 45% by weight, particularly preferably about 20 to about 40% by weight relative to the sum of the three, on the other hand, the content of a "water-treated multivalent metal compound which is hardly water-soluble" is usually about 0.5 to about 20% by weight, more preferably about 1 to about 15% by weight, particularly preferably about 2 to about 10% by weight.

The form of sustained-release preparation containing an AII antagonist is not particularly limited, but a parenteral preparation is preferable, a percutaneous preparation, an implant and a microcapsule injectable are contemplated, and an injectable preparation using a microcapsule which has a long duration of sustained release and less burden on a patient is preferable.

A process for preparing a sustained-release preparation containing a compound having an AII antagonism and a biodegradable polymer, for example, a microcapsule (hereinafter, referred to as microsphere in some cases) will be exemplified.

(I) In water drying method

(i) O/W method

A compound having an AII antagonism, or further a multivalent metal compound, a "component obtained by treating a hardly water-soluble multivalent metal compound with water" and, optionally, water are added to a solution of biodegradable polymer in an organic solvent so that the weight ratio represented by the aforementioned content is attained, to prepare a solution of biodegradable polymer in an organic solvent which contains a compound having an AII antagonism. Thereupon, the compound having an AII antagonism and multivalent metal compound may remain partially undissolved and may be dispersed in a solution of biodegradable polymer in an organic solvent, respectively, and it is preferable to disperse finely by a known method such as homogenizer and ultrasound.

As the organic solvent, for example, halogenated hydrocarbons (e.g. dichloromethane, chloroform, dichloroethane, trichloroethane, carbon tetrachloride etc.), ethers (e.g. ethyl ether, isopropyl ether etc.), fatty acid ethers (e. g. ethyl acetate, butyl acetate etc.), aromatic hydrocarbons (e.g. benzene, toluene, xylene etc.), alcohols (e.g. ethanol, methanol etc.), and acetonitrile are used. These may be used by mixing at an appropriate ratio. Among them, as halogenated hydrocarbon, dichloromethane is suitable and, as an alcohol, ethanol and methanol are suitable. These may be used by mixing at an appropriate ratio. As an alcohol to be combined with dichloromethane, ethanol is preferable when the compound having an AII antagonism has a tetrazolyl group, and methanol is suitable when the compound has a 4,5-dihydro-5-oxo-1,2,4-oxadiazol-3-yl group.

An additive may be added to the aforementioned organic solvent solution. As the additive, for example, as a solubilizer for retaining the stability and solubility of a drug, carbonic acid, oxalic acid, citric acid, phosphoric acid, hydrochloric acid, sodium hydroxide, arginine, lysine or a salt thereof may be added. In addition, as a stabilizer for drug, albumin, gelatin, citric acid, sodium ethylenediaminetetraacetate, dextrin, sodium hydrogen sulfite, polyol compound such as polyethylene glycol, and the like may be added. Or, as a preservative, paraoxybenzoic acid ethers (e. g. methylparaben, propylparaben etc.), benzyl alcohol, chlorobutanol, thimerosal and the like which are generally used

may be used.

The concentration of biodegradable polymer in the organic solvent solution varies depending on a molecular weight of biodegradable polymer and a kind of an organic solvent, and for example, when dichloromethane is used as an organic solvent, the concentration is generally selected from about 0.5 to about 70% by weight, more preferably about 1 to about 60% by weight, particularly preferably about 2 to about 50% by weight.

In addition, when ethanol or methanol is used as a mixing organic solvent with dichloromethane, the ratio of dichloromethane in the mixed organic solvent is generally selected from about 10 to about 99% by volume, more preferably about 20 to about 98% by volume, particularly preferably about 30 to about 95% by volume.

Then, the resulting solution of biodegradable polymer in an organic solvent which contains a compound having an AII antagonism, or further a multivalent metal compound, a "component obtained by treating a hardly water-soluble multivalent metal compound with water" and, optionally, water is added to the aqueous phase to form an O (oil phase) /W (aqueous phase) emulsion, and then the solvent in the oil phase is evaporated to prepare a microcapsule. The aqueous phase volume thereupon is generally selected from about 1-fold to about 10.000-fold, more preferably about 5-fold to about 5,000-fold, particularly preferably about 10-fold to about 2,000-fold of the oil phase volume.

An emulsifier may be added to the aforementioned outer aqueous phase. The emulsifier may be any one as far as it can generally form a stable O/W emulsion. Specifically, for example, anionic surfactants (sodium oleate, sodium stearate, sodium laurylsulfate etc.), nonionic surfactants (polyoxyethylene sorbitan fatty acid esters [Tween80, Tween60, Atlas Powder], polyoxyethylene castor oil derivative [HCO-60, HCO-50, Nikko Chemicals] etc.), polyvinylpyrrolidone, polyvinyl alcohol, carboxymethylcellulose, lecithin, gelatin, and hyaluronic acid are used. Among them, one kind may be used or two or more kinds may be used in combination. The concentration upon use is preferably in a range of about 0.01 to about 10% by weight, more preferably in a range of about 0.05 to about 5% by weight.

An osmotic pressure controlling agent may be added to the aforementioned outer aqueous phase. The osmotic pressure controlling agent may be any one as far as it has an osmotic pressure in an aqueous solution.

Examples of the osmotic regulator include polyhydric alcohols, monohydric alcohols, monosaccharides, disaccharides, oligosaccharides, amino acids and derivatives thereof.

As the aforementioned polyhydric alcohols, for example, dihydric alcohols such as glycerin and the like, pentahydric alcohols such as arabitol, xylitol, adonitol and the like, and hexahydric alcohols such as mannitol, sorbitol, dulcitol and the like are used. Inter alia, hexahydric alcohols are preferable, and mannitol is particularly suitable.

Examples of the monohydric alcohols above include methanol, ethanol and isopropyl alcohol, and among them, methanol is preferable.

As the monosaccharides above, for example, pentoses such as arabinose, xylose, ribose, 2-deoxyribose and the like, hexoses such as glucose, fructose, galactose, mannose, sorbose, rhamnose, fucose and the like are used, and among them, hexoses are preferable.

As the oligosaccharides above, for example, trisaccharides such as maltotriose, raffinose and the like, tetrasaccharides such as stachyose and the like are used and, among them, trisaccharides are preferable.

As the above-mentioned derivatives of monosaccharides, disaccharides and oligosaccharides, for example, glucosamine, glactosamine, glucuronic acid, and galacturonic acid are used.

As the above-mentioned amino acids, any amino acids can be used as far as they are L-amino acids, and examples thereof include glycine, leucine and arginine. Among them, L-arginine is preferable.

These osmotic pressure controlling agents may be used alone, or by mixing them.

These osmotic regulators are used at such the concentration that the osmotic pressure of outer aqueous phase becomes about 1/50-fold to about 5-fold, preferably about 1/25 to about 3-fold of the osmotic pressure of physiological saline.

As a method of removing a solvent such as organic solvent and water, methods known per se or similar methods thereto are used. Examples thereof include a method of evaporating a solvent at a normal pressure or by reducing a pressure gradually while stirring with a propeller-type stirrer or a magnetic stirrer, and a method of evaporating a solvent while controlling the degree of vacuum using a rotary evaporator.

The thus obtained microcapsule is collected by centrifugation or filtration. Then the compound having an AII antagonism, drug retaining substance, emulsifier and the like which are attached on the surface of microcapsule are washed with distilled water several times, and the resulting microcapsule is dispersed again in distilled water to lyophilize.

During a production process, a coagulation preventing agent may be added in order to prevent mutual coagulation of particles. As the coagulation preventing agent, for example, water-soluble polysaccharides such as mannitol, lactose, glucose and starches (e.g. corn starch etc.), amino acid such as glycine, and proteins such as fibrin and collagen are used. Among them, mannitol is suitable.

In addition, after lyophilization, if necessary, water and organic solvent in a microcapsule may be removed by warming under such the conditions that microcapsules are not mutually fused under reduced pressure. Preferably, warming is performed at a temperature slightly higher than the intermediate point glass transition temperature of a

biodegradable polymer measured by a differential scanning calorimeter, under the conditions of a temperature rising rate of 10 to 20°C per minute. More preferably, warming is performed within the temperature range from an intermediate point glass transition temperature of biodegradable polymer to a temperature higher by about 30°C than the intermediate point glass transition temperature. Inter alia, when a lactic acid-glycholic acid polymer is used as a biodegradable polymer, warming is preferably performed at a temperature within the range from an intermediate point glass transition temperature to a temperature higher than the intermediate point glass transition temperature by 10°C, more preferably within the range from an intermediate point glass transition temperature to a temperature higher than the intermediate point glass transition temperature by 5°C.

Though the warming time varies depending on the amount of microcapsules and the like, and it is generally about 12 hours to about 168 hours, preferably about 24 hours to about 120 hours, particularly preferably about 48 hours to about 96 hours, after the microcapsule itself has reached a given temperature.

A warming method is not particularly limited provided that a set of microcapsules can be uniformly heated.

As the warm drying method, for example, a method of warm drying in a constant temperature chamber, fluidized chamber, moving chamber or kiln, a method of warm drying with microwave, and the like are used. Among them, a method of warm drying in a constant temperature chamber is preferable.

In addition, removal of water and organic solvent in a microcapsule can be performed by a method using supercritical fluid ($CO_2$ etc.) or carbon dioxide in high pressure gas state.

(ii) W/O/W method

A compound having an AII antagonism is dissolved in water and, if necessary, a drug retaining substance such as a multivalent metal compound (e.g. zinc acetate), a basic amino acid (e.g. arginine, histidine, lysine), gelatin, agar and polyvinyl alcohol are added thereto to dissolve, which is used as an inner aqueous phase.

The concentration of drug in the inner aqueous phase is generally selected from about 0.1 to 80% by weight, more preferably about 1 to 70% by weight, particularly preferably about 2 to 60% by weight.

As the pH adjusting agent for retaining the stability and solubility of drug, carbonic acid, oxalic acid, citric acid, phosphoric acid, hydrochloric acid, sodium hydroxide, arginine, lysine or a salt thereof, or the like may be added to the inner aqueous phase. In addition, as the stabilizing agent for drug, albumin, gelatin, citric acid, sodium ethylenediaminetetraacetate, dextrin, sodium hydrogen sulfite, or a polyol compound such as polyethylene glycol may be further added. Or, as a preservative, paraoxybenzoic acid ethers (methylparaben, propylparaben etc.), benzyl alcohol, chlorobutanol, thimerosal and the like which are generally used may be added.

The thus obtained inner aqueous phase is added to a solution (oil phase) of biodegradable polymer in an organic solvent which may contain a multivalent metal if necessary, and this mixture is emulsified by a known method such as homogenizer and ultrasound to form a W/O emulsion.

As the aforementioned organic solvent, for example, halogenated hydrocarbons (e.g. dichloromethane, chloroform, dichloroethane, trichloroethane, carbon tetrachloride etc.), ethers (e.g. ethyl ether, isopropyl ether etc.), fatty acid ethers (e.g. ethyl acetate, butyl acetate etc.), aromatic hydrocarbons (e.g. benzene, toluene, xylene etc.), alcohols (e.g. ethanol, methanol etc.), and acetonitrile are used. These may be used by mixing at an appropriate ratio. Among them, halogenated hydrocarbons are preferable, and dichloromethane is particularly suitable.

The concentration of biodegradable polymer in an organic solvent solution varies depending on a molecular weight of biodegradable polymer and a kind of organic solvent and, for example, when dichloromethane is used as an organic solvent, the concentration is generally selected from about 0.5 to about 70% by weight, more preferably about 1 to about 60% by weight, particularly preferably about 2 to about 50% by weight.

Then, the resulting W/O emulsion containing a compound having an AII antagonism and a biodegradable polymer is added to an aqueous phase (outer aqueous phase) to form a W (inner aqueous phase)/O (oil phase)/W (outer aqueous phase) emulsion, and then, a solvent in the oil phase is evaporated to prepare a microcapsule. Thereupon, the volume of outer aqueous phase is generally selected from about 1-fold to about 10,000-fold, more preferably about 5-fold to about 5,000-fold, particularly preferably about 10-fold to about 2,000-fold of the volume of oil phase.

The above-mentioned emulsifier and osmotic pressure controlling agent which may be added to the outer aqueous phase, and the subsequent preparation method are the same as those described in the above (I) (i).

(II) Phase separation method

When a microcapsule is prepared by the present method, a coacervation agent is gradually added while stirring into an organic solvent solution of biodegradable polymer containing a compound having an AII antagonism, or further a multivalent metal compound, a "component obtained by treating a hardly water-soluble multivalent metal compound with water" and, optionally, water which is described in the in-water drying method of the aforementioned (I), to precipitate and solidify a microcapsule. The amount of the coacervation agent is selected from about 0.01 to

1,000-fold, preferably about 0.05 to 500-fold, particularly preferably about 0.1 to 200-fold of the volume of oil phase.

The coacervation agent is not particularly limited as far as it is a compound of polymer-based, mineral oil-based or vegetable oil-based compounds which is miscible with an organic solvent and does not dissolve both of a compound having an AII antagonism and a biodegradable polymer. Specifically, for example, a silicone oil, a sesame oil, a soybean oil, a corn oil, a cotton seed oil, a coconut oil, a linseed oil, a mineral oil, n-hexane and n-heptane are used. These may be used in admixture of two or more of them.

The thus obtained microcapsule is separated, then, washed with heptane or the like repeatedly to remove the coacervation agent and the like other than the compound having an AII antagonism and biodegradable polymer, followed by drying under reduced pressure. Alternatively, washing is effected in the same manner as in the above-mentioned in-water drying method of section (I)(i), then, freeze-dried, further, dried under heating.

(III) Spray drying method

When a microcapsule is prepared by the present method, the organic solvent solution of biodegradable polymer which contains a compound having an AII antagonism, or further a multivalent metal compound, a "component obtained by treating a hardly water-soluble multivalent metal compound with water" and, optionally, water which is described in the above-mentioned in-water drying method of (I), is sprayed into a drying chamber of a spray dryer using a nozzle, and an organic solvent in a micronized liquid droplet is evaporated in an extremely short period of time to prepare a microcapsule. As the nozzle, for example, two-fluid nozzle type, pressure nozzle type, rotation disc type and the like are mentioned. Thereafter, if necessary, washing may be effected in the same manner as in the above-mentioned in-water drying method of (I), then, freeze-dried, further, dried under heat.

As a dosage form other than the aforementioned microcapsule, the organic solvent solution of biodegradable polymer containing a compound having an AII antagonism, or further a multivalent metal compound, a "component obtained by treating a hardly water-soluble multivalent metal compound with water" and, optionally, water which is described in the in-water drying method for the microcapsule production method (I) may be dried to solid by evaporating an organic solvent or water while controlling the degree of vacuum using a rotary evaporator, then, ground by a jet mill and the like to give fine particles.

Further, the finely ground particles may be washed in the same manner as in the in-water drying method of the microcapsule production method (I), then, freeze-dried, further, dried under heat.

In thus obtained microcapsule or fine particles, drug release can be controlled in response to degradation rate of a biodegradable polymer used or a kind and amount of multivalent metal compound added.

The sustained-release preparation containing an AII antagonist can be administered as it is or, by preparing various dosage forms using it as a raw material in combination with (B) a sustained-release preparation containing one or more drugs selected from a remedy for hypertension, a hypoglycemic drug, a remedy for hyperlipemia, an antithrombotic drug, a remedy for climacteric disturbance and an anticancer drug, as a single preparation or separate preparations such as injectables or preparations for implantation into muscle, hypodermis, organs and the like, preparations for administering to mucosa onto cavitas nasi, rectum, uterus and the like, oral preparations (e.g. solid preparation such as capsule (e.g. hard capsule, soft capsule etc.), granule, powder etc., liquid preparation such as syrup, emulsion, suspension etc.) and the like. Alternatively, the preparation can be administered with a jet injector.

For example, for formulating the sustained-release preparation containing an AII antagonist into an injectable, a practically usable sustained-release injectable can be obtained by formulating the preparation together with a dispersing agent (surfactant such as Tween 80, HCO-60 etc, polysaccharides such as sodium hyaluronate, carboxymethylcellulose, sodium alginate etc.), a preservative (e.g. methylparaben, propylparaben etc.), an isotonic (e.g. sodium chloride, mannitol, sorbitol, glucose, proline etc,) and the like into an aqueous suspension, or dispersing with a vegetable oil such as sesame oil, corn oil and the like into an oily suspension.

When used as a suspended injectable, the particle diameter of the sustained-release preparation containing an AII antagonist may be within the range satisfying the requirements for the degree of dispersion and the needle passability for the injection and, for example, it is in a range of about 0.1 to 300 µm, preferably about 0.5 to 150 µm, more preferably about 1 to 100 µm.

Methods for preparing the sustained-release preparation containing an AII antagonist as a sterile preparation include, but are not limited to, a method in which the entire production process is sterile, a method of sterilizing with gamma-ray, and a method of adding an antiseptic.

Since the sustained-release preparation containing an AII antagonist is low toxic, it can be used as a safe medicine to a mammal (e.g. human, cow, pig, dog, cat, mouse, rat, rabbit etc.).

A dose of the sustained-release preparation containing an AII antagonist varies variously depending on a kind and content of compound having an AII antagonism which is a basic active ingredient, a kind and content of concomitant drug, dosage form, duration time of release of compound having the AII antagonism, subject disease and subject animal, and may be an effective amount of a compound having an AII antagonism. For example, when the

sustained-release preparation is a one month-preparation, the single dose of compound having an AII antagonism can be appropriately selected from a range of, preferably, about 0.01 mg to 10 mg/kg body weight, more preferably about 0.05 mg to 5 mg/kg body weight per adult.

The single dose of sustained-release preparation containing an AII antagonist can be appropriately selected from a range of, preferably, about 0.05 mg to 50 mg/kg body weight, more preferably about 0.1 to 30 mg/kg body weight per adult.

The administration time can be appropriately selected depending on a kind and content of compound having an AII antagonism, dosage form, duration time of release of a compound having an AII antagonism, subject disease, and subject animal, such as once per a few days, once per a few weeks, once per a month, and once per a few months (e.g. three months, four months, six months, etc.).

[Sustained release preparation containing one or more drugs selected from a remedy for hypertension, a hypoglycemic drug, a remedy for hyperlipemia, an antithrombotic drug, a remedy for climacteric disturbance and an anticancer drug]

The sustained-release preparation containing one or more (preferably two to three) drugs (hereinafter, abbreviated as concomitant drug in some cases) selected from a remedy for hypertension, a hypoglycemic drug, a remedy for hyperlipemia, an antithrombotic drug, a remedy for climacteric disturbance and an anticancer drug can be similarly formulated into a sustained-release preparation (e.g. microcapsule, microcapsule injectable, preparation for implantation etc.) by replacing the "AII antagonist" (e.g. the aforementioned compound having an angiotensin II antagonism, a prodrug thereof or a salt thereof) in the "sustained-release preparation containing an AII antagonist" with "one or more (preferable two to three) drugs selected from a remedy for hypertension, a hypoglycemic drug, a remedy for hyperlipemia, an antithrombotic drug, a remedy for climacteric disturbance and an anticancer drug".

The sustained-release preparation containing a concomitant drug can be administered as it is or, by preparing various dosage forms using it as a raw material, in combination with (A) a sustained-release preparation containing an AII antagonist, as a single preparation or separate preparations such as injectables or preparations for implantation into muscle, hypodermis, organs and the like, preparations for administering to mucosa onto cavitas nasi, rectum, uterus and the like, oral preparations (e.g. solid preparation such as capsule (e.g. hard capsule, soft capsule etc.), granule, powder etc., liquid preparation such as syrup, emulsion, suspension etc.) and the like.

Since the sustained-release preparation containing a concomitant drug is low toxic, it can be used as a safe medicine to a mammal (e.g. human, cow, pig, dog, cat, mouse, rat, rabbit etc.).

The dose of sustained-release preparation containing a concomitant drug differs variously depending on a king and content of a drug to be used together, dosage form, duration time of release of a concomitant drug, subject disease, and subject animal, and may be an effective amount of each concomitant drugs. For example, when the sustained-release preparation is a one month-preparation, the single dose of concomitant drug can be appropriately selected from a range of, preferably, about 0.01 mg to 200 mg/kg body weight, more preferably about 0.05 mg to 150 mg/kg body weight per adult.

The single dose of sustained-release preparation containing a concomitant drug can be appropriately selected preferably from a range of about 0.05 mg to 50 mg/kg body weight per adult.

The administration time can be appropriately selected depending on a kind and content of a concomitant drug, dosage form, duration time of release of a concomitant drug, subject disease and subject animal, such as once per a few days, once per a few weeks, once per a month, and once per a few months (e.g. three months, four months, six months).

One specific example of formulation method and a method of implementation will be shown below by way of GnRH agonist and antagonist as an anticancer drug.

The aforementioned GnRH agonist or antagonist (preferably, a peptide represented by the formula 5-oxo-Pro-His-Trp-Ser-Tyr-DLeu-Leu-Arg-Pro-NH-$C_2H_5$ or a salt thereof (hereinafter, simply referred to as "leuprorelin or a slat thereof" in some cases)), more preferably leuprorelin acetate is preferably administered as a sustained-release injectable. In addition, when a sustained-release preparation is a sustained-release microcapsule, the preparation is preferably a long term sustained-release microcapsule which releases GnRH agonist or antagonist over 2 months or longer.

The aforementioned sustained-release preparation (in particular, sustained-release microcapsule) containing a GnRH agonist or antagonist (preferably, leuprorelin or a salt thereof, more preferably leuprorelin acetate) can be prepared by a method known per se, for example, the methods described in JP-A 60-100516, JP-A 62-201816, JP-A 4-321622, JP-A 6-192068, JP-A 9-132524, JP-A 9-221417, JP-A 11-279054, and W099/360099.

Among the above sustained-release preparations, the "long term sustained-release microcapsule which releases a physiologically active substance according to a zero-order kinetics over 2 months or longer" described in JP-A 4-321622 is particularly preferably used.

One example of a process for preparing the sustained-release microcapsule will be described below.

First, a GnRH agonist or antagonist (preferably, leuprorelin or a salt thereof, more preferably, leuprorelin acetate) is dissolved in water at about 20% to 70% (W/W), preferably 25 to 65% (W/W), more preferably 35 to 60% (W/W) and, if necessary, a drug retaining substance such as gelatin and basic amino acid is dissolved or suspended therein to form an inner aqueous phase solution.

To this inner aqueous phase solution may be added carbonic acid, acetic acid, oxalic acid, citric acid, phosphoric acid, hydrochloric acid, sodium hydroxide, arginine, lysine, a salt thereof and the like as a pH adjusting agent for retaining the stability and solubility of GnRH agonist or antagonist (preferably, leuprorelin or a salt thereof, more preferably leuprorelin acetate). Further, as a stabilizing agent for a GnRH agonist or antagonist (preferably leuprorelin or a salt thereof, more preferably leuprorelin acetate), albumin, gelatin, citric acid, sodium ethylenediaminetetraacetate, dextrin, sodium hydrogen sulfite, polyol compounds such as polyethylene glycol and the like may be added, or as a preservative, paraoxybenzoic acid esters (methylparaben, propylparaben etc.), benzyl alcohol, chlorobutanol and thimerosal may be added.

The thus obtained inner aqueous phase solution is added to a solution (oil phase) containing a high-molecular polymer and then emulsification procedure is carried out to prepare a W/O emersion. As the emulsification procedure, a known dispersing method is used, and examples thereof include an intermittent shaking method, a method using a mixer such as a propeller-type stirrer and a turbine-type stirrer, a colloid mill method, a homogenizer method, and an ultrasound irradiation method.

Then, the thus prepared W/O emulsion is subjected to a microcapsulation step and, as the step, an in water drying method or a phase separating method can be applied. When a microcapsule is prepared by an in water drying method, the W/O emulsion is further added to an aqueous phase which is the third phase, to form a three-phase emulsion of W/O/W, and a solvent in the oil phase is evaporated to prepare a microcapsule.

An emulsifier may be added to the aforementioned outer aqueous phase, and any emulsifiers may be used as far as they form a generally stable O/W emulsion. Examples thereof include anionic surfactants (sodium oleate, sodium stearate, sodium laurylsulfate etc.), nonionic surfactants (polyoxyethylene sorbitan fatty acid ester [Tween 80, Tween 60, Atlas Powder], polyoxyethylene castor oil derivative [HCO-60, HCO-50, Nikko Chemicals] etc.), polyvinylpyrrolibone, polyvinyl alcohol, carboxymethylcellulose, lecithin, and gelatin. One kind of them may be used, or some of them may be used in combination. Upon use, the concentration can be appropriately selected from a range of about 0.01 % to 20 %, more preferably 0.05 % to 10 %.

For evaporating a solvent in oil phase, normally used methods are adopted. As the method, evaporation is performed by gradually reducing a pressure while stirring with a propeller-type stirrer or a magnetic stirrer, or by using a rotary evaporator while controlling the degree of vacuum. In this case, at a time when solidification of a high-molecular polymer has proceeded to an extent, evaporation is performed by gradually warming the W/O/W emulsion in order to make desorption of a solvent more complete, thereby, a necessary time can be shortened.

The thus obtained microcapsule is separated by centrifugation or filtration, thereafter, a free GnRH agonist or antagonist (preferably, leuprorelin or a salt thereof, more preferably leuprorelin acetate), a drug retaining substance, an emulsifier and the like which are attached to the surface of microcapsule are repeatedly washed several times with distilled water, and then dispersed again in distilled water to lyophilize. Thereupon, a coagulation preventing agent (e. g. mannitol, sorbitol, lactose, glucose etc.) may be added. Removal of water and an organic solvent in a microcapsule is performed more completely under reduced pressure, if necessary, by warming.

When a microcapsule is prepared by a phase separation method, a coacervation agent is gradually added to the W/O emersion under stirring, to precipitate and solidify a high-molecular polymer.

Any coacervation agents may be used as far as they are polymer, mineral oil or vegetable oil compounds which are miscible with a solvent for high-molecular polymer and do not dissolve a polymer for capsulation. Examples thereof include a silicone oil, a sesame oil, a soybean oil, a corn oil, a cottonseed oil, a coconut oil, a linseed oil, a mineral oil, n-hexane and n-heptane. These may be used by mixing two or more of them.

The thus obtained microcapsule is separated by filtration, and repeatedly washed with heptane or the like to remove coacervation agent. Further, removal of free drug and removal of solvent are performed by the same method as in water drying method. In order to prevent mutual coagulation of particles during washing, an coagulation preventing agent may be added.

The above-obtained microcapsule is slightly ground if needed, and sieved to remove a too large microcapsule. For the particle diameter of microcapsule, average diameter is in a range of about 0.5 to 1000 μm, more preferably, a range of about 2 to 500 μm is desired. When used as a suspension injectable, the diameter may be in such a range that its dispersibility and needle passability are satisfied and, for example, a range of about 2 to 100 μm is desirable.

As the aforementioned high-molecular polymer, a biodegradable polymer, for example, a polymer or a copolymer which is synthesized from one or more of α-hydroxycarboxylic acids such as α-hydroxymonocarboxylic acids (e. g. glycolic acid, lactic acid etc.), α-hydroxydicarboxylic acids (e.g. malic acid), α-hydroxytricarboxylic acids (e.g. citric acid) and the like and has a free carboxyl group, or a mixture thereof; poly(α-cyanoacrylic acid ester); polyamino acid (e.g. poly(γ-benzyl-L-glutamic acid) etc.); anhydrous maleic acid copolymer (e.g. styrene-maleic acid copolymer etc.)

are used.

The bonding pattern of monomer may be any of random, block and graft. In addition, in the case that the aforementioned α-hydroxymonocarboxylic acids, α-hydroxydicarboxylic acids, or α-hydroxytricarboxylic acids have an optically active center in a molecule, any of D-, L- and DL-isomers may be used. Among them, lactic acid-glycolic acid polymer (hereinafter, referred to as poly(lactide-co-glycolide), poly(lactic acid-co-glycolic acid) or lactic acid-glycolic acid copolymer in some cases and, unless otherwise is indicated, homopolymer (polymer) and copolymer of lactic acid and glycolic acid are referred generically; alternatively, lactic acid homopolymer is referred to as lactic acid polymer, polylactic acid, polylactide or the like, and glycolic acid homopolymer is referred to as glycolic acid polymer, polyglycolic acid polyglycolide or the like, in some cases), poly(α-cyanoacrylic acid ester) and the like are preferable. Further preferable is a lactic acid-glycolic acid polymer, and more preferable is a lactic acid-glycolic acid polymer having a free carboxyl group at a terminal.

The biodegradable polymer may be a salt. Examples of the salt include salts with inorganic basis (e.g. alkali metal such as sodium, potassium etc., alkaline earth metal such as calcium, magnesium etc.) or organic bases (e.g. organic amines such as triethylamine etc., basic amino acids such as arginine), as well as salts and complexes with transition metals (e.g. zinc, iron, copper etc.).

In the case that a lactic acid-glycolic acid polymer is used as a biodegradable polymer, a composition ratio (mol%) is preferably about 100/0 to about 40/60, more preferably about 100/0 to about 50/50. In addition, in the case of a long term sustained-release microcapsule which releases a physiologically active substance according to a zero-order kinetics over 2 months, a lactic acid homopolymer having a composition ratio of 100/0 is used preferably.

For an optical isomer ratio of lactic acid which is one of minimum repetition units of the "lactic acid-glycolic acid polymer", the D-isomer/L-isomer (mol/mol%) is preferably within the range of about 75/25 to about 25/75. D-isomer/L-isomer (mol/mol%) of in the range of particularly about 60/40 to about 30/70 is used generally.

A weight average molecular weight of the "lactic acid-glycolic acid polymer" is usually about 3,000 to about 100,000, preferably about 3,000 to about 60,000, more preferably about 3,000 to about 50,000.

In addition, for the dispersity (weight average molecular weight/number average molecular weight), about 1.2 to about 4.0 is usually preferred, and about 1.5 to 3.5 is more preferred.

For the amount of free carboxyl group of the "lactic acid/glycolic acid polymer", about 20 to about 1,000 μmol (micromole) per unit mass (gram) of polymer is usually preferred, and about 40 to about 1,000 μmol (micromole) is particularly preferred.

The aforementioned weight average molecular weight, number average molecular weight and dispersity refer to the molecular weight in terms of polystyrene measured by gel permeation chromatography (GPC) using, as standard substance, 15 kinds of monodisperse polystyrenes having weight average molecular weight of 1,110,000, 707,000, 455,645, 354,000, 189,000, 156,055, 98,900, 66,437, 37,200, 17,100, 9,830, 5,870, 2,500, 1,303 and 504, and the dispersity calculated therefrom. Measurement is carried out by using a high speed GPC apparatus (manufactured by Tosoh Corporation, HLC-8120GPC, detection method is by differential refractive index) and GPC column KF804L x 2 (manufactured by SHOWA DENKO K.K.), and chloroform as a mobile phase. The flow rate is 1 ml/min.

The aforementioned amount of free carboxyl group refers to the amount obtained by a labeling method (hereinafter, referred to as "carboxyl group amount according to labeling method"). Specifically, in the case of polylactic acid, Wmg of polylactic acid is dissolved in 2ml of 5N hydrochloric acid/acetonitrile (v/v = 4/96) mixed solution, and 2ml of 0.01M o-nitrophenylhydrazine hydrochloride (ONPH) solution (5N hydrochloric acid/acetonitrile/ethanol = 1.02/35/15) and 2ml of 0.15M 1-ethyl-3-(3-dimethylaminopropyl)carbodiimide hydrochloride solution (pyridine/ethanol = 4 v/96 v) are added thereto, followed by reacting at 40°C for 30 minutes. Then solvent is distilled off. The residue is washed with water (four times), dissolved in 2 ml of acetonitrile, and 1ml of 0.5 mol/l ethanolic potassium hydroxide solution is added to react at 60°C for 30 minutes. The reaction solution is diluted with 1.5N aqueous sodium hydroxide solution to Yml, and the absorbance A (/cm) at 544 nm is measured using 1.5N aqueous sodium hydroxide solution as a control. On the other hand, the amount of free carboxyl group (Cmol/L) is determined by alkali titration using aqueous DL-lactic acid solution as a standard, and the absorbance at 544 nm of DL-lactic acid hydrazide obtained by a ONPH labeling method is assigned to B (/cm), then the mol amount of free carboxylic group per unit mass (gram) of polymer is obtained by the following equation.

$$[COOH](mol/g) = (AYC)/(WB)$$

Alternatively, the "carboxyl group amount" may be obtained by dissolving a biodegradable polymer in a toluene-acetone-methanol mixed solvent, and titrating the carboxyl group in this solution with an alcoholic potassium hydroxide solution using phenolphthalein as an indicator (hereinafter, the value obtained by this method is referred to as "carboxyl group amount according to alkali titrating method"). However, a hydrolysis reaction of polyester main chain competes during titration and, as a result, there is a possibility that titration end point becomes unclear, and it is desirable

to quantify by the aforementioned labeling method.

The "lactic acid-glycolic acid polymer" can be prepared, for example, by non-catalytic dehydration polycondensation from lactic acid and glycolic acid (JP-A 61-28521) or ring-opening polymerization using a catalyst from a cyclic diester compound such as lactide and glycolide (Encyclopedic Handbook of Biomaterials and Bioengineering Part A: Materials, Volume 2, Marcel Dekker, Inc. 1995). The polymer obtained by the aforementioned known ring-opening polymerization method does not necessarily have a free carboxyl group at a terminal of the obtained polymer. However, the polymer can be converted into a polymer having a carboxyl group amount to an extent per unit mass, for example, by subjecting to a hydrolysis reaction described in EP-A-0839525, and this can be used.

The aforementioned "lactic acid-glycolic acid polymer having a free carboxyl group at a terminal" can be prepared by the same method as a known process (for example, see non-catalytic dehydration polycondensation method, JP-A 61-28521) or a similar method thereto.

For formulating the microcapsule into an injectable, a practically usable sustained-release injectable can be obtained by formulating the microcapsule together with a dispersing agent (e.g. Tween 80, HCO-60, carboxymethylcellulose, sodium alginate etc), a preservative (e.g. methylparaben, propylparaben etc.), and an isotonic (e.g. sodium chloride, mannitol, sorbitol, glucose etc.) and the like into an aqueous suspension, or dispersing with a vegetable oil such as sesame oil, corn oil and the like into an oily suspension.

The sustained-release preparation containing the aforementioned GnRH agonist or antagonist (preferably leuprorelin or a salt thereof, more preferably leuprorelin acetate) (preferably, a preparation containing a sustained-release microcapsule containing leuprorelin or a salt thereof (preferably leuprorelin acetate)), in combination with a sustained-release preparation containing an AII antagonist, can be easily administered subcutaneously, intramuscularly or intravascularly (preferably subcutaneously) as an injectable or preparation for implantation (preferably injectable). Alternatively, the preparation may be administered by molding into various preparations described above, and can be used as a raw material upon production of such the preparation.

In addition, the dose of sustained-release preparation containing GnRH agonist or antagonist varies variously depending on a content of GnRH agonist or antagonist (preferably leuprorelin or a salt thereof, more preferably leuprorelin acetate), a dose of sustained-release preparation containing an AII antagonist to be used together, dosage form, duration time of GnRH agonist or antagonist (preferably, leuprorelin or a salt thereof, more preferably leuprorelin acetate), an administration subject animal [e.g. warm-blooded mammal (e.g. human, mouse, rat, rabbit, sheep, pig, cow, horse etc.)] and the like, and may be an effective amount of the GnRH agonist or antagonist (preferably, leuprorelin or a salt thereof, more preferably leuprorelin acetate) as an agent for preventing or treating prostate cancer or breast cancer. For example, a single dose to the aforementioned warm-blooded mammal can be appropriately selected from a range of about 0.01 mg to 100/kg body weight, preferably about 0.02 mg 50 mg/kg body weight, more preferably 0.05 mg to 20 mg/kg body weight.

In addition, when a sustained-release preparation containing the aforementioned GnRH agonist or antagonist is administered as an injectable, for example, in an adult prostate cancer patient (weight 60 kg), usually around about 0.01 to 50 mg, preferably around 0.1 to 20 mg, more preferably around about 0.1 to 15 mg of a GnRH agonist or antagonist (preferably, leuprorelin or a salt thereof, more preferably leuprorelin acetate) may be subcutaneously or intramuscularly administered per once. In addition, for example, when a preventing or treating agent for prostate cancer containing a sustained-release microcapsule containing the aforementioned GnRH agonist or antagonist (preferably leuprorelin or a salt thereof, more preferably leuprorelinacetate) is administered as an injectable, the dose varies depending on a drug sustained-release term of sustained-release microcapsule and, for example, when administered once per about one month, for example, in an adult prostate cancer patient (weight 60 kg), usually around about 0.01 to 20 mg, preferably around about 0.1 to 10 mg, more preferably around about 0.1 to 5 mg of GnRH agonist or antagonist (preferably, leuprorelin or a salt thereof, more preferably leuprorelin acetate) may be subcutaneously or intramuscularly administered per once and, for example, when administered once per about 3 months, for example, in an adult prostate cancer patient (weight 60 kg), usually around about 0.1 to 30 mg, preferably around about 0.1 to 20 mg, more preferably around about 1 to 15 mg of GnRH agonist or antagonist (preferably leuprorelin or a salt thereof, more preferably leuprorelin acetate) may be subcutaneously or intramuscularly administered per once.

In the case of other animals, an amount in terms of body weight 60 kg may be administered.

Alternatively, the sustained-release preparation containing GnRH agonist or antagonist (preferably leuprorelin or a salt thereof, more preferably leuprorelin acetate) can be administered together with an anti-estrogen agent (e.g. Tamoxifen etc.) for preventing or treating prostate cancer, breast cancer, and the like in addition to combining with a sustained-release preparation containing an AII antagonist.

[Sustained-release medicine containing an AII antagonist, and one or more drugs selected from a remedy for hypertension, a hypoglycemic drug, a remedy for hyperlipemia, an antithrombotic drug, a remedy for climacteric disturbance and an anticancer drug]

The sustained-release preparation containing an AII antagonist and one or more (preferably two to three) drugs selected from a remedy for hypertension, a hypoglycemic drug, a remedy for hyperlipemia, an antithrombotic drug, a remedy for climacteric disturbance and an anticancer drug (hereinafter, abbreviated as AII antagonist and concomitant drug in some cases) can be similarly formulated into a sustained-release preparation (e.g. microcapsule, microcapsule injectable, preparation for implantation etc.) by adding (mixing) further "one or more (preferably two to three) drugs selected from a remedy for hypertension, a hypoglycemic drug, a remedy for hyperlipemia, an antithrombotic drug, a remedy for climacteric disturbance and an anticancer drug" to the "AII antagonist" in the aforementioned "sustained-release preparation containing an AII antagonist", and can be implemented.

Contents of the AII antagonist and concomitant drug in a sustained-release preparation containing an AII antagonist and a concomitant drug differs depending on a kind of AII antagonist and concomitant drug, desired pharmacological effect, duration term of effect and the like, and when the sustained-release preparation (pharmaceutical composition) containing an AII antagonist and a concomitant drug is composed of three of an AII antagonist, a concomitant drug and a biodegradable polymer, the composition ratios of AII antagonist, concomitant drug and biodegradable polymer relative to a whole preparation are about 5 to about 50% by weight (preferably, about 5 to about 40% by weight), about 0.1 to about 50% by weight (preferably about 0.1 to about 40% by weight) and about 0.1 to about 95% by weight (preferably about 1 to about 90% by weight), respectively. The ratio of physiologically active compound combined an AII antagonist and a concomitant drug relative to the sum of three of an AII antagonist, a concomitant drug and a biodegradable polymer is usually about 1 to about 90% by weight, more preferably about 5 to about 80% by weight.

In addition, when the sustained-release preparation containing an AII antagonist and a concomitant drug is composed of four of an AII antagonist, a concomitant drug, a multivalent metal compound and a biodegradable polymer, the composition ratios of an AII antagonist, a concomitant drug, a multivalent metal compound and a biodegradable polymer relative to a whole preparation are about 1 to about 50% by weight (preferably about 15 to 45% by weight), about 0.1 to about 50% by weight (preferably about 0.1 to about 45% by weight), about 0.1 to about 20% by weight (preferably about 2 to about 15% by weight), and about 1 to about 90% by weight (preferably about 5 to about 80% by weight), respectively. The ratio of physiologically active compound combined an AII antagonist and a concomitant drug relative to the sum of four of an AII antagonist, a concomitant drug, a multivalent metal compound and a biodegradable polymer is usually about 1 to about 90% by weight, more preferably about 5 to about 80% by weight.

Also in the sustained-release preparation containing an AII antagonist and a concomitant drug, as shown in the aforementioned "sustained-release preparation containing an AII antagonist", the preparation may further contain (D') a component obtained by treating a hardly water-soluble multivalent metal compound with water and, as in the "sustained-release preparation containing an AII antagonist", the preparation may be prepared and used.

The contents of a physiologically active compound and a "component obtained by treating a hardly water-soluble multivalent metal compound with water" in the sustained-release preparation containing an AII antagonist and a concomitant drug differ depending on a kind of physiologically active compound (AII antagonist and concomitant drug), desired pharmacological effect, duration term of effect and the like, and when a physiologically active compound (AII antagonist and concomitant drug), a "component obtained by treating a hardly water-soluble multivalent metal compound with tater" and a biodegradable polymer are used as a starting raw material, the physiologically active compound, that is, the sum of an AII antagonist and a concomitant drug, relative to the sum of three is usually about 0.1 to about 90% by weight, more preferably about 1 to 80% by weight, on the other hand, the "hardly water-soluble multivalent metal compound to be treated with water" is usually about 0.5 to about 20% by weight, more preferably about 1 to about 15% by weight, particularly preferably about 2 to about 10% by weight.

The form of sustained-release preparation containing an AII antagonist and a concomitant drug is not particularly limited, and a parenteral preparation is preferable, and a percutaneous preparation, a preparation for implantation, a microcapsule injectable and the like are contemplated, and an injectable preparation using microcapsule which has a long sustained-release time and little burden on a patient is preferable.

A sustained-release preparation, for example, a microcapsule, containing an AII antagonist (compound having AII antagonism), a concomitant drug and a biodegradable polymer can be prepared as in the aforementioned "sustained-release preparation containing an AII antagonist". That is, the preparation can be prepared by the following methods of (I) to (III) depending on a nature of concomitant drug.

(I) In water drying method

(i) O/W method

A compound having an AII antagonism and a concomitant drug, or further a multivalent metal compound, a "component obtained by treating a hardly water-soluble multivalent metal compound with water" and, optionally, water are added to a solution of biodegradable polymer in organic solvent so that the weight ratio represented by the aforementioned content is attained, to prepare an organic solvent solution of biodegradable polymer containing a compound having an AII antagonism and a concomitant drug. Thereupon, the compound having an AII antagonism and a concomitant drug, and a multivalent metal compound may remain partially undissolved and may be dispersed in the solution of biodegradable polymer in organic solvent, respectively, it is preferable to disperse finely by a known method such as a homogenizer and an ultrasound.

Examples of the organic solvent include the same solvents as those exemplified in the aforementioned process for preparing the "sustained release preparation containing an AII antagonism".

An additive may be added to the aforementioned organic solvent solution. Examples of the additive include the same additives as those exemplified in the aforementioned process for preparing the "sustained-release preparation containing an AII antagonism".

The concentration in organic solvent solution of biodegradable polymer varies depending on a molecular weight of biodegradable polymer and a kind of organic solvent and, for example, when dichloromethane is used as an organic solvent, the concentration is generally selected from about 0.5 to about 70% by weight, more preferably about 1 to about 60% by weight, particularly preferably about 2 to about 50% by weight.

In addition, when ethanol or methanol is used as mixed organic solvent with dichloromethane, the ratio of dichloromethane in the mixed organic solvent is generally selected from about 10 to about 99% by volume, more preferably about 20 to about 98% by volume, particularly preferably about 30 to about 95% by volume.

Then, the resulting organic solvent solution of biodegradable polymer containing a compound having an AII antagonism and a concomitant drug, or further a multivalent metal compound, a "component obtained by treating a hardly water-soluble multivalent metal compound with water " and, optionally, water is added into an aqueous phase to form an O (oil phase) /W (aqueous phase) emulsion, and the solvent in the oil phase is evaporated to prepare a microcapsule. The volume of aqueous phase thereupon is generally selected from about 1-fold to about 10,000-fold, more preferably about 5-fold to about 5,000-fold, particularly preferably about 10-fold to about 2,000-fold of the volume of an oil phase.

An emulsifier may be added to the aforementioned outer aqueous phase. Examples of the emulsifier and the concentration upon use include the same emulsifiers and concentrations as those exemplified in the aforementioned process for preparing the "sustained-release preparation containing an AII antagonist".

An osmotic pressure controlling agent may be added to the aforementioned outer aqueous phase. Any osmotic pressure controlling agents may be used as far as they show an osmotic pressure when formulated into an aqueous solution.

Examples of the osmotic pressure controlling agent include the same osmotic pressure controlling agents as those exemplified in the aforementioned process for preparing the "sustained-release preparation containing an AII antagonist".

The osmotic pressure controlling agent is used at such the concentration that an osmotic pressure of an outer aqueous phase becomes about 1/50 to about 5-fold, preferably about 1/25 to about 3-fold of the osmotic pressure of physiological saline.

Examples of the method of removing solvent such as an organic solvent and water include the same methods as those shown in the aforementioned process for preparing the "sustained-release preparation containing an AII antagonist ".

The thus obtained microcapsule is separated by centrifugation or filtration, and the compound having an AII antagonism, concomitant drug, drug retaining substance, emulsifier and the like which are attached on the surface of microcapsule are repeatedly washed several times with distilled water, and then dispersed again into distilled water, followed by lyophilizing.

In order to prevent mutual coagulation of particles during production process, a coagulation preventing agent may be added. Examples of the coagulation preventing agent include the same coagulation preventing agents as those exemplified in aforementioned process for preparing of the "sustained-release preparation containing an AII antagonist".

Alternatively, as exemplified in the aforementioned process for preparing of the "sustained-release preparation containing an AII antagonist", after lyophilization, water and organic solvent in a microcapsule may be removed, if necessary, by warming within the conditions in which microcapsules are not fused mutually, under reduced pressure.

(ii) W/O/W method

Taking the solubilities of a compound having an AII antagonism and a concomitant drug in aqueous phase and oil phase into consideration, a W/O/W method can be used. That is, although plural compounds contained can be dissolved in the same phase to prepare a microcapsule, they may not be necessarily dissolved in the same phase, and may be dissolved in separate phases to prepare a microcapsule. A compound having an AII antagonism and a concomitant drug are dissolved in water and, if necessary, a drug retaining substance such as multivalent metal compound (e.g. zinc acetate), basic amino acid (e.g. arginine, histidine, lysine), gelatin, agar and polyvinyl alcohol is added thereto and dissolved to form an inner aqueous phase.

The concentrations of a compound having an AII antagonism and a concomitant drug in the inner aqueous phase are generally selected from about 0.1 to 80% by weight, more preferably about 1 to 70% by weight, particularly preferably about 2 to 60% by weight.

The same pH adjusting agents as those shown for the aforementioned "sustained-release preparation containing an AII antagonist" may be added to the inner aqueous phase in order to retain the stability and solubility of drug.

The thus obtained inner aqueous phase is added to a solution of biodegradable polymer in organic solvent (oil phase) optionally containing a multivalent metal, and this mixture is emulsified by a known method such as homogenizer and ultrasound to form a W/O emulsion.

Examples of the aforementioned organic solvent include the same organic solvents as those exemplified in the aforementioned process for preparing the "sustained-release preparation containing an AII antagonist".

The concentration of biodegradable polymer in the organic solvent solution varies depending on molecular weight of biodegradable polymer and a kind of organic solvent and, for example, when dichloromethane is used as an organic solvent, the concentration is generally selected from about 0.5 to about 70% by weight, more preferably about 1 to about 60% by weight, particularly preferably about 2 to about 50% by weight.

Then, the resulting W/O emulsion containing a compound having an AII antagonism, a concomitant drug and a biodegradable polymer is added to an aqueous phase (outer aqueous phase) to form a W (inner aqueous phase) /O (oil phase)/ W (outer aqueous phase) emulsion, and the solvent in the oil phase is evaporated to prepare a microcapsule. The volume of outer aqueous phase thereupon is generally selected from about 1-fold to about 10,000-fold, more preferably about 5-fold to about 5,000-fold, particularly preferably about 10-fold to about 2,000-fold of the volume of oil phase.

An emulsifier and an osmotic pressure controlling agent which may be added to the aforementioned outer aqueous phase, and a process thereafter are as described in the above (I) (i).

(II) Phase separating method

When a microcapsule is prepared by the present method, a coacervation agent is gradually added under stirring to the solution of biodegradable polymer in organic solvent containing a compound having an AII antagonism, a concomitant drug, or further a multivalent metal compound, a "component obtained by treating a hardly water-soluble multivalent metal compound with water" and, optionally water, described in water drying method of the above (I), to precipitate and solidify a microcapsule. The coacervation agent is selected from about 0.01 to 1,000-fold, preferably about 0.05 to 500-fold, particularly preferably about 0.1 to 200-fold of the volume of oil phase.

The coacervation agent is not particularly limited as far as it is a polymer, mineral oil or vegetable oil compound which is miscible with an organic solvent and does not dissolve three of a compound having an AII antagonism, a concomitant drug and a biodegradable polymer. Specifically, for example, a silicone oil, a sesame oil, a soybean oil, a corn oil, a cottonseed oil, a coconut oil, a linseed oil, a mineral oil, n-hexane and n-heptane are used. These may be used by mixing two or more of them.

The thus obtained microcapsule is separated, repeatedly washed with heptane to remove the coacervation agent other than a compound having an AII antagonism, a concomitant drug and a biodegradable polymer, and dried under reduced pressure. Alternatively, washing is performed and, thereafter, lyophilization and further warm drying are performed by the same method as that described in in-water drying method of the aforementioned (I) (i).

(III) Spray drying method

When a microcapsule is prepared by the present method, the solution of biodegradable polymer in an organic solvent containing a compound having an AII antagonism, a concomitant drug, or further a multivalent metal compound, a "component obtained by treating a hardly water-soluble multivalent metal compound with water" and, optionally water, described in in-water drying method of the aforementioned (I), is sprayed into a drying chamber of spray dryer using a nozzle, and the organic solvent in a finely-divided liquid droplet is volatilized in an extremely short time to prepare a microcapsule. As the nozzle, for example, a two-fluid nozzle type, a pressure nozzle type, a rotation disc type and the

like are exemplified. Thereafter, if necessary, after washing is performed by the same method as that described in in-water drying method of the aforementioned (I), and then lyophilization and further warm drying may be carried out.

As a dosage form other than the aforementioned microcapsule, the organic solvent and water in the organic solvent solution of biodegradable polymer containing a compound having an AII antagonism, a concomitant drug, or further a multivalent metal compound, a "component obtained by treating a hardly water-soluble multivalent metal compound with water" and, optionally water, described in the in water drying method of the process for preparing a microcapsule (I), are evaporated to dryness using, for example, a rotary evaporator while regulating the degree of vacuum and, thereafter, the residue may be ground with a jet mill to obtain a fine powder.

Further, after the ground fine powder is washed by the same method as that described in the in water drying method of the process for preparing a microcapsule (I), and lyophilization and further warm drying may be carried out.

In the microcapsule or fine powder obtained herein, release of drug can be controlled depending on a degradation rate of biodegradable polymer used or a kind and amount of multivalent metal compound added.

The sustained-release preparation containing an AII antagonist and a concomitant drug can be administered as it is, or by preparing various dosage forms using it as a raw material, as injectables or preparations for implantation into muscle, hypodermis, organs and the like, preparations for administering to mucosa onto cavitas nasi, rectum, uterus and the like, oral preparations (e.g. solid preparation such as capsule (e.g. hard capsule, soft capsule etc.), granule, powder etc., liquid preparation such as syrup, emulsion, suspension etc.) and the like. Alternatively, the preparation may be administered by a jet injector.

For example, in order to formulate the sustained-release preparation containing an AII antagonist and a concomitant drug into an injectable, a practically usable sustained-release injectable can be obtained by formulating the preparation together with a dispersing agent (e.g. surfactant such as Tween 80, HCO-60 etc, polysaccharides such as sodium hyaluronate, carboxymethylcellulose, sodium alginate etc.), a preservative (e.g. methylparaben, propylparaben etc.), an isotonic (e.g. sodium chloride, mannitol, sorbitol, glucose, proline etc,) and the like into an aqueous suspension, or dispersing with a vegetable oil such as sesame oil, corn oil and the like into an oily suspension.

When used as a suspension injectable, the particle diameter of sustained-release preparation containing an AII antagonist and a concomitant drug may be in such the range that its dispersibility and needle passability are satisfied and, for example, the average particle diameter is in a range of about 0.1 to 300 μm, preferably about 0.5 to 150 μm, more preferably about 1 to 100 μm.

Methods for preparing the sustained-release preparation containing an AII antagonist and a concomitant drug as a sterile preparation include, but are not limited to, a method in which the entire production process is sterile, a method of sterilizing with gamma-ray, a method of adding an antiseptic and the like.

A release term of AII antagonist contained in a sustained-release preparation and a release term of concomitant drug therein may be adjusted to the same term, or may be different and, since a release term can be appropriately regulated depending on symptom, it is not particularly limited.

Since the sustained-release preparation containing an AII antagonist and a concomitant drug is low toxic, it can be used as a safe medicine to a mammal (e.g. human, cow, pig, dog, cat, mouse, rat, rabbit, etc.).

The dose of sustained-release preparation containing an AII antagonist and a concomitant drug varies variously depending on kinds and contents of compound having an AII antagonism which is a basic active ingredient and concomitant drug, dosage form, duration times of release of compound having an AII antagonism and concomitant drug, subject disease, subject animal and the like, and may be effective amounts of the compound having an AII antagonism and concomitant drug. For example, when the sustained-release preparation is a one month-preparation, the single dose of compound having an AII antagonism can be appropriately selected from a range of about 0.01 mg to 200 mg/kg body weight, more preferably about 0.05 mg to 150 mg/kg body weight per adult. When the sustained-release preparation is a one month-preparation, the single dose of concomitant drug can be appropriately selected from a range of about 0.05 mg to 200 mg/kg body weight per adult.

The single dose of sustained-release preparation containing an AII antagonist and a concomitant drug can be appropriately selected from a range of preferably about 0.05 mg to 50 mg/kg body weight, more preferably about 0.1 mg to 30 mg/kg body weight per adult.

The number of administration can be appropriately selected depending on kinds and contents of compound having an AII antagonism and concomitant drug, dosage form, duration times of release of compound having an AII antagonism and concomitant drug, subject disease, subject animal and the like such as once per a few days, once per a few weeks, once per a month, once per a few months (e.g. three months, four months, six months etc.) and the like.

The sustained-release medicine of the present invention can be also advantageously used in a bed-ridden patient, a patient with dementia, guttural or esophageal disease, digestive tract disease, a patient with ingestion or swallowing disorder, or a patient at operation who is difficult or impossible to treat with medicine for internal application.

The sustained-release medicine of the present invention is used as an agent for preventing or treating, for example, angiotensin II-mediated various diseases or complexes of the various diseases of an animal, inter alia, a mammal (e.g. human, dog, rabbit, rat, mouse etc.).

Examples of subject disease of the compound having an angiotensin II antagonism as physiologically active compound include diseases which are developed or, development of which is promoted by constriction and proliferation of blood vessel or organ disorder developed via an angiotensin II receptor, by the presence of angiotensin II, or factors which are induced by the presence of angiotensin II.

Examples of such the angiotensin II-mediated various diseases or complexes of the various diseases include systemic diseases such as hypertension, blood pressure circadian rhythm abnormality, cardiac disease (hypercardia, acute heart failure, chronic heart failure including congestive, cardiomyopathy, angina, myocarditis, arrhythmia, tachycardia, cardiac infarction etc.), cerebrovascular disorder (silent cerebrovascular disorder, transient cerebral ischemic ceager, cerebral stroke, cerebrovascular dementia, hypertensive encephalopathy etc.), cerebral edema, cerebral circulation disorder, recurrence and sequela of cerebrovascular disorder (neurotic, mental symptomatic, subjective symptom, daily life activity disorder etc.), ischemic peripheral circulation disorder, cardiovascular ischemia, vein dysfunction, heart failure progression after cardiac infarction, diabetes, diabetic complex (diabetic retinopathy, diabetic nephropathy, diabetic neuropathy etc.), renal disease (nephritis, glomerulonephritis, glomerulosclerosis, renal insufficiency, thrombotic microangiopathy, dialysis complex, organ disorder including nephropathy due to radiation irradiation etc.), arteriosclerosis including atherosclerosis (aneurysm, coronary sclerosis, cerebral atherosclerosis, peripheral atherosclerosis etc.), vascular hypertrophy, vascular hypertrophy or occlusion and organ disorder after intervention (transdermal coronary plasty, stent dwelling, coronary endoscope, intravascular ultrasound, coronary injection thrombolytic therapy etc.), vascular reocclusion·restenosis after bypass operation, erythrocytosis•hypertension•organ disorder•vascular hypertrophy after transplantation, rejection reaction after transplantation, ocular disease (glaucoma, hyper-ocular tension etc.), thrombosis, multiple organ failure, endothelium dysfunction, hypertensive susurrus aurium, other circulatory diseases (deep venous thrombosis, obstructive peripheral circulation disorder, obstructive atherosclerosis, obstructive thrombotic vasculitis, ischemic cerebral circulation disorder, Raynaud's disease, Paget's disease etc.), metabolism·nutrient disorder (obesity, hyperlipemia, hypercholesterolemia, diabetes, glucose tolerance abnormality, hyperuricemia, hyperkalemia, hypernatremia etc.), neural denaturation disease (Alzheimer's disease, Parkinson's disease, amyotrophic lateral sclerosis, AIDS encephalopathy etc.), central nervous disorder (disorder such as cerebral hemorrhage and cerebral infarction and sequela·complex thereof, head trauma, spinal injury, cerebral edema, sensory dysfunction, sensory function abnormality, autonomic nerve dysfunction, autonomic nerve function abnormality, multiple sclerosis etc.), dementia, memory disorder, consciousness disorder, amnesia, anxiety symptom, tension symptom, disphoria mental status, mental disease (depression, epilepsy, alcohol dependency etc.), inflammatory disease (diabetic complex such as retinopathy, nephropathy, nervous disorder, great vessel disorder etc.; arthritis such as chronic rheumatoid arthritis, osteoarthritis, rheumatoid myelitis, periostosteitis; inflammation after operation·trauma; remission of enlargement; pharyngitis; urocystitis; pneumonia; atopic dermatitis; inflammatory bowl disease such as Crohn's disease, ulcerous colitis etc.; meningitis; inflammatory ocular disease; inflammatory pulmonary disease such as pneumonia, pneumosilicosis, pulmonary sarcoidosis, pulmonary tuberculosis etc.), allergy disease (allergic rhinitis, conjunctivitis, gastrointestinal allergy, pollinosis, anaphylaxis etc.), chronic obstructive pulmonary disease, pneumonitis, carinii pneumonia, collagenosis (e.g. systemic lupus erythematosus, scleroderma, multiple arteritis etc.), liver disease (hepatitis including chronic, cirrhosis etc.), portal vein pressure hyperfunction, gastrointestinal disease (gastritis, gastric ulcer, gastric cancer, disorder after gastric operation, dyspepsia, esophageal ulcer, pancreatitis, large intestine polyp, cholelithiasis, hemorrhoid disease etc.), blood·hematopoietic organ disease (polycythemia, vascular purpura, autoimmune hemolytic anemia, disseminated intravascular coagulation syndrome, multiple osteomyelitis etc.), bone disease (e.g. fracture, refracture, osteoporosis, osteomalacia, bone Behcet's disease, rigorous myelitis, chronic rheumatoid arthritis, gonarthrosis and destruction of joint tissue in similar diseases thereof), solid tumor, tumor (malignant melanoma, malignant lymphoma, gastrointestinal (e.g. stomach, intestine etc.) cancer etc.), cancer and cachexia accompanied therewith, metastasis of cancer, endocrine disease (Addison's disease, Cushing's syndrome, melanocytoma, primary aldosteronism etc.), Creutzfeldt-Jakob disease, urinary organ ·male sexual organ disease (cystitis, benign prostatic hyperplasia, prostate cancer, sexual infectious disease etc.), gynecological disease (climacteric disturbance, pregnant toxicosis, endometriosis, uterine myoma, ovary disease, mammary gland disease, sexual infectious disease etc.), pollakiuria, diseases due to environmental•occupational factors (radiation disorder, disorder due to ultraviolet•infrared•laser light, altitude sickness etc.), respiratory disease (cold syndrome, pneumonia, asthma, pulmonary hypertension, pulmonary thrombus·pulmonary embolus etc.), infectious disease (virus infectious disease such as cytomegalovirus, influenzavirus, herpesvirus etc., rickettsia infectious disease, bacteria infectious disease etc.), toxemia (sepsis, septic shock, endotoxin shock, Gram-negative sepsis, toxin shock syndrome etc.), otolaryngological disease (Ménière's syndrome, tinnitus, gustation disorder, vertigo, balance disorder, swallowing disorder etc.), skin disease (keloid, angioma, psoriasis etc.), dialysis hypotension, myasthenia gravis, and chronic fatigue syndrome.

Inter alia, the preparation is preferably used as an agent for preventing or treating circulatory disease or organ protecting agent. As used herein, circulatory disease includes central nervous disease derived from circulatory disorder. Among circulatory diseases, the sustained-release medicine of the present invention is preferably used for preventing or treating arteriosclerosis, hyperlipemia and the like, inter alia, it is preferable to use for preventing or treating arteriosclerosis. Further, the sustained-release medicine of the present invention may be used for a therapeutic method using for reducing cholesterol.

The sustained-release medicine of the present invention exerts remarkable effect on prevention or treatment of diseases which have, as a complex, hypertension such as diabetic, obese, hyperlipemic, essential or thrombotic hypertension, or climacteric disturbance accompanied with hypertension, or cancer. Inter alia, the sustained-release medicine of the present invention is preferably used for preventing or treating diabetes, hyperlipemia or arteriosclerosis which has hypertension as a complex.

The sustained-release medicine of the present invention is also useful for preventing or treating the subject diseases of remedy for hypertension, hypoglycemic drug, remedy for hyperlipemia, antithrombotic drug, remedy for climacteric disturbance and anticancer drug which is used together, in addition to angiotensin II-mediated various diseases or complexes with the various diseases which are direct subjects of an AII antagonist.

Examples of disease which is a subject of remedy for hypertension as a concomitant drug include hypertension, blood pressure circadian rhythm abnormality, hypertensive encephalopathy and hypertensive tinnitus.

Examples of disease which is a subject of hypoglycemic drug as a concomitant drug include diabetes and diabetic complex (diabetic retinopathy, diabetic nephropathy, diabetic nervous disorder etc.).

Examples of disease which is a subject of remedy for hyperlipemia as a concomitant drug include obesity, hyperlipemia and hypercholesterolemia.

Examples of disease which is a subject of antithrombotic drug as a concomitant drug include thrombosis, cardiac diseases (cardiac hypertrophy, acute heart failure and chronic heart failure including congestive heart failure, cardiomyopathy, cardiac angina, myocarditis, cardiac arrhythmia, tachycardia, cardiac infarct), cerebrovascular diseases (silent cerebrovascular disorder, transient cerebral ischemic stroke, cerebral apoplexy, cerebrovascular dementia, hypertensive encephalopathy etc.), cerebral circulation disorder, ischemic peripheral circulation disorder, myocardial ischemia, vein dysfunction, other circulatory diseases (deep venous thrombosis, obstructive peripheral circulation disorder, obstructive atherosclerosis, obstructive thrombotic vasculitis, ischemic cerebral circulation disorder, Raynaud's disease, Paget's disease etc.).

Examples of disease which is a subject of remedy for climacteric disturbance as a concomitant drug include menopause.

Examples of disease which is a subject of anticancer drug as a concomitant drug include breast cancer, uterus cancer, esophagus cancer, stomach cancer, large intestine cancer, lung cancer, prostate caner, ovary cancer, testis cancer, lever cancer, kidney cancer, pancreas cancer, leukemia, skin cancer, malignant melanoma and malignant lymphoma.

Such the subject diseases may not have the causal relation with the aforementioned angiotensin II-mediated various diseases such as hypertension and heart failure, and may be independently developed jointly.

In the sustained-release medicine of the present invention, sustained-release preparations of the aforementioned various forms may be orally or parenterally administered as they are, or by further formulating into, for example, a granule, a powder, a dust, a tablet, a capsule, a syrup, an emulsion, a suppository (e.g. rectal suppository, vaginal suppository etc.), an injectable (e.g. subcutaneous injectable, intravenous injectable, intramuscular injectable, intraperitoneal injectable etc.), a drop, an external use agent (e.g. transnasal preparation, transdermal preparation, ointment etc.), an emulsion, an elixir, a suspension, a solution or the like using those sustained- release preparations. These preparations can be formulated into a preparation according to a method known per se which are generally used in a pharmacy process. In the present specification, parenteral includes subcutaneous injection, intravenous injection, intramuscular injection, intraperitoneal injection and drip infusion. It is particularly preferable that the sustained-release medicine of the present invention is formulated into an injectable preparation.

An injectable dispensation, for example, a sterile injectable aqueous suspension or oily suspension can be prepared by a method known in the art using an appropriate dispersing agent or a wetting agent and a suspending agent. The sterile injectable dispensation may be a sterile injectable solution or suspension in a diluent or a solvent which is non-toxic and can be parenterally administered, such as an aqueous solution. Examples of usable vehicle or acceptable solvent include water, Ringer's solution and an isotonic sodium chloride solution. Further, a sterile fixed oil may be used as normal solvent or suspending solvent.

For this purpose, any fixed oils or fatty acids may be used, including natural or synthetic or semisynthetic aliphatic oils or fatty acids, and natural or synthetic or semisynthetic mono- or di- or triglycerides.

Further, additives such as a preservative, an isotonic, a solubilizer, a stabilizer and a soothing agent may be appropriately used.

Suppositories for rectal administration can be prepared by mixing the drug with a suitable non-stimulating additive, for example, an additive which is a solid at a normal temperature but is a liquid at a temperature of an intestinal tract, and melts in rectum and releases a drug such as cocoa butter and polyethylene glycols.

Examples of solid dosage form for oral administration include a powder, a granule, a tablet, a pill and a capsule as described above. In such the dosage form, active ingredient can be mixed with at least one additive, for example,

sucrose, lactose, cellulose sugar, mannitol, maltitol, dextran, starches, agar, alginates, chitins, chitosans, pectins, tragacanth gums, gums arabic, gelatins, collagens, caseins, albumin, synthetic or semisynthetic polymers or glycerides. Such the dosage form may contains further additives as usual, and examples thereof include an inert diluent, a lubricant such as magnesium stearate, a preservative such as prabens and sorbic acid, an antioxidant such as ascorbic acid, α-tocopherol and cysteine, an excipient, a disintegrating agent, a binder, a thickener, a buffer, a sweetner, a flavoring agent, a perfume and a coating agent. A tablet and pill may be prepared by further enteric coating. Example of liquid preparation for oral administration include pharmaceutically acceptable emulsion, syrup, elixir, suspension and solution and, they may contain an inert diluent which is usually used in the art, for example, water.

The dose of sustained-release medicine of the present invention can be appropriately selected depending on administration subject, age and weight of administration subject, symptom, administration time, administration method, dosage form, combination of drug and the like, using minimum recommended clinical doses of individual drugs as a standard.

A dose to a particular patient is determined depending on age, weight, general physical state, sex, diet, administration time, administration method, excretion rate and combination of drug, or extent of disease of a patient being treated at that time, and in view of other factors.

Typically, when an AII antagonist is combined with at least one kind of a remedy for hypertension, a hypoglycemic drug, a remedy for hyperlipemia, an antithrombotic drug, a remedy for climacteric disturbance and an anticancer drug, the individual daily dose is in a range of about 1/50 of minimum recommended clinical dose or larger and a maximum recommended level or lower regarding the actual aspect when they are administered alone (preferably a minimum recommended clinical dose or smaller, more preferably 1/2 of minimum recommended clinical dose or smaller).

The dose range can be regulated based on a unit necessary for dividing daily dose and, as described above, the dose is determined depending on nature and extent of disease, age, weight and, general physical state and sex of patient, diet, administration time, administration method, excretion rate and combination of drug, and in view of other factors.

In the preventing or treating agent of the present invention, the unit dose is administered once or twice in one day to a few months.

The aforementioned compound having an AII antagonism has high safety and, even when the blood concentration is increased immediately after administration, the blood pressure is not lowered too much. The sustained-release medicine of the present invention comprising a combination with AII antagonist can be used as an agent for treating the aforementioned various diseases and the like, and since a constant blood level can be maintained day or night, the dose and administration time can be reduced as compared with administration of an oral agent and, further, since the variation of blood concentration of drug is small and change in symptom due to interruption of dosing does not occur, the therapeutic effect is expected to be clearer.

Regarding the safety, a risk such as excessive pressure drop is small under normal use circumstance for the aforementioned reasons as compared with internal use and, even when excessive pressure drop occurs due to development of situation accompanied with great loss of body fluid such as traffic accident, since instantaneous pressure rise is possible by intravenous administration of not only angiotensin II but also a drug which is usually used at emergent medical scene (catecholamine preparation etc.) and further, persistent pressure rise is also possible by oral administration of a remedy for hypotension, not only acute response at emergency but also long term response are possible.

The sustained-release medicine of the present invention contains a compound having an angiotensin II antagonism and, by suppressing the activity of angiotensin II for a long time, disorders or abnormalities of living body function and physiological action which are causes for various diseases accompanied with adult disease or aging can be improved, or enhancement thereof can be suppressed, and primary and secondary prevention or development of diseases or pathologies derived therefrom can be suppressed. Examples of such the disorder or abnormality of living body function and physiological action include disorder or abnormality of cerebral circulation·renal circulation automatic regulating ability, circulatory disorder (peripheral, cerebral, microcirculation etc.), disorder of blood-brain barrier, reduction in insulin sensitivity, sodium salt sensitivity, coagulation·fibrinolysis abnormality, abnormality of nature of blood·hemocyte components (enhancement of platelet coagulation ability, abnormality of erythrocyte deforming ability, enhancement of leukocyte adhering ability, increase in blood viscosity etc.), enhancement of production and action of growth factors and cytokines (PDGF, VEGF, FGF, interleukin, TNF-α, MCP-1 etc.), enhancement of production and infiltration of inflammatory cells, enhancement of production of free radical, promotion of fat sedimentation, endothelial function disorder, endothelium, cell and organ disorder, edema, change in morphology of cells such as smooth muscle (morphology change to proliferating type), enhancement of production and function of vessel acting substance and thrombus inducer (endothelin, thromboxane $A_2$ etc.), abnormal constriction of vessel, glucose tolerance abnormality, metabolism abnormality (serum lipid abnormality, blood sugar abnormality etc.), abnormal proliferation of cells, and vascularization (including abnormal vascular formation at formation of abnormal capillary network of atherosclerosis lesion tunica externa). Inter alia, the medicine can be advantageously used as a primary or secondary preventing or

treating agent for organ disorders accompanied with various diseases (e.g. cerebrovascular disorder and organ disorder accompanied therewith, organ disorder accompanied with circulatory disease, organ disorder accompanied with diabetes, organ disorder after intervention etc.

Further, the medicine can be advantageously used as an agent for preventing or treating hyperfunction of portal pressure. It is known that rupture of esophageal varix develops frequently at night (Hepatology 1994; 19:595-601) and, since the present agent can maintain a constant blood concentration whether day or night, a dose and administration times can be reduced as compared with administration by an oral agent and, since variation in blood concentration of drug is small, stable reduction in portal pressure can be expected. The aforementioned characteristic of the present agent shows usefulness as a medicine for preventing rupture of esophageal or gastric varix. In addition, since change in symptom due to interruption of dosing does not occur, the therapeutic effect is expected to be clearer. Further, a compound having an angiotensin II antagonism as physiologically active compound (in particular, Candesartan cilexetil, Candesartan etc.) is expected to be effective for promoting production of HGF (Hepatocyte Growth Factor), and contribution to hepatic regeneration and hepatic function recovery can be expected.

In addition, by maintaining blood concentration of compound having an angiotensin II antagonism as physiologically active compound (in particular, Candesartan cilexetil, Candesartan etc.) at constant day or night, the effect of preventing or treating cerebrovascular disorder such as cerebral infarction can be expected to be clearer.

As a method for treating patient, it can be also contemplated that an oral agent of angiotensin II antagonist is administered for a certain term, and responsiveness of the patient is confirmed and, thereafter, the sustained-release preparation of the present invention is administered. The angiotensin II antagonist to be administered orally and angiotensin II antagonist contained in the sustained-release preparation may be the same or different. Alternatively, a depressor other than angiotensin II antagonist (calcium antagonist, diuretic, beta-blocker etc.) is orally administered in advance, and responsiveness of the patient is confirmed and, thereafter, the sustained-release preparation of the present invention may be administered. Alternatively, the sustained-release preparation of the present invention and a diuretic depressor (oral agent) which is usually used together with angiotensin II antagonist, may be used together.

Alternatively, the sustained-release preparation may be used together with other medicine components (provided that physiologically active compounds contained in the present sustained-release preparation are excluded) containing other hypolipidemic or cholesterol decreasing drug, HMG-Co A reductase (3-hydroxy-3-methylglutaryl coenzyme A reductase) inhibitor, insulin sensitizer, bone disease treating drug, myocardial protecting drug, coronary disease treating drug, other hypertension treating drug, chronic heart failure treating drug, diabetes treating drug, liver disease treating drug, gastric·duodenal ulcer treating drug, biliary tract disease treating drug, hypothyroism treating drug, nephrosis syndrome treating drug, chronic renal failure treating drug, gynecologic disease treating drug, urinary organ·male sexual organ disease treating drug and infectious disease treating drug. In this case, these compounds may be administered as an oral preparation or, if necessary, may be formulated into a rectal preparation and may be administered as a form of suppository. For this case, examples of components which can be combined include fibrates [e.g. clofibrate, benzafibrate, gemfibrozil etc.], nicotinic acid, a derivative and an analog thereof [e.g. acipimox and probucol], bile acid binding resin [e.g. cholestyramin, colestipol etc.], a compound which suppresses absorption of cholesterol [e.g. sitosterol and neomycin etc.], a squaleneepoxidase inhibitor [e.g. NB-598 and analogs etc.].

Further other possible combination components include oxidosqualene-lanosterolcyclase, for example, decalin derivatives, azadecalin derivatives and indan derivatives.

In addition, a combination with following various treating drugs (provided that physiologically active compounds contained in the sustained-release preparation of the present invention are excluded) is also possible.

Hypertension treating drug: diuretic [e.g. furosemide (lasix), bumetanide (lunetoron), azosemide (diart)], depressor [e.g. ACE inhibitor (enalapril maleate (renivace) etc.) Ca antagonist (manidipine, amlodipine etc.), α or β receptor blocker etc.] etc.,

Chronic heart failure treating drug: cardiotonic drug [e.g. cardiac glycoside (digoxin etc.), β receptor stimulating drug (catecholamine preparation such as denopamine and dobutamine) and PDE inhibitor etc.], diuretic [e.g. furosemide (lasix), spironolactone (aldactone) etc.], ACE inhibitor [e.g. enalapril maleate (renivace) etc.], Ca agonist [e.g. amlodipine etc.] and β receptor blocker,

Antiarrhythmic drug: disopyramide, lidocaine, quinidine sulfate, flecainide acetate, mexiletine hydrochloride, amiodarone hydrochloride, and β-blocker, Ca antagonist etc.,

Born disease treating drug: calcium preparation (e.g. calcium carbonate etc.), calcitonin preparation, active vitamin $D_3$ preparation (e.g. alfacalcidol (alfarol etc.), calcitriol (rocaltrol) etc.), sex hormones (e.g. estrogen, estradiol etc.), hormone preparations [e.g. conjugated estrogen (premarin) etc.], ipriflavone preparation (osten etc.), vitamin $K_2$, vitamin $K_2$ preparation [e.g. menatetrenone (glakay) etc.] bisphosphonic acid preparation (etidronate etc.), prostaglandin E2, fluorine compound (e.g. sodium fluoride etc.), bone morphogenetic protein (BMP), fibroblast growth factor (FGF), platelet derived growth factor (PDGF), transforming growth factor (TGF-β), insulin-like growth factor-1 and -2 (IGF-1, -2), parathyroid hormone (PTH), compounds described in EP-A1-376197, EP-A1-460488 and EP-A1-719782 (e.g. (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide etc.);

Diabetes treating drug: actos, rosiglitazone, kinedak, penfill, humulin, euglucon, glimicron, daonil, novolin; monotard, insulins, glucobay, dimelin, rastinon, bacilcon, deamelin S, iszilins etc.;

Hepatic disease treating drug: glycyrrhizin preparation [e.g. potent minophagen etc.], hepatic hydrolysate, SH compound [e.g. glutathione etc.], special amino acid preparation [e.g. aminoleban etc.], phospholipid [e.g. polyene phosphatidylcholine etc.], vitamins [e.g. vitamins B1, B2, B6, B12, C etc.], adrenocortical hormone [e.g. dexamethasone, betamethasone etc.], interferon [e.g. interferon $\alpha$, $\beta$ etc.], hepatic encephalopathy treating drug [e.g. lactulose etc.], hemostat used at rupture of esophageal or gastric varix [e.g. vasopressin, somatostatin etc.] etc;

Gastric·duodenal ulcer treating drug: antacid [e.g. histamine H2 antagonist (cimetidine etc.), proton pump inhibitor (lansoprazole etc.) etc];

Biliary tract disease treating drug: choleretic [e.g. dehydrocholic acid etc.], cholekinetic [e.g. magnesium sulfate etc.] etc.;

Hypothyroidism treating drug: dry thyroid gland (thyreoid), levothyroxine sodium (thyradin S), liothyronine sodium (thyronine, thyromin) etc.;

Nephrosis syndrome treating drug: Usually, in steroid therapy adopted as first selection, predonisolone (predonine), predonisolone sodium succinate (predonine), methylpredonisolone sodium succinate (solu-medrol), betamethasone (rinderon) and the like are used. In addition, in anti-coagulation therapy, anti-platelet drug and anti-coagulant drug such as dipyridamole (persantin), dilazep hydrochloride (comelian), ticlopidine, clopidogrel, FXa inhibitor and the like are used;

HMG-Co A reductase inhibitor: cerivastatin, atrovastatin, pravastatin, simvastatin, itavastatin, lovastatin, fluvastatin, (+)-3R,5S-7-[4-(4-fluorophenyl)-6-isopropyl-2-(N-methyl-N-methanesulfonylamino)pyrimidin-5-yl]-3,5-dihydroxy-6(E)-heptenoic acid;

Chronic renal failure treating drug: When administered by combining with a diuretic [e.g. furosemide (lasix), bumetanide (lunetoron), azosemide (diart)], a depressor (e.g. ACE inhibitor (enalapril maleate (renivace)) and Ca antagonist (manidipine), an $\alpha$ receptor blocker or the like, it can be preferably used with oral administration.

Thrombus formation preventing or treating drug: blood coagulation inhibitor [e.g. heparin sodium, heparin calcium, warfarin calcium (warfarin), blood coagulation factor Xa inhibitor as well as a drug having a function of correcting balance of coagulation fibrinolytic system], thrombolytic drug [e.g. tPA, urokinase], anti-platelet drug [e.g. aspirin, sulphinpyrazolo (anturan), dipyridamole (persantin), ticlopidine (panaldine), cilostazol (Pletaal), GPIIb/IIIa antagonist (Reo Pro)] etc.,

Coronary vasodilator: nifedipine, diltiazem, nicorandil, nitrous acid agent etc.,

Cardiac muscle protecting drug: heart ATP-K opener, Na-H exchange inhibitor, endothelin antagonist, urotensin antagonist etc.,

Anti-inflammatory drug: aspirin, acetoaminophen, non-steroidal anti-inflammatory drug [e.g. indomethacin etc.], steroid agent [e.g. dexamethasone etc.] etc.,

Anti-allergic drug: anti-histamine drug [e.g. chlorpheniramine maleate etc.], stimulation therapeutic agent [e. g. bucillamine etc.], other azelastine hydrochloride, seratrodast, tranilast, oxatomide, potent neo-minophagen c, tranexamic acid, ketotifen fumarate etc.,

Anti-tumor drug: alkylating agent, metabolism antagonist, antitumor antibiotic preparation, antitumor plant component preparation and other antitumor drug etc.,

Central nervous agonist: antianxiety, sedative hypnotic, anesthetic, antispasmodic, autonomic nerve drug, anti-Parkinson drug and other mental nervous drug etc.,

Gynecologic disease treating drug: e.g. menopausal disorder treating drug (conjugated estrogen, estradiol, testosterone enanthate•estradiol valerate etc.), breast cancer treating drug (tamoxifen citrate etc.), endometriosis•uterine myoma treating drug (leuprorelin acetate, Danazol etc.)] etc.,

Urinary organ·male sexual organ disease treating drug: [e.g. benign prostatic hyperplasia treating drug (tamuslosin hydrochloride, prazosin hydrochloride, chlormadinone acetate), prostate cancer (leuprorelin acetate, goserelin acetate, chlormadinone acetate, etc.)] etc.,

Infectious disease treating drug: [e.g. antibiotic preparation (cefotiam hydrochloride, cefozopran hydrochloride, ampicillin etc.), chemotherapeutic agent (sulfa agent, synthetic antibacterial agent, anti-virus agent etc.), biological preparation (vaccines, blood preparations such as immunoglobulin) etc.] etc.,

Other anti-obesity drug (mazindol etc.), anti-rheumatoid drug.

Further, therapy by living body-derived various factors or gene introduction thereof (e.g. ischemic disease treatment by vascularization promoting factors such as HGF, VEGF etc. or their gene introduction) etc.

When these drugs and the sustained release preparation of the present invention are used in combination, although respective drugs may be incorporated into one sustained-release preparation [including the case where the drugs are incorporated into (A) a sustained-release preparation containing an AII antagonist and the case where the drugs are incorporated into (B) a sustained-release preparation containing one or more drugs selected from a remedy

for hypertension, a hypoglycemic drug, a remedy for hyperlipemia, an antithrombotic drug, a remedy for climacteric disturbance and an anticancer drug], the aforementioned drugs may be formulated into a preparation by mixing with pharmacologically acceptable carrier, excipient, binder and diluent, and the preparation may be administered separately or at the same time with the sustained-release preparation of the present invention. When drugs are separately formulated into preparations, although separately formulated preparations may be administered by mixing with a diluent and the like upon use, separately formulated individual preparations may be administered to the same subject at the same time, or separately at different times.

As another aspect of the present invention, a sustained-release medicine comprising a combination of two or three drugs selected from an AII antagonist, a remedy for hypertension, a hypoglycemic drug and a remedy for hyperlipemia is provided.

Examples of the AII antagonist, remedy for hypertension, hypoglycemic drug and remedy for hyperlipemia include those as described above.

An AII antagonist, a remedy for hypertension, a hypoglycemic drug and a remedy for hyperlypemia can be orally or parenterally administered separately or at the same time as a pharmaceutical composition by formulating into a sustained-release preparation. When drugs are separately formulated into preparations, although separately formulated preparations may be administered by mixing with a diluent and the like upon use, separately formulated individual sustained-release preparations may be administered to the same subject at the same time or separately at different times. The medicine of the present invention includes a kit product for administering separately formulated preparations by mixing with a diluent and the like upon use (e.g. an injectable kit containing an ample containing individual powdery drugs and a diluent and the like for mixing to dissolve two or more drugs upon use), and a kit product for administering separately formulated individual sustained-release preparations to the same subject at the same time, or separately at different times (e.g. a tablet kit for administering two or more tablets at the same time, or separately at different times in which tablets containing individual drugs are placed into the same bag or separate bags and, if necessary, a column for describing drug administration times is provided).

Such the sustained-release medicine can be formulated into a preparation and implemented like the aforementioned "sustained-release preparation containing an AII antagonist", and "sustained-release preparation containing one or more drugs selected from a remedy for hypertension, a hypoglycemic drug, a remedy for hyperlipemia, an antithrombotic drug, a remedy for climacteric disturbance and an anticancer drug".

Meanings of abbreviations used in the present specification are as follows:

| Abbreviation | Name |
|---|---|
| N(4$H_2$-furoyl)Gly | N-tetrahydrofuroylglycine residue |
| NAc | N-acetyl group |
| D2Nal | D-3-(2-naphthyl)alanine residue |
| D4ClPhe | D-3-(4-chloro)phenylalanine residue |
| D3Pal | D-3-(3-pyridyl)alanine residue |
| NMeTyr | N-methyltyrosine |
| Aph(Atz) | N-[5'-(3'-amino-1'H-1',2',4'-triazolyl)]phenylalanine residue |
| NMeAph(Atz) | N-methyl-[5'-(3'-amino-1'H-1',2',4'-triazolyl)]phenylalanine residue |
| DLys(Nic) | D-(e-N-nicotinoyl)lysine residue |
| Dcit | D-citrulline residue |
| DLys(AzaglyNic) | D-(azaglycylnicotinoyl)lysine residue |
| DLys(AzaglyFur) : | D-(azaglycylfuranyl)lysine residue |
| DhArg($Et_2$) | D-(N,N'-diethyl)homoarginine residue |
| DAph(Atz) | D-N-[5'-(3'-amino-1'H-1',2',4'-triazolyl)]phenylalanine residue |
| DhCi | D-homocitrulline residue |
| Lys(Nisp) | (e-N-isopropyl)lysine residue |
| hArg($Et_2$) | (N,N'-diethyl)homoarginine residue |
| D2Nal | D-3-(2-naphthyl)alanine residue |
| DSer(tBu) | O-tert-butyl-D-serine |
| Dhis(ImBzl) | $N^{im}$-benzyl-D-hystidine |

Besides, when amino acids are expressed by abbreviations, expressions are based on abbreviations by IUPAC-IUB Commission on Biochemical Nomenclature (European Journal of Biochemistry, vol. 138, pp. 9-37 (1984)) or conventional abbreviations in the art and, when there can be an optical isomer regarding amino acids, L-isomer is

shown unless otherwise indicated.

The present invention will be explained in more detail below by way of Reference Examples, Examples and Experimental Examples, but the present invention is not limited by them.

[Examples]

Reference Example 1

0.25 g of 2-ethoxy-1-[[2'-(4,5-dihydro-5-oxo-1,2,4-oxadiazol-3-yl)biphenyl-4-yl]methyl]benzimidazole-7-carboxylic acid (hereinafter, abbreviated as Compound A) and 2.25 g of lactic acid-glycolic acid copolymer (lactic acid/glycolic acid = 75/25 (mol%), weight average molecular weight 10,700, number average molecular weight 6,100, number average molecular weight according to terminal group quantitation 3,770, manufactured by Wako Pure Chemical Industries, Ltd. ) were dissolved in a mixed solvent of 3.5 ml of dichloromethane and 1.5ml of methanol. The solution was poured into 500 ml of 0.1% (w/w) aqueous polyvinyl alcohol solution regulated at 18°C in advance, and made into O/W emulsion at 7,000 rpm using a turbine-type homomixer. This O/W emulsion was stirred at room temperature for 3 hours to volatilize dichloromethane and methanol, and the oil phase was solidified, followed by collection at 2,000 rpm using a centrifuge. This was dispersed again in distilled water, and subjected to further centrifugation to wash free drug and the like. Collected microcapsules were dispersed again by adding small amount of distilled water, and lyophilized to give a powder. The recovery rate was 69%, encapsulation rate of Compound A into microcapsule was 92%, and the content of Compound A in microcapsule was 9.2%.

Reference Example 2

A solution obtained by dissolving 0.25 g of disodium salt of Compound A in 0.4 ml of distilled water was mixed with a solution obtained by dissolving 2.25 g of lactic acid-glycolic acid copolymer (same in Reference Example 1) in 4 ml of dichloromethane, and emulsified with homogenizer to form a W/O emulsion. Then, this W/O emulsion was poured into 500 ml of 0.1% (w/w) aqueous polyvinyl alcohol solution regulated at 18°C in advance, and made into W/O/W emulsion at 7,000 rpm using a turbine type homomixer. This W/O/W emulsion was stirred at room temperature for 3 hours to volatilize dichloromethane, and the oil phase was solidified, followed by collection at 2,000 rpm using a centrifuge. This was dispersed again in distilled water, and subjected to further centrifugation to wash free drug and the like. Collected microcapsules were dispersed again by adding small amount of distilled water, and lyophilized to give a powder. The recovery rate was 50%, encapsulation rate of Compound A into a microcapsule was 37%, and the content of Compound A in microcapsule was 3.7%.

Reference Example 3

0.4 g of Compound A and 1.6 g of lactic acid polymer ethyl ester compound (biodegradable polymer in which the terminal carboxy group of lactic acid polymer is ethyl esterfied, weight average molecular weight 10,200, number average molecular weight 5,680, manufactured by Wako Pure Chemical Industries, Ltd.) were dissolved in mixed solvent of 3.5 ml of dichloromethane and 2.5 ml of methanol. The solution was poured into 800 ml of 0.1% (w/w) polyvinyl alcohol aqueous solution containing 5% mannitol, which had been regulated at 18°C in advance, and made into an O/W emulsion at 7,000 rpm using a turbine-type homomixer. This O/W emulsion was stirred at room temperature for 3 hours to volatilize dichloromethane and methanol, and the oil phase was solidified, followed by collection at 2,000 rpm using a centrifuge. This was dispersed again in distilled water, and subjected to further centrifugation to wash free drug and the like. Collected microcapsules were dispersed again by adding small amount of distilled water, and lyophilized to give a powder. The recovery rate was 83%, encapsulation rate of Compound A in microcapsule was 86%, and the content of Compound A in microcapsule was 17.1%.

Reference Example 4

0.6 g of 2-ethoxy-1-[[2'-(1H-tetrazol-5-yl)biphenyl-4-yl]methyl]benzimidazole-7-carboxylic acid (hereinafter, abbreviated as Compound B) and 0.09 g of zinc oxide having a particle diameter of 0.02 µm were added to a solution obtained by dissolving 2.4 g of lactic acid-glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries Ltd.) in 4.5 ml of dichloromethane and 1ml of ethanol, the mixture was shaken and stirred at room temperature for 12 hours to obtain a slightly cloudy solution. This solution was poured into 400 ml of 0.1% by weight polyvinyl alcohol aqueous solution regulated at 15°C in advance, and made into an O/W emulsion at 7,000 rpm using a turbine-type homomixer. This O/W emulsion was

stirred at room temperature for 3 hours to volatilize dichloromethane and ethanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol were dissolved, and lyophilized to give a powder. The encapsulation rate of Compound B in microcapsule was 97%, and the content of Compound B in microcapsule was 18.8%.

Reference Example 5

According to the same manner as in Reference Example 1 except that the amount of zinc oxide was changed to 0.057 g, a microcapsule was obtained. The encapsulation rate of Compound B in microcapsule was 97%, and the content of Compound B in microcapsule was 19.0%.

Reference Example 6

According to the same manner as in Example 1 except that the amount of Compound B, amount of zinc oxide and amount of lactic acid-glycolic acid copolymer were changed to 0.9 g, 2.1 g and 0.12 g, respectively, a microcapsule was obtained. The encapsulation rate of Compound B in microcapsule was 96%, and the content of Compound B in microcapsule was 27.8%.

Reference Example 7

According to the same manner as in Reference Example 3 except that the amount of zinc oxide was changed to 0.18 g, a microcapsule was obtained. The encapsulation rate of Compound B in microcapsule was 92%, and the content of Compound B in microcapsule was 26.2%.

Reference Example 8

1.8 g of Compound B and 0.3 g of zinc oxide having a particle diameter of 0.02 μm were added to a solution obtained by dissolving 4.2 g of lactic acid-glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries, Ltd.) in 9 ml of dichloromethane and 1.5 ml of ethanol. The mixture was shaken and stirred at room temperature for 12 hours to obtain a slightly cloudy solution. This solution was poured into 800 ml of 0.1% by weight polyvinyl alcohol aqueous solution regulated at 15°C in advance, and made into an O/W emulsion at 7,000 rpm using a turbine-type homomixer. This O/W emulsion was stirred at room temperature for 3 hours to volatilize dichloromethane and ethanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound B in microcapsule was 94%, and the content of Compound B in microcapsule was 26.8%.

Reference Example 9

0.3 g of Compound A and 0.05 g of zinc oxide having a particle diameter of 0.02 μm were added to a solution obtained by dissolving lactic acid-glycolic acid copolymer (lactic acid/glycolic acid 75/25 (mol%), weight average molecular weight 14,000, number average molecular weight 4,200, number average molecular weight according to terminal quantitation 4,090, manufactured by Wako Pure Chemical Industries, Ltd.) in 1.5 ml of dichloromethane and 1 ml of methanol, and the mixture was shaken and stirred at room temperature for 12 hours to obtain a slightly cloudy solution. This solution was poured into 300 ml of 0.1% by weight polyvinyl alcohol aqueous solution regulated at 15°C in advance, and made into an O/W emulsion at 6,500 rpm using a turbine-type homomixer. This O/W emulsion was stirred at room temperature for 3 hours to volatilize dichloromethane and methanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound A in microcapsule was 91%, and the content of Compound A in microcapsule was 25.9%.

Reference Example 10

1 g of Compound B and 0.18 g of zinc oxide having a particle diameter of 0.02 μm were added to a solution

obtained by dissolving 1.8 g of lactic acid-glycolic acid copolymer (lactic acid/glycolic acid 75/25 (mol%), weight average molecular weight 14,000, number average molecular weight 4,200, number average molecular weight according to terminal group quantitaion 4,090, manufactured by Wako Pure Chemical Industries, Ltd.) in 5ml of dichloromethane, and the mixture was emulsified by mixing with a small homogenizer for 60 seconds to obtain a cloudy dispersion. This dispersion was poured into 400 ml of 0.1% by weight polyvinyl alcohol aqueous solution regulated at 15°C in advance, and made into an O/W emulsion at 8,000 rpm using a turbine-type homomixer. This O/W emulsion was stirred at room temperature for 3 hours to volatilize dichloromethane and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound B in microcapsule was 96%, and the content of Compound B in microcapsule was 32.0%.

Reference Example 11

According to the same manner as in Example 7 except that a slightly cloudy solution obtained by adding 0.8 ml of ethanol to dichloromethane and shaking and stirring at room temperature for 12 hours was used, a microcapsule was obtained. The encapsulation rate of Compound B in microcapsule was 95%, and the content of Compound B in microcapsule was 32.0%.

Reference Example 12

0.9 g of 1-(cyclohexyloxycarbonyloxy)ethyl 2-ethoxy-1-[[2'-(1H-tetrazol-5-yl)biphenyl-4-yl]methyl]benzimidazole-7-carboxylate (hereinafter, abbreviated as Compound C) and 2.1 g of lactic acid-glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries, Ltd.) were dissolved in a mixed solvent of 4.5 ml of dichloromethane and 0.7 ml of ethanol. 0.15 g of zinc oxide having a particle diameter of 0.02 μm was added to this solution, and the mixture was shaken and stirred at room temperature for 12 hours to obtain a slightly cloudy solution. This solution was poured into 400 ml of 0.1% by weight polyvinyl alcohol aqueous solution regulated at 15°C in advance, and made into an O/W emulsion at 7,500 rpm using a turbine-type homomixer. This O/W emulsion was stirred at room temperature for 3 hours to volatilize dichloromethane and ethanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound C in microcapsule was 96%, and the content of Compound C in microcapsule was 27.4%.

Reference Example 13

According to the same manner as in Reference Example 12 except that zinc oxide was not added, a microcapsule was prepared. The encapsulation rate of Compound C in microcapsule was 98%, and the content of Compound C in microcapsule was 30.0%.

Reference Example 14

1.2 g of Compound C and 1.8 g of lactic acid-glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries, Ltd.) were dissolved in 5 ml of dichloromethane. 0.18 g of zinc oxide having a particle diameter of 0.02 μm was added to this solution, and the mixture was shaken and stirred at room temperature for 1 hour to obtain a slightly cloudy solution. This solution was poured into 400 ml of 0.1% by weight polyvinyl alcohol aqueous solution regulated at 15°C in advance, and made into an O/W emulsion at 8,000 rpm using a turbine-type homomixer. This O/W emulsion was stirred at room temperature for 3 hours to volatilize dichloromethane, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound C in microcapsule was 95%, and the content of Compound C in microcapsule was 35.9%.

Reference Example 15

According to the same manner as in Example 4 except that zinc oxide was not added, a microcapsule was prepared. The encapsulation rate of Compound B in microcapsule was 99%, and the content of Compound B in microcapsule was 19.8%.

Reference Example 16

According to the same manner as in Reference Example 9 except that zinc oxide was not added, a microcapsule was prepared. The encapsulation rate of Compound A in microcapsule was 95%, and the content of Compound A in microcapsule was 28.4%.

Reference Example 17

2 g of 2-ethoxy-1-[[2'-(1H-tetrazol-5-yl)biphenyl-4-yl]methyl]benzimidazole-7-carboxylic acid (Compound B) and 0.36 g of zinc oxide (TYPE V, manufactured by Wako Pure Chemical Industries, Ltd.) were added to a solution obtained by dissolving 3.6 g of lactic acid/glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries) in 11 ml of dichloromethane and 0.4 ml of ethanol, and the mixture was shaken and stirred at room temperature for 14 hours to obtain a cloudy solution. This solution was poured into 800 ml of 0.1% by weight polyvinyl alcohol aqueous solution regulated at 15°C in advance, and made into an O/W emulsion at 8,500 rpm using a turbine-type homomixer. This O/W emulsion was stirred at room temperature for 3 hours to volatilize dichloromethane and ethanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound B in microcapsule was 98%, and the content of Compound B in microcapsule was 33.0%.

Reference Example 18

According to the same manner as in Reference Example 17 except that 0.4 ml of distilled water was added, and shaking and stirring for 14 hours was changed to dispersing (emulsifying) mixing together with a solid (Compound B and zinc oxide) at the same rotation number for 1 minute by a homogenizer, a microcapsule was obtained. The encapsulation rate of Compound B in microcapsule was 97%, and the content of Compound B in microcapsule was 32.6%.

Reference Example 19

According to the same manner as in Reference Example 17 except that the amount of distilled water to be added was changed to 0.08 ml, a microcapsule was obtained. The encapsulation rate of Compound B in microcapsule was 97%, and the content of Compound B in microcapsule was 32.5%.

Reference Example 20

4 g of Compound B and 0.72 g of zinc oxide (TYPE V, manufactured by Wako Pure Chemical Industries, Ltd.) were added to a solution obtained by dissolving 7.2 g of lactic acid-glycolic acid copolymer (lactic acid/glycolic acid 75/25 (mol%), weight average molecular weight 10,600) in 22 ml of dichloromethane and 0.8 ml of ethanol. 0.16 ml of distilled water was added thereto and, thereafter, dispersing (emulsifying) mixing by a homogenizer was carried out immediately under the same conditions as in Reference Example 18 to obtain a cloudy solution. This was cast into a circle having a radius of about 5 cm on a plate, and dried at room temperature for 15 hours under reduced pressure to obtain a dry material. This dry material was roughly ground and sieved on a sieve having a pore diameter of 250 μm. 5 g of the resulting dry material and 0.4 g of mannitol were mixed, and the mixture was ground with a gas at an air pressure of 2 $kg/cm^2$ using jet mill apparatus (A-OJET, manufactured by SEISHIN ENTERPRISE CO.,LTD.) to give a fine particle having an average particle diameter of 21 μm. The content of Compound B in fine particle was 31.0%.

Reference Example 21

A cloudy solution obtained by dispersing (emulsifying) mixing according to the same formulation·procedure

as in Reference Example 20 was spray-dried (Mobile Minor, manufactured by NIROJAPAN) under the following conditions, to give a fine particle having an average particle diameter of 32 μm as a dry material under cyclone.

Spraying method: two-fluid nozzle (nozzle diameter 1.2mm)
Air pressure: 1 kg/cm$^2$
Drying chamber inlet temperature: 90°C
Drying chamber outlet temperature: 40 to 43°C

The content of Compound B in the resulting fine particle was 28.1%.

Reference Example 22

2 g of Compound B and 0.996 g of zinc acetate dihydrate (manufactured by Wako Pure Chemical Industries, Ltd.) were added to a solution obtained by dissolving 3.67 g of polylactic acid (weight average molecular weight 14,500, manufactured by Wako Pure Chemical Industries, Ltd.) in 7.5 ml of dichloromethane and 3.5 ml of methanol, and the mixture was shaken and stirred at room temperature for 2 hours to obtain a uniform solution. This solution was poured into 800 ml of 0.1% by weight polyvinyl alcohol aqueous solution regulated at 15°C in advance, and made into an O/W emulsion at 7,000 rpm using a turbine-type homomixer. This O/W emulsion was stirred at room temperature for 3 hours to volatilize dichloromethane and methanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The encapsulation rate of Compound A in microcapsule was 101.6%, and the content of Compound B in microcapsule was 26.6%.

Reference Example 23

According to the same manner as in Reference Example 22 except that a uniform solution obtained by adding 2 g of Compound B, 0.757 g of zinc acetate dihydrate (manufactured by Wako Pure Chemical Industries Ltd.) and 0.277 g of zinc oxide (TYPE V, manufactured by Wako Pure Chemical Industries Ltd.) to a solution in which 3.64 g of polylactic acid (weight average molecular weight 14,500, manufactured by Wako Pure Chemical Industries, Ltd.) was dissolved in 7.5ml of dichloromethane and 3.5 ml of methanol was used, a microcapsule was obtained. The encapsulation rate of Compound B in microcapsule was 101.9%, and the content of Compound B in microcapsule was 25.9%.

Reference Example 24

2 g of Compound B and 0.996 g of zinc acetate (manufactured by Wako Pure Chemical Industries, Ltd.) were added to a solution obtained by dissolving 3 g of lactic acid/glycolic acid copolymer (lactic acid/glycolic acid 75/25 (mol%), weight average molecular weight 10,600, manufactured by Wako Pure Chemical Industries, Ltd.) in 7 ml of dichloromethane and 3 ml of methanol, to obtain a uniform solution. This was cast into a circle having a radius of about 5 cm on a plate, and dried at room temperature for 16 hours under reduced pressure to obtain a dry material. This dry material was roughly ground and sieved on a sieve having a pore diameter of 150 mm, and 3.6 g of the resulting dry material and 0.4 g of mannitol were mixed, and the mixture was ground with an air at an air pressure of 2 kg/cm$^2$ using a jet mill apparatus (A-OJET, manufactured by SEISHIN ENTERPRISE CO.,LTD.), to give a fine particle having an average particle diameter of 21 mm. The content of Compound B in fine particle was 29.1%.

Reference Example 25

2.0 g of Compound B and 3.97 g of lactic acid-glycolic acid copolymer (lactic acid/glycolic acid 75/25 (mol%), weight average molecular weight 8,700, manufactured by Wako Pure Chemical Industries) were added to a mixed solution of 12.75 ml of dichloromethane, 2.25 ml of methanol and 0.136 mL of acetic acid, and the mixture was shaken and stirred at room temperature overnight to obtain a suspension. This suspension was poured into 800 ml of 0.1% by weight polyvinyl alcohol aqueous solution containing 30 mM zinc acetate which had been regulated at 18°C in advance, and made into a S/O/W emulsion at 7,000 rpm using a turbine-type homomixer. This S/O/W emulsion was stirred at room temperature for 3 hours to volatilize dichloromethane, methanol and acetic acid, and the polymer was solidified, followed by collection at 3,000 rpm using a centrifuge. This was dispersed again in distilled water, and subjected to further centrifugation to wash free drug and the like. Collected microcapsules were lyophilized to give a powder. The encapsulation rate of Compound B in microcapsule was 94.9%.

Reference Example 26

2.0 g of Compound B, 0.37 g of zinc oxide (manufactured by Hakusui Tec. Co., Ltd.) and 3.6 g of lactic acid-glycolic acid copolymer (lactic acid/glycolic acid 75/25 (mol%), weight average molecular weight 8,700, manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a uniform solution. This solution was poured into 800 ml of 0.1% by weight polyvinyl alcohol aqueous solution containing 10 mM of zinc acetate which had been regulated at 18°C in advance, and made into an O/W emulsion at 7,000 rpm using a turbine-type homomixer. This O/W emulsion was stirred at room temperature for 3 hours to volatilize dichloromethane, methanol and acetic acid, and the oil phase was solidified, followed by collection at 3,000 rpm using a centrifuge. This was dispersed again in distilled water, and subjected to further centrifugation to wash free drug and the like. Collected microcapsules were dispersed again by adding distilled water in which 0.8 g of mannitol had been dissolved, and lyophilized to give a powder. The encapsulation rate of Compound B in microcapsule was 90.7%, and the content of Compound B in microcapsule/mannitol powder was 26.4%.

Reference Example 27

2.0 g of Compound B, 0.37 g of zinc oxide (manufactured by Hakusui Tec. Co., Ltd.) and 3.6 g of lactic acid-glycolic acid copolymer (lactic acid/glycolic acid 75/25 (mol%), weight average molecular weight 8,700, manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a uniform solution. This solution was poured into 800 ml of 0.1% by weight polyvinyl alcohol aqueous solution containing 30 mM zinc acetate which had been regulated at 18°C in advance, and made into an O/W emulsion at 7,000 rpm using a turbine-type homomixer. This O/W emulsion was stirred at room temperature for 3 hours to volatilize dichloromethane, methanol and acetic acid, and the oil phase was solidified, followed by collection at 3,000 rpm using a centrifuge. This was dispersed again in distilled water, and subjected to further centrifugation to wash free drug and the like. Collected microcapsules were dispersed again by adding distilled water in which 0.8 g of mannitol had been dissolved, and lyophilized to give a powder. The encapsulation rate of Compound B in microcapsule was 92.2%, and the content of Compound B in microcapsule/mannitol powder was 26.6%.

Reference Example 28

1.5 g of Compound B, 0.278 g of zinc oxide (manufactured by Hakusui Tec. Co., Ltd.) and 2.7 g of lactic acid-glycolic acid copolymer (lactic acid/glycolic acid 75/25 (mol%), weight average molecular weight 10,500, manufactured by Wako Pure Chemical Industries, Ltd.) were added to a mixed solution of 11.25 ml of dichloromethane, 1.69 ml of methanol and 0.102 mL of acetic acid, and the mixture was shaken and stirred at room temperature overnight to obtain a uniform solution. This solution was poured into 800 ml of 0.1% by weight polyvinyl alcohol aqueous solution containing 30 mM zinc acetate which had been regulated at 18°C in advance, and made into an O/W emulsion at 7,000 rpm using a turbine type homomixer. This O/W emulsion was stirred at room temperature for 3 hours to volatilize dichloromethane, methanol and acetic acid, and the oil phase was solidified, followed by collection at 3,000 rpm using a centrifuge. This was dispersed again in distilled water, and subjected to further centrifugation to wash free drug and the like. Collected microcapsules were dispersed again by adding distilled water in which 0.8 g of mannitol had been dissolved, and lyophilized to give a powder. The encapsulation rate of Compound B in microcapsule was 88.0%, and the content of Compound B in microcapsule/mannitol powder was 25.4%.

Reference Example 29

2 g of Compound B, 0.37 g of zinc oxide (manufactured by Hakusui Tec. Co., Ltd.) and 3.6 g of lactic acid-glycolic acid copolymer (lactic acid/glycolic acid 75/25 (mol%), weight average molecular weight 8,700, manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a uniform solution. This solution was poured into 800 ml of 0.1% by weight polyvinyl alcohol aqueous solution containing 10 mM zinc acetate which had been regulated at 18°C in advance, and made into an O/W emulsion at 7,000 rpm using a turbine-type homomixer. This O/W emulsion was stirred at room temperature for 3 hours to volatilize dichloromethane, methanol and acetic acid, and the oil phase was solidified, followed by collection at 3,000 rpm using a centrifuge. This was dispersed again in distilled water, and subjected to further centrifugation to wash free drug and the like. Collected microcapsules were dispersed again by adding distilled water in which 0.8 g of mannitol had been dissolved, and lyophilized to give a powder. The encapsulation rate of Compound B in microcapsule was 89.1%, and the content of

Compound B in microcapsule/mannitol powder was 26.2%.

Example 1

3 mL of dichloromethane and 0.9 mL of methanol were added to 0.2 g of 2-ethoxy-1-[[2'-(1H-tetrazol-5-yl) biphenyl-4-yl]methyl]benzimidazole-7-carboxylic acid (Compound B), 0.1 g of zinc acetate dihydrate (manufactured by Wako Pure Chemical Industries, Ltd.) and 1.5 g of lactic acid-glycolic acid copolymer (lactic acid/glycolic acid 75/25 (mol%), weight average molecular weight 14,000, number average molecular weight 4,200, number average molecular weight according to terminal group quantitation 4,090, manufactured by Wako Pure Chemical Industries, Ltd.), to obtain a solution. A solution obtained by further adding and dissolving 0.2 g of actos thereto was poured into 800 mL of 0.1% by weight polyvinyl alcohol aqueous solution regulated at 15°C in advance, and made into an O/W emulsion at 7,200 rpm using a turbine-type homomixer. This O/W emulsion was stirred at room temperature for 3 hours to volatilize dichloromethane and methanol, and the oil phase was solidified, followed by collection at 2,000 rpm using a centrifuge. This was dispersed again in distilled water, and subjected to further centrifugation to wash free drug and the like. Collected microcapsules were dispersed again by adding distilled water in which a small amount of mannitol had been dissolved, and lyophilized to give a powder. The contents of Compound B and actos in microcapsule were 10% and 12%, respectively.

Experimental Example 1

25 mg of the microcapsule obtained in Example 1 was dispersed in 0.1 mL of dispersing medium (a solution in which 5 mg of sodium carboxymethylcellulose, 1 mg of Polysorbate 80 and 50 mg of mannitol were dissolved in 1 mL of distilled water), and this was subcutaneously administered to a 9 week old male SD rat with 23G injection needle at a rear neck part. The concentrations of drugs in plasma obtained by taking from a tail vein with time after administration were measured. The results are shown in Table 1.

[Table 1]

| Average drug concentration in plasma of rat after administration of microcapsule (n = 4) | | | | |
|---|---|---|---|---|
| | After 1 day | After 1 week | After 2 weeks | After 3 weeks |
| Compound B (ng/mL) | 170 | 238 | 94 | 4 |
| actos (ng/mL) | 313 | 226 | 155 | 30 |

As apparent from Table 1, the plasma concentration of drug lasts, and sustained-release of both compounds from the present preparation was confirmed.

Industrial applicability

The sustained-release medicine of the present invention comprising a combination of (A) an angiotensin II antagonist and (B) one or more drugs selected from a remedy for hypertension, a hypoglycemic drug, a remedy for hyperlipemia, an antithrombotic drug, a remedy for climacteric disturbance and an anticancer drug can considerably reduce doses for the case that respective active ingredients are used alone and, as a result, it becomes possible to suppress manifestation of side effects of the drugs as compared with the use of each of them alone. Thus, the sustained-release medicine is advantageously used as an agent for preventing or treating angiotensin II-mediated various diseases, in particular, as an agent for preventing or treating arterial hypertension, diabetes, hyperlipemia, thrombosis and menopausal disorder which are complexed with arteriosclerosis or hypertension. Further, the sustained-release medicine can potentiate the activities possessed by drugs alone to be used, such as hypotensive activity, blood sugar lowering activity, cholesterol lowering activity, antithrombotic activity and anticancer activity and, at the same time, the synergistic effect for suppressing cardiovascular event can also be expected.

Since the sustained-release medicine of the present invention contains an AII antagonist and a concomitant drug selected from a remedy for hypertension, a hypoglycemic drug, a remedy for hyperlipemia, an antithrombotic drug, a remedy for climacteric disturbance and an anticancer drug at high content, and can control its release rate, it shows angiotensin II antagonism and hypoglycemic activity, cholesterol lowering activity, antithrombotic activity, anticancer activity and the like while maintaining circadian rhythm of a blood pressure over the long term. In addition, since a constant blood concentration can be maintained day or night, variation in blood concentration of drug is small as compared with administration of oral agent, and continuation of stable pharmacological activity can be expected. Therefore, the present sustained-release medicine hardly causes symptom exacerbation due to intentional avoidance of

dosing such as variation in dosing time and interruption of dosing for a patient group having less subjective symptom, and effects of treatment of hypertension, blood pressure circadian rhythm abnormality, heart disease (hypercardia, heart failure, cardiac infarction etc.), cerebrovascular disorder (silent cerebral infarction, transient cerebral ischemic attack, cerebral stroke, cerebrovascular dementia, hypertensive encephalopathy etc.), ischemic peripheral circulatory disorder, obstructive arteriosclerosis, obstructive thrombotic angitis, myocardial ischemia, cardiomyopathy, venous dysfunction, heart failure progression after cardiac infarction, and cerebrovascular disorder sequela as well as diabetic complex, diabetic retinopathy, diabetic nephropathy, nephritis, glomerular nephritis, nephropathy due to radiation irradiation, atherosclerosis, arteriosclerosis, vascular hypertrophy, vascular hypertrophy or occulusion after intervention, vascular reocculusion after bypass operation, polycythemia• hypertension• organ disorder• vascular hypertrophy after transplantation, rejection reaction after transplantation, hyperaldosteronism, glomerulosclerosis, renal failure, portal pressure hyperfunction, glaucoma, hyper-ocular tension, hyperlipemia, cardiac angina, aneurysm, coronary sclerosis, cerebral arthrosclerosis, peripheral arthrosclerosis, thrombosis, central nervous disorder, Alzheimer's disease, memory deficiency, depression, amnesia, senile dementia, sensory dysfunction, multiple organ failure, endothelial dysfunction, hypertensive tinnitus, Ménière's syndrome, scleroderma, or anxiety accompanied with dizziness, tonic symptom and unpleasant mental status, further maldigestion, autonomic dysfunction, myasthenia gravis or cancer and cancer-associated disease, climacteric disturbance and the like are expected to be clearer. In addition, the sustained-release preparation of the present invention is a potent medicament which can be also used to a bed-ridden patient, a patient with dementia, throat•esophageal disease, digestive organ disease, or a patient with anorexia•dysphagia, or a patient at operation who is difficult or impossible to treat with oral medicine.

Recently, it is known that lipid metabolism abnormality, hypertension, diabetes and the like are risk factors of cardiovascular diseases and, when these are overlapped, the risk is increased more than alone. For example, vascular endothelial dysfunction and arteriosclerosis are progressed, and severe cardiovascular diseases such as coronary disease and cerebral stroke are caused by destabilizing plaques.

The AII antagonist, remedy for hypertension, hypoglycemic drug, remedy for hyperlipemia and the like used in the present invention give multilateral risk decreasing effects to a group that has such the risk factors of arteriosclerosis·cardiovascular disease and has high cardiovascular risk.

The great target of pressure decreasing therapy by an AII antagonist is to prevent or block the progression of important organ disorder or cardiovascular complex which has great influence on prognosis of hypertensive patient, and reduce the morbidity and mortality of cardiovascular disease. Many of patients who are subjects of pressure decreasing therapy need concurrent treatment, and have plural risk factors, and there are many cases accompanied with organ disorder and complex. Therefore, it is desirable to select a combination of drugs which have little influence on other risk factors such as sugar•lipid metabolism and also have organ protecting activity together (in particular, renal•vascular protection, anti-arteriosclerosis activity etc). From such the point of view, the combinatorial preparation of the present invention, in particular, a long-acting sustained-release agent is useful for therapy depending on pathology of individual symptoms, for the purpose of preventing or treating organ disorder or complex.

## Claims

**1.** A sustained-release medicine comprising combining (A) an angiotensin II antagonist with (B) one or more drugs selected from a remedy for hypertension, a hypoglycemic drug, a remedy for hyperlipemia, an antithrombotic drug, a remedy for climacteric disturbance and an anticancer drug.

**2.** The medicine according to claim 1, wherein the angiotensin II antagonist is a compound represented by the formula (I):

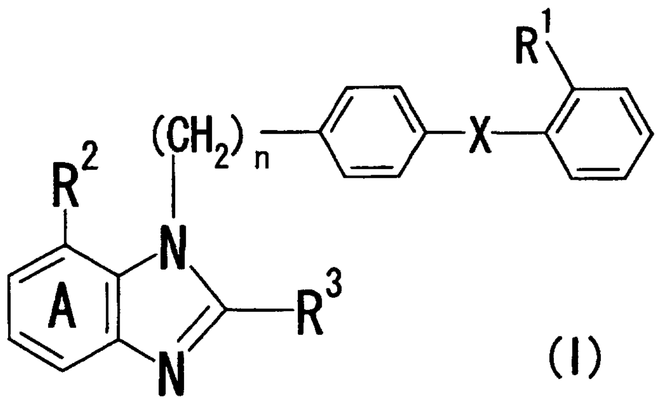

(I)

wherein $R^1$ represents a group capable of forming an anion or a group capable of converting to said group, X represents that a phenylene group and a phenyl group are linked directly or via a spacer having a chain of 2 or less of atoms, n represents 1 or 2, ring A represents a benzene ring which may further have a substituent, $R^2$ represents a group capable of forming an anion or a group capable of converting to said group, and $R^3$ represents a hydrocarbon group which may be bound through a hetero atom and may have a substituent, or a salt thereof.

**3.** The medicine according to claim 1, wherein the angiotensin II antagonist is Losartan, Eprosartan, Candesartan cilexetil, Candesartan, Valsartan, Telmisartan, Irebesartan, Olmesartan or Tasosartan.

**4.** The medicine according to claim 1, wherein the angiotensin II antagonist is 2-ethoxy-1-[[2'-(1H-tetrazol-5-yl)biphenyl-4-yl]methyl]benzimidazole-7-carboxylic acid, 1-(cyclohexyloxycarbonyloxy)ethyl 2-ethoxy-1-[[2'-(1H-tetrazol-5-yl)biphenyl-4-yl]methyl]benzimidazole-7-carboxylate, or 2-ethoxy-1-[[2'-(2,5-dihydro-5-oxo-1,2,4-oxadiazol-3-yl)biphenyl-4-yl]methyl]benzimidazole-7-carboxylic acid or a salt thereof.

**5.** The medicine according to claim 1, wherein the remedy for hypertension is a drug selected from an angiotensin converting enzyme inhibitor, a diuretic, a calcium antagonist, a vasopressin antagonist, an angiotensin converting enzyme and neutral endopeptidase inhibitor, a β blocker and an aldesterone antagonist.

**6.** The medicine according to claim 5, wherein the angiotensin converting enzyme inhibitor is a drug selected from enalapril, cilazapril, tamocapril, trandolapril, lisinopril and ramipril.

**7.** The medicine according to claim 5, wherein the diuretic is a drug selected from indapamide, trichlormethiazide, bumetanide, hydrochlorothiazide and metolazone.

**8.** The medicine according to claim 5, wherein the calcium antagonist is a drug selected from amlodipine, nitrendipine and manidipine.

**9.** The medicine according to claim 5, wherein the vasopressin antagonist is a drug selected from tolvaptan, conivaptan hydrochloride and relcovaptan.

**10.** The medicine according to claim 5, wherein the angiotensin converting enzyme and neutral endopeptidase inhibitor is a drug selected from omapatrilat , fasidotril and sampatrilat .

**11.** The medicine according to claim 5, wherein the β blocker is a drug selected from carvedilol, metoprolol and propranolol.

**12.** The medicine according to claim 5, wherein the aldrosterone antagonist is spironolactone.

**13.** The medicine according to claim 1, wherein the hypoglycemic drug is an insulin sensitizer, an insulin secretagogue or an insulin preparation.

**14.** The medicine according to claim 13, wherein the insulin sensitizer is a drug selected from pioglitazone hydrochloride, troglitazone and rosiglitazone maleate.

**15.** The medicine according to claim 13, wherein the insulin secretagogue is a drug selected from glibenclamide, nateglinide and repaglinide.

**16.** The medicine according to claim 1, wherein the remedy for hyperlipemia is a statin medicament, a fibrate medicament or a nicotinic acid derivative.

**17.** The medicine according to claim 16, wherein the statin medicament is a drug selected from sodium cerivastatin, sodium pravastatin, simvastatin and calcium atrovastatin hydrate.

**18.** The medicine according to claim 16, wherein the fibrate medicament is fenofibrate, fenofibrinic acid, bezafibrate or gemfibrozil.

**19.** The medicine according to claim 16, wherein the nicotinic acid derivative is niceritrol or cholexamin.

**20.** The medicine according to claim 1, wherein the antithrombotic drug is a drug selected from a GPIIb/IIIa antagonist, low-molecular weight heparin, a thrombin inhibitor and an anti-platelet drug.

**21.** The medicine according to claim 20, wherein the GPIIb/IIIa antagonist is abciximab.

**22.** The medicine according to claim 20, the low-molecular weight heparin is sodium enoxaparin.

**23.** The medicine according to claim 20, wherein the thrombin inhibitor is argatroban.

**24.** The medicine according to claim 20, wherein the anti-platelet drug is clopidogrel sulfate or aspirin.

**25.** The medicine according to claim 1, wherein the remedy for climacteric disturbance is an estrogen selected from estradiol, estradiol valerate and conjugated estrogen.

**26.** The medicine according to claim 1, wherein the anticancer drug is a GnRH agonist or antagonist.

**27.** The medicine according to claim 26, wherein the GnRH agonist is leuprorelin or a salt thereof.

**28.** The medicine according to claim 1, which contains (A) an angiotensin II antagonist and (B) one or more drugs selected from a remedy for hypertension, a hypoglycemic drug, a remedy for hyperlipemia, an antithrombotic drug, a remedy for climacteric disturbance and an anticancer drug.

**29.** The medicine according to claim 1, which contains (A) a sustained-release preparation containing an angiotensin II antagonist and (B) a sustained-release preparation containing one or more drugs selected from a remedy for hypertension, a hypoglycemic drug, a remedy for hyperlipemia, an antithrombotic drug, a remedy for climacteric disturbance and an anticancer drug.

**30.** The medicine according to claim 1, which comprises a combination of (A) a sustained-release preparation containing an angiotensin II antagonist and (B) a sustained release preparation containing one or more drugs selected from a remedy for hypertension, a hypoglycemic drug, a remedy for hyperlipemia, an antithrombotic drug, a remedy for climacteric disturbance and an anticancer drug.

**31.** The medicine according to claim 1, which contains (C) a biodegradable polymer.

**32.** The medicine according to claim 31, wherein the biodegradable polymer is an $\alpha$-hydroxycarboxylic acid polymer.

**33.** The medicine according to claim 32, wherein the $\alpha$-hydroxycarboxylic acid polymer is a lactic acid-glycolic acid polymer.

**34.** The medicine according to claim 33, wherein a molar ratio of lactic acid to glycolic acid is 100/0 to 40/60.

**35.** The medicine according to claim 32, wherein a weight average molecular weight of the polymer is 3,000 to 50,000.

**36.** The medicine according to claim 1, which is for injection.

**37.** The medicine according to claim 1, which is an agent for prevention or treatment of circulatory diseases.

**38.** The medicine according to claim 1, which is an agent for prevention or treatment of hypertension.

**39.** The medicine according to claim 1, which is an agent for prevention or treatment of blood pressure circadian rhythm abnormality.

**40.** The medicine according to claim 1, which is an agent for prevention or treatment of organ disorder.

**41.** The medicine according to claim 1, which is an agent for prevention or treatment of cancer.

**42.** The medicine according to claim 1, which is an agent for protecting organs.

**43.** A method for treating circulatory disease, hypertension, blood pressure circadian rhythm abnormality, organ disorder or cancer, which comprises administering the medicine according to claim 1 to a mammal.

**44.** A use of the medicine according to claim 1 for preparing a medicament for treatment of circulatory disease, hypertension, blood pressure circadian rhythm abnormality, organ disorder or cancer.

**45.** A sustained-release medicine comprising a combination of two or three drugs selected from an angiotensin II antagonist, a remedy for hypertension, a hypoglycemic drug and a remedy for hyperlipemia.

## INTERNATIONAL SEARCH REPORT

International application No.
PCT/JP02/07862

A. CLASSIFICATION OF SUBJECT MATTER
Int.Cl[7] A61K45/06, 31/41, 31/4178, 31/4184, 31/4245, 31/519, 47/34, A61P9/00, 9/12, 35/00, 43/00

According to International Patent Classification (IPC) or to both national classification and IPC

B. FIELDS SEARCHED

Minimum documentation searched (classification system followed by classification symbols)
Int.Cl[7] A61K45/06, 31/41, 31/4178, 31/4184, 31/4245, 31/519, 47/34

Documentation searched other than minimum documentation to the extent that such documents are included in the fields searched

Electronic data base consulted during the international search (name of data base and, where practicable, search terms used)
CAplus(STN), MEDLINE(STN), JICST FILE(JOIS), WPI(DIALOG)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 01/05428 A1 (Takeda Chemical Industries, Ltd.), 25 January, 2001 (25.01.01), Particularly, Claims; description, page 28, lines 22 to 26; page 29, lines 18 to 23; page 30, lines 13 to 21<br>& JP 2001-89393 A & AU 200060197 A<br>& EP 1197226 A1 | 1-24,28-30, 36-40,42,44<br>25-27,31-35, 41 |
| X<br><br>Y | WO 99/44590 A1 (Takeda Chemical Industries, Ltd.), 10 September, 1999 (10.09.99), Especially, see Claims and page 45, second paragraph.<br>& AU 9927451 A & JP 11-315034 A<br>& NO 200004350 A & CZ 200003164 A3<br>& BR 9908474 A & EP 1058541 A1<br>& ZA 9901706 A & SK 200001190 A3<br>& MX 2000008171 A1 & CN 1291888 A<br>& HU 200101439 A2 | 1-12,28-40, 42,44<br>13-27,41 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 November, 2002 (20.11.02) | 03 December, 2002 (03.12.02) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/07862

C (Continuation). DOCUMENTS CONSIDERED TO BE RELEVANT

| Category* | Citation of document, with indication, where appropriate, of the relevant passages | Relevant to claim No. |
|---|---|---|
| Y | JP 9-221420 A (Takeda Chemical Industries, Ltd.), 26 August, 1997 (26.08.97), (Family: none) | 1-42,44 |
| Y | EP 930076 A1 (Sankyo Co., Ltd.), 21 July, 1999 (21.07.99), Especially, see Claims & EP 1175902 A1 & WO 98/02183 A1 & AU 9734595 A & AU 714618 B & JP 10-81632 A & NO 9900166 A & CZ 9900102 A3 & CN 1230122 A & MX 9900607 A1 & NZ 333723 A & HU 9903166 A2 & KR 2000023757 A & US 2002/0013308 A1 & RU 2183128 C2 | 1-42,44 |
| Y | WO 97/37688 A2 (Takeda Chemical Industries, Ltd.), 16 October, 1997 (16.10.97), Especially, see Claims & JP 9-323940 A & AU 9721780 A & AU 713277 B & NO 9804123 A & CZ 9802886 A3 & EP 914158 A2 & EP 914158 B1 & EP 1192951 A2 & SK 9801278 A3 & NZ 330774 A & CN 1215338 A & BR 9708517 A & HU 9902746 A2 & US 6107323 A & US 6432996 B1 & MX 9807129 A1 & KR 99087076 A & DE 69713890 E | 1-42,44 |
| Y | Akihiko YASUMIZU, "Shin'yaku Tenbo 2000 Konenki Shogai Chiryoyaku", Iyaku Journal, 01 January, 2000 (01.01.00), Vol.36, extra issue, pages 220 to 226 | 1,25,28-40, 42,44 |
| Y | WO 99/01764 A2 (VAN GROENINGHEN, Johannes, Christianus), 14 January, 1999 (14.01.99), & DE 19728737 C1 & AU 9892515 A & EP 993613 A2 & JP 2002-511937 A | 1,26-42,44 |

Form PCT/ISA/210 (continuation of second sheet) (July 1998)

**INTERNATIONAL SEARCH REPORT**

International application No.
PCT/JP02/07862

**Box I Observations where certain claims were found unsearchable (Continuation of item 1 of first sheet)**

This international search report has not been established in respect of certain claims under Article 17(2)(a) for the following reasons:

1. [X] Claims Nos.: 43
because they relate to subject matter not required to be searched by this Authority, namely:
Since humans fall within the category of mammals, the invention as set forth in claim 43 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
(See extra sheet.)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.: 1 to 42 and 44

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/07862

Continuation of Box No.II of continuation of first sheet(1)

The requirement of unity of invention (Rule 13.1 of the PCT) in an international application is not fulfilled unless there is a technical relationship involving one or more of the same or corresponding special technical features in a group of claimed inventions. This "special technical feature" means a contribution which each of the claimed inventions, considered as a whole, makes over the prior art (Rule 13.2 of the PCT).

In the present case, the matter common to the inventions as set forth in claims 1 to 42 and 44 resides in combining "an angiotensin II antagonist" with other drugs in a sustained-release medicine. In the invention as set forth in claim 45, on the other hand, "an angiotensin II antagonist" is merely one of ingredients usable in a sustained-release medicine having a plural number of drugs combined together. Namely, it is not always necessary to employ the angiotensin II antagonist. Sustained-release medicines having a plural number of drugs combined together had been publicly known without citing any documents. Even though a remedy for circulatory diseases is combined therewith, such a combination is publicly known or self-evident to those skilled in the art. Therefore, there is no "special technical feature" common to the inventions as set forth in claims 1 to 42 and 44 and the invention as set forth in claim 45.

Such being the case, the present international application has 2 groups of inventions, i.e., (1) the inventions as set forth in claims 1 to 42 and 44, and (2) the invention as set forth in claim 45.

Form PCT/ISA/210 (extra sheet) (July 1998)